(19) Europäisches Patentamt
European Patent Office
Office européen des brevets

(11) EP 4 498 324 A1

# (12) EUROPÄISCHE PATENTANMELDUNG

(43) Veröffentlichungstag:
**29.01.2025 Patentblatt 2025/05**

(21) Anmeldenummer: **24189305.6**

(22) Anmeldetag: **18.07.2024**

(51) Internationale Patentklassifikation (IPC):
***G06T 11/00*** *(2006.01)* ***G06T 5/60*** *(2024.01)*

(52) Gemeinsame Patentklassifikation (CPC):
**G06T 11/008; G06T 5/60;** G06T 2207/10072; G06T 2207/20081; G06T 2207/20084; G06T 2211/441; G06T 2211/444

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH MA MD TN**

(30) Priorität: **25.07.2023 EP 23187518**

(71) Anmelder: **Bayer AG**
**51373 Leverkusen (DE)**

(72) Erfinder:
- **Lenga, Matthias**
  **24159 Kiel (DE)**
- **Baltruschat, Ivo Matteo**
  **22299 Hamburg (DE)**

(74) Vertreter: **BIP Patents**
**c/o Bayer Intellectual Property GmbH**
**Alfred-Nobel-Straße 50**
**40789 Monheim am Rhein (DE)**

## (54) ERKENNEN VON ARTEFAKTEN IN SYNTHETISCHEN BILDERN

(57) Die vorliegende Offenbarung betrifft das technische Gebiet der Erzeugung von synthetischen Bildern, insbesondere synthetischen medizinischen Aufnahmen. Gegenstände der vorliegenden Offenbarung sind ein Verfahren, ein Computersystem und ein computerlesbares Speichermedium umfassend ein Computerprogramm zum Erkennen von Artefakten in synthetischen Bildern, insbesondere synthetischen medizinischen Aufnahmen.

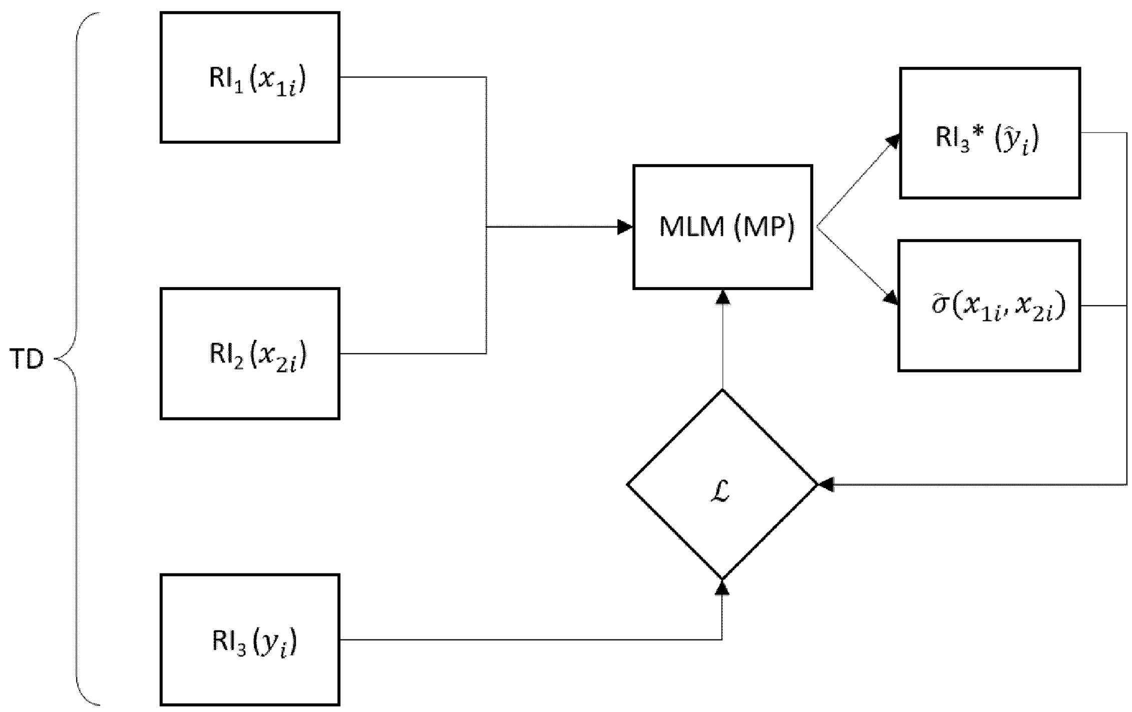


Fig. 4

## Beschreibung

HINWEIS ZUM URHEBERRECHT

Ein Teil der Offenbarung dieser Patentschrift enthält Material, das dem Urheberrechtsschutz unterliegt. Der Urheberrechtsinhaber hat keine Einwände gegen die Faksimile-Reproduktion der Patentschrift, wie sie in einer Patentakte oder in den Akten des Patentamts erscheint, behält sich aber ansonsten alle Urheberrechte und Rechte jeglicher Art vor. © 2024 Bayer AG

TECHNISCHES GEBIET

Die vorliegende Offenbarung betrifft das technische Gebiet der Erzeugung von synthetischen Bildern, insbesondere synthetischen medizinischen Aufnahmen. Gegenstände der vorliegenden Offenbarung sind ein Verfahren, ein Computersystem und ein computerlesbares Speichermedium umfassend ein Computerprogramm zum Erkennen von Artefakten in synthetischen Bildern, insbesondere synthetischen medizinischen Aufnahmen.

EINLEITUNG

Künstliche Intelligenz findet zunehmend Einzug in die Medizin. Modelle des maschinellen Lernens werden nicht nur verwendet, um Anzeichen von Krankheiten in medizinischen Aufnahmen des menschlichen oder tierischen Körpers zu identifizieren (siehe z.B. WO2018202541A1, WO2020229152A1), sie werden zunehmend auch eingesetzt, um synthetische (künstliche) medizinische Aufnahmen zu erzeugen.

WO2021052896A1 und WO2021069338A1 beschreiben beispielsweise Verfahren zum Erzeugen einer künstlichen medizinischen Aufnahme. Die künstliche medizinische Aufnahme wird mit Hilfe eines trainierten Modells des maschinellen Lernens auf Basis von medizinischen Aufnahmen, die den Untersuchungsbereich eines Untersuchungsobjekts in einer ersten Zeitspanne zeigen, erzeugt. Die künstliche medizinische Aufnahme zeigt den Untersuchungsbereich des Untersuchungsobjekts in einer zweiten Zeitspanne, die der ersten Zeitspanne zeitlich nachgelagert ist. Mit Hilfe des Verfahrens können beispielsweise radiologische Untersuchungen beschleunigt werden; anstatt radiologische Aufnahmen über eine längere Zeitspanne zu messen, werden Messungen nur innerhalb eines Teils der Zeitpanne gemessen und eine oder mehrere radiologische Aufnahmen für den übrigen Teil der Zeitspanne mit Hilfe des trainierten Modells vorhergesagt.

WO2019/074938A1 und WO2022184297A1 beschreiben beispielsweise Verfahren zum Erzeugen einer künstlichen radiologischen Aufnahme, die einen Untersuchungsbereich eines Untersuchungsobjekts nach der Applikation einer Standardmenge eines Kontrastmittels zeigen, obwohl nur eine geringere Menge an Kontrastmittel als die Standardmenge appliziert worden ist. Die Standardmenge ist die vom Hersteller und/oder Vertreiber des Kontrastmittels empfohlene und/oder die von einer Zulassungsbehörde zugelassene und/oder die in einem Beipackzettel zum Kontrastmittel aufgeführte Menge. Die in WO2019/074938A1 und WO2022184297A beschriebenen Verfahren können also zur Reduzierung der Menge an Kontrastmittel eingesetzt werden.

Die von den trainierten Modellen des maschinellen Lernens erzeugten medizinischen Aufnahmen können Fehler aufweisen (siehe z.B.: K. Schwarz et al.: On the Frequency Bias of Generative Models, https://doi.org/10.48550/arXiv.2111.02447).

Solche Fehler können problematisch sein, da ein Arzt auf Basis der künstlichen medizinischen Aufnahmen eine Diagnose stellen und/oder eine Therapie initiieren könnte. Wenn ein Arzt künstliche medizinische Aufnahmen begutachtet, muss der Arzt wissen, ob Merkmale in den künstlichen medizinischen Aufnahmen auf reale Merkmale des Untersuchungsobjekts zurückgeführt werden können, oder ob es sich um Artefakte handelt, die auf Fehler bei der Vorhersage durch das trainierte Modell des maschinellen Lernens zurückzuführen sind.

ZUSAMMENFASSUNG

Diese und weitere Probleme werden durch die Gegenstände der vorliegenden Offenbarung adressiert.

Ein erster Gegenstand der vorliegenden Offenbarung ist ein computer-implementiertes Verfahren zum Erzeugen mindestens eines Vertrauenswertes für ein synthetisches Bild, umfassend die Schritte:

- Bereitstellen eines trainierten Modells des maschinellen Lernens,

    o wobei das trainierte Modell des maschinellen Lernens anhand von Trainingsdaten trainiert wurde,

    o wobei die Trainingsdaten für jedes Referenzobjekt einer Vielzahl von Referenzobjekten (i) mindestens ein

Eingabe-Referenzbild eines Referenzbereichs des Referenzobjekts in einem ersten Zustand und (ii) ein Ziel-Referenzbild des Referenzbereichs des Referenzobjekts in einem zweiten Zustand umfassen, wobei das mindestens eine Eingabe-Referenzbild und das Ziel-Referenzbild jeweils eine Vielzahl an Bildelementen umfassen,

o wobei das Modell des maschinellen Lernens konfiguriert ist und trainiert wurde, für jedes Referenzobjekt auf Basis des mindestens einen Eingabe-Referenzbildes ein synthetisches Referenzbild zu erzeugen,

▪ wobei das synthetische Referenzbild eine Vielzahl an Bildelementen umfasst, wobei jedes Bildelement des synthetischen Referenzbildes mit jeweils einem Bildelement des Ziel-Referenzbildes korrespondiert,

▪ wobei das Modell des maschinellen Lernens trainiert wurde, für jedes Bildelement des synthetischen Referenzbildes einen Farbwert und einen Unsicherheitswert für den vorhergesagten Farbwert vorherzusagen,

▪ wobei das Trainieren ein Minimieren einer Fehlerfunktion umfasst, wobei die Fehlerfunktion (i) den vorhergesagten Farbwert und/oder eine Abweichung des vorhergesagten Farbwerts von einem Farbwert des korrespondierenden Bildelements des synthetischen Referenzbildes und (ii) den vorhergesagten Unsicherheitswert als Parameter umfasst,

- Empfangen mindestens eines Eingabe-Bildes eines Untersuchungsbereichs eines Untersuchungsobjekts, wobei das mindestens eine Eingabe-Bild den Untersuchungsbereich des Untersuchungsobjekts in dem ersten Zustand repräsentiert,
- Zuführen des mindestens einen Eingabe-Bildes dem trainierten Modell des maschinellen Lernens,
- Empfangen eines synthetischen Bildes von dem trainierten Modell des maschinellen Lernens, wobei das synthetische Bild den Untersuchungsbereich des Untersuchungsobjekts in dem zweiten Zustand repräsentiert,
- Empfangen eines Unsicherheitswertes für jedes Bildelement des synthetischen Bildes,
- Ermitteln mindestens eines Vertrauenswertes auf Basis der empfangenen Unsicherheitswerte,
- Ausgeben des mindestens einen Vertrauenswertes.

Ein weiterer Gegenstand der vorliegenden Offenbarung ist ein computerlesbares Speichermedium umfassend ein Computerprogramm, das in einen Arbeitsspeicher eines Computersystems geladen werden kann und dort das Computersystem dazu veranlasst, folgende Schritte ausführen:

- Bereitstellen eines trainierten Modells des maschinellen Lernens,

o wobei das trainierte Modell des maschinellen Lernens anhand von Trainingsdaten trainiert wurde,

o wobei die Trainingsdaten für jedes Referenzobjekt einer Vielzahl von Referenzobjekten (i) mindestens ein Eingabe-Referenzbild eines Referenzbereichs des Referenzobjekts in einem ersten Zustand und (ii) ein Ziel-Referenzbild des Referenzbereichs des Referenzobjekts in einem zweiten Zustand umfassen, wobei das mindestens eine Eingabe-Referenzbild und das Ziel-Referenzbild jeweils eine Vielzahl an Bildelementen umfassen,

o wobei das Modell des maschinellen Lernens konfiguriert ist und trainiert wurde, für jedes Referenzobjekt auf Basis des mindestens einen Eingabe-Referenzbildes ein synthetisches Referenzbild zu erzeugen,

▪ wobei das synthetische Referenzbild eine Vielzahl an Bildelementen umfasst, wobei jedes Bildelement des synthetischen Referenzbildes mit jeweils einem Bildelement des Ziel-Referenzbildes korrespondiert,

▪ wobei das Modell des maschinellen Lernens trainiert wurde, für jedes Bildelement des synthetischen Referenzbildes einen Farbwert und einen Unsicherheitswert für den vorhergesagten Farbwert vorherzusagen,

▪ wobei das Trainieren ein Minimieren einer Fehlerfunktion umfasst, wobei die Fehlerfunktion (i) den vorhergesagten Farbwert und/oder eine Abweichung des vorhergesagten Farbwerts von einem Farbwert des korrespondierenden Bildelements des Ziel-Referenzbildes und (ii) den vorhergesagten Unsicherheitswert als Parameter umfasst,

- Empfangen mindestens eines Eingabe-Bildes eines Untersuchungsbereichs eines Untersuchungsobjekts, wobei das mindestens eine Eingabe-Bild den Untersuchungsbereich des Untersuchungsobjekts in dem ersten Zustand repräsentiert,

- Zuführen des mindestens einen Eingabe-Bildes dem trainierten Modell des maschinellen Lernens,

- Empfangen eines synthetischen Bildes von dem trainierten Modell des maschinellen Lernens, wobei das synthetische Bild den Untersuchungsbereich des Untersuchungsobjekts in dem zweiten Zustand repräsentiert,

- Empfangen eines Unsicherheitswertes für jedes Bildelement des synthetischen Bildes,

- Ermitteln mindestens eines Vertrauenswertes auf Basis der empfangenen Unsicherheitswerte,

- Ausgeben des mindestens einen Vertrauenswertes.

Ein weiterer Gegenstand der vorliegenden Offenbarung ist ein Kontrastmittel zur Verwendung in einem radiologischen Untersuchungsverfahren, wobei das radiologische Untersuchungsverfahren umfasst:

- Bereitstellen eines trainierten Modells des maschinellen Lernens,

  o wobei das trainierte Modell des maschinellen Lernens anhand von Trainingsdaten trainiert wurde,

  o wobei die Trainingsdaten für jedes Referenzobjekt einer Vielzahl von Referenzobjekten (i) mindestens eine radiologische Eingabe-Referenzaufnahme eines Referenzbereichs des Referenzobjekts in einem ersten Zustand und (ii) eine radiologische Ziel-Referenzaufnahme des Referenzbereichs des Referenzobjekts in einem zweiten Zustand umfassen,

  o wobei der erste Zustand den Referenzbereich des Referenzobjekts in einer ersten Zeitspanne vor und/oder nach der Applikation des Kontrastmittels repräsentiert und der zweite Zustand den Referenzbereich des Referenzobjekts in einer zweiten Zeitspanne nach der Applikation des Kontrastmittels repräsentiert, und/oder der erste Zustand den Referenzbereich des Referenzobjekts vor und/oder nach der Applikation einer ersten Menge des Kontrastmittels repräsentiert und der zweite Zustand den Referenzbereich des Referenzobjekts nach der Applikation einer zweiten Menge des Kontrastmittels repräsentiert,

  o wobei die mindestens eine radiologische Eingabe-Referenzaufnahme und die radiologische Ziel-Referenzaufnahme jeweils eine Vielzahl an Bildelementen umfassen,

  o wobei das Modell des maschinellen Lernens konfiguriert ist und trainiert wurde, für jedes Referenzobjekt auf Basis der mindestens einen radiologischen Eingabe-Referenzaufnahme eine synthetische radiologische Referenzaufnahme zu erzeugen,

    ▪ wobei die synthetische radiologische Referenzaufnahme eine Vielzahl an Bildelementen umfasst, wobei jedes Bildelement der synthetischen radiologischen Referenzaufnahme mit jeweils einem Bildelement der radiologischen Ziel-Referenzaufnahme korrespondiert,

    ▪ wobei das Modell des maschinellen Lernens trainiert wurde, für jedes Bildelement der synthetischen radiologischen Referenzaufnahme einen Farbwert und einen Unsicherheitswert für den vorhergesagten Farbwert vorherzusagen,

    ▪ wobei das Trainieren ein Minimieren einer Fehlerfunktion umfasst, wobei die Fehlerfunktion (i) den vorhergesagten Farbwert und/oder eine Abweichung des vorhergesagten Farbwerts von einem Farbwert des korrespondierenden Bildelements der radiologischen Ziel-Referenzaufnahme und (ii) den vorhergesagten Unsicherheitswert als Parameter umfasst,

- Empfangen oder Erzeugen mindestens einer radiologischen Eingabe-Aufnahme eines Untersuchungsbereichs eines Untersuchungsobjekts, wobei die mindestens eine radiologische Eingabe-Aufnahme den Untersuchungsbereich des Untersuchungsobjekts in dem ersten Zustand repräsentiert,

- Zuführen der mindestens einen radiologischen Eingabe-Aufnahme dem trainierten Modell des maschinellen Lernens,

- Empfangen einer synthetischen radiologischen Aufnahme von dem trainierten Modell des maschinellen Lernens, wobei die synthetische radiologische Aufnahme den Untersuchungsbereich des Untersuchungsobjekts in dem zweiten Zustand repräsentiert,

- Empfangen eines Unsicherheitswertes für jedes Bildelement der synthetischen radiologischen Aufnahme,

- Ermitteln mindestens eines Vertrauenswertes auf Basis der empfangenen Unsicherheitswerte,

- Ausgeben des mindestens einen Vertrauenswertes.

Ein weiterer Gegenstand der vorliegenden Offenbarung ist eine Verwendung eines Kontrastmittels in einem radiologischen Untersuchungsverfahren, wobei das radiologische Untersuchungsverfahren umfasst:

- Bereitstellen eines trainierten Modells des maschinellen Lernens,

  o wobei das trainierte Modell des maschinellen Lernens anhand von Trainingsdaten trainiert wurde,

  o wobei die Trainingsdaten für jedes Referenzobjekt einer Vielzahl von Referenzobjekten (i) mindestens eine radiologische Eingabe-Referenzaufnahme eines Referenzbereichs des Referenzobjekts in einem ersten Zustand und (ii) eine radiologische Ziel-Referenzaufnahme des Referenzbereichs des Referenzobjekts in einem zweiten Zustand umfassen,

  o wobei der erste Zustand den Referenzbereich des Referenzobjekts in einer ersten Zeitspanne vor und/oder nach der Applikation des Kontrastmittels repräsentiert und der zweite Zustand den Referenzbereich des Referenzobjekts in einer zweiten Zeitspanne nach der Applikation des Kontrastmittels repräsentiert, und/oder der erste Zustand den Referenzbereich des Referenzobjekts vor und/oder nach der Applikation einer ersten Menge des Kontrastmittels repräsentiert und der zweite Zustand den Referenzbereich des Referenzobjekts nach der Applikation einer zweiten Menge des Kontrastmittels repräsentiert,

  o wobei die mindestens eine radiologische Eingabe-Referenzaufnahme und die radiologische Ziel-Referenzaufnahme jeweils eine Vielzahl an Bildelementen umfassen,

  o wobei das Modell des maschinellen Lernens konfiguriert ist und trainiert wurde, für jedes Referenzobjekt auf Basis der mindestens einen radiologischen Eingabe-Referenzaufnahme eine synthetische radiologische Referenzaufnahme zu erzeugen,

    ▪ wobei die synthetische radiologische Referenzaufnahme eine Vielzahl an Bildelementen umfasst, wobei jedes Bildelement der synthetischen radiologischen Referenzaufnahme mit jeweils einem Bildelement der radiologischen Ziel-Referenzaufnahme korrespondiert,

    ▪ wobei das Modell des maschinellen Lernens trainiert wurde, für jedes Bildelement der synthetischen radiologischen Referenzaufnahme einen Farbwert und einen Unsicherheitswert für den vorhergesagten Farbwert vorherzusagen,

    ▪ wobei das Trainieren ein Minimieren einer Fehlerfunktion umfasst, wobei die Fehlerfunktion (i) den vorhergesagten Farbwert und/oder eine Abweichung des vorhergesagten Farbwerts von einem Farbwert des korrespondierenden Bildelements der radiologischen Ziel-Referenzaufnahme und (ii) den vorhergesagten Unsicherheitswert als Parameter umfasst,

- Empfangen oder Erzeugen mindestens einer radiologischen Eingabe-Aufnahme eines Untersuchungsbereichs eines Untersuchungsobjekts, wobei die mindestens eine radiologische Eingabe-Aufnahme den Untersuchungsbereich des Untersuchungsobjekts in dem ersten Zustand repräsentiert,

- Zuführen der mindestens einen radiologischen Eingabe-Aufnahme dem trainierten Modell des maschinellen Lernens,
- Empfangen einer synthetischen radiologischen Aufnahme von dem trainierten Modell des maschinellen Lernens, wobei die synthetische radiologische Aufnahme den Untersuchungsbereich des Untersuchungsobjekts in dem zweiten Zustand repräsentiert,
- Empfangen eines Unsicherheitswertes für jedes Bildelement der synthetischen radiologischen Aufnahme,
- Ermitteln mindestens eines Vertrauenswertes auf Basis der empfangenen Unsicherheitswerte,
- Ausgeben des mindestens einen Vertrauenswertes.

Ein weiterer Gegenstand der vorliegenden Offenbarung ist ein Kit umfassend ein Kontrastmittel und ein Computerprogramm, das in einen Arbeitsspeicher eines Computersystems geladen werden kann und dort das Computersystem dazu veranlasst, folgende Schritte ausführen:

- Bereitstellen eines trainierten Modells des maschinellen Lernens,
  - o wobei das trainierte Modell des maschinellen Lernens anhand von Trainingsdaten trainiert wurde,
  - o wobei die Trainingsdaten für jedes Referenzobjekt einer Vielzahl von Referenzobjekten (i) mindestens eine radiologische Eingabe-Referenzaufnahme eines Referenzbereichs des Referenzobjekts in einem ersten Zustand und (ii) eine radiologische Ziel-Referenzaufnahme des Referenzbereichs des Referenzobjekts in einem zweiten Zustand umfassen,
  - o wobei der erste Zustand den Referenzbereich des Referenzobjekts in einer ersten Zeitspanne vor und/oder nach der Applikation des Kontrastmittels repräsentiert und der zweite Zustand den Referenzbereich des Referenzobjekts in einer zweiten Zeitspanne nach der Applikation des Kontrastmittels repräsentiert, und/oder der erste Zustand den Referenzbereich des Referenzobjekts vor und/oder nach der Applikation einer ersten Menge des Kontrastmittels repräsentiert und der zweite Zustand den Referenzbereich des Referenzobjekts nach der Applikation einer zweiten Menge des Kontrastmittels repräsentiert,
  - o wobei die mindestens eine radiologische Eingabe-Referenzaufnahme und die radiologische Ziel-Referenzaufnahme jeweils eine Vielzahl an Bildelementen umfassen,
  - o wobei das Modell des maschinellen Lernens konfiguriert ist und trainiert wurde, für jedes Referenzobjekt auf Basis der mindestens einen radiologischen Eingabe-Referenzaufnahme eine synthetische radiologische Referenzaufnahme zu erzeugen,
    - ▪ wobei die synthetische radiologische Referenzaufnahme eine Vielzahl an Bildelementen umfasst, wobei jedes Bildelement der synthetischen radiologischen Referenzaufnahme mit jeweils einem Bildelement der radiologischen Ziel-Referenzaufnahme korrespondiert,
    - ▪ wobei das Modell des maschinellen Lernens trainiert wurde, für jedes Bildelement der synthetischen radiologischen Referenzaufnahme einen Farbwert und einen Unsicherheitswert für den vorhergesagten Farbwert vorherzusagen,
    - ▪ wobei das Trainieren ein Minimieren einer Fehlerfunktion umfasst, wobei die Fehlerfunktion (i) den vorhergesagten Farbwert und/oder eine Abweichung des vorhergesagten Farbwerts von einem Farbwert des korrespondierenden Bildelements der radiologischen Ziel-Referenzaufnahme und (ii) den vorhergesagten Unsicherheitswert als Parameter umfasst,
- Empfangen oder Erzeugen mindestens einer radiologischen Eingabe-Aufnahme eines Untersuchungsbereichs eines Untersuchungsobjekts, wobei die mindestens eine radiologische Eingabe-Aufnahme den Untersuchungsbereich des Untersuchungsobjekts in dem ersten Zustand repräsentiert,

- Zuführen der mindestens einen radiologischen Eingabe-Aufnahme dem trainierten Modell des maschinellen Lernens,
- Empfangen einer synthetischen radiologischen Aufnahme von dem trainierten Modell des maschinellen Lernens, wobei die synthetische radiologische Aufnahme den Untersuchungsbereich des Untersuchungsobjekts in dem zweiten Zustand repräsentiert,
- Empfangen eines Unsicherheitswertes für jedes Bildelement der synthetischen radiologischen Aufnahme,
- Ermitteln mindestens eines Vertrauenswertes auf Basis der empfangenen Unsicherheitswerte,
- Ausgeben des mindestens einen Vertrauenswertes.

Weitere Gegenstände der vorliegenden Offenbarung sind in der nachfolgenden Beschreibung, den abhängigen Patentansprüchen und den Zeichnungen offenbart.

## KURZE BESCHREIBUNG DER ZEICHNUNGEN

Fig. 1 zeigt beispielhaft und schematisch ein Verfahren zum Trainieren des Modells des maschinellen Lernens.

Fig. 2 zeigt beispielhaft und schematisch ein computer-implementiertes Verfahren zum Erzeugen eines synthetischen Bildes eines Untersuchungsbereichs eines Untersuchungsobjekts.

Fig. 3 zeigt eine Ausführungsform des Verfahrens zum Trainieren des Modells des maschinellen Lernens in Form eines Ablaufschemas.

Fig. 4 zeigt schematisch eine weitere Ausführungsform des computer-implementierten Verfahrens zum Trainieren des Modells des maschinellen Lernens.

Fig. 5 zeigt eine Ausführungsform des Verfahrens zum Erzeugen mindestens eines Vertrauenswertes für ein synthetisches Bild.

Fig. 6 zeigt schematisch eine weitere Ausführungsform des computer-implementiertes Verfahren zum Erzeugen eines synthetischen Bildes eines Untersuchungsbereichs eines Untersuchungsobjekts.

Fig. 7 zeigt beispielhaft und schematisch ein Computersystem gemäß der vorliegenden Offenbarung.

Fig. 8 zeigt beispielhaft und schematisch eine weitere Ausführungsform des Computersystems der vorliegenden Offenbarung.

## AUSFÜHRLICHE BESCHREIBUNG

Die Erfindung wird im Folgenden näher erläutert, ohne zwischen den Gegenständen der vorliegenden Offenbarung (Verfahren, Computersystem, computerlesbares Speichermedium, Verwendung, Kontrastmittel zur Verwendung, Kit) zu unterscheiden. Vielmehr sollen die nachfolgenden Ausführungen sinngemäß für alle Gegenstände der Erfindung gelten, unabhängig davon, in welchem Zusammenhang (Verfahren, Computersystem, computerlesbares Speichermedium, Verwendung, Kontrastmittel zur Verwendung, Kit) sie beschrieben werden.

Wenn in der vorliegenden Beschreibung oder in den Ansprüchen Schritte in einer Reihenfolge angegeben sind, bedeutet dies nicht unbedingt, dass die Erfindung auf die angegebene Reihenfolge beschränkt ist. Vielmehr ist es denkbar, dass die Schritte auch in einer anderen Reihenfolge oder auch parallel zueinander ausgeführt werden können, es sei denn, ein Schritt baut auf einem anderen auf, was zwingend erfordert, dass der aufbauende Schritt anschließend ausgeführt wird (dies wird aber im Einzelfall klar). Die genannten Reihenfolgen sind somit bevorzugte Ausführungsformen der vorliegenden Offenbarung.

Die Erfindung wird an einigen Stellen in Bezug auf Zeichnungen näher erläutert. Dabei sind in den Zeichnungen konkrete Ausführungsformen mit konkreten Merkmalen und Merkmalskombinationen dargestellt, die in erster Linie der Veranschaulichung dienen; die Erfindung soll nicht so verstanden werden, dass sie auf die in den Zeichnungen dargestellten Merkmale und Merkmalskombinationen beschränkt ist. Ferner sollen Aussagen, die bei der Beschreibung der Zeichnungen in Bezug auf Merkmale und Merkmalskombinationen getroffen werden, allgemein gelten, das heißt auch auf andere Ausführungsformen übertragbar und nicht auf die gezeigten Ausführungsformen beschränkt sein.

Die vorliegende Offenbarung beschreibt Mittel zum Beurteilen der Vertrauenswürdigkeit eines synthetischen Bildes eines Untersuchungsbereichs eines Untersuchungsobjekts.

Unter dem Begriff "Vertrauenswürdigkeit" wird verstanden, dass eine Person, die das synthetische Bild begutachtet, darauf vertrauen kann, dass Strukturen und/oder Morphologien und/oder Texturen, die in dem synthetischen Bild dargestellt sind, auf reale Strukturen und/oder reale Morphologien und/oder reale Texturen des Untersuchungsbereichs des Untersuchungsobjekts zurückgeführt werden können und keine Artefakte sind.

Der Begriff "synthetisch" bedeutet, dass das synthetische Bild nicht das unmittelbare Ergebnis einer Messung an einem realen Untersuchungsobjekt ist, sondern künstlich erzeugt (berechnet) wurde. Dabei kann ein synthetisches Bild jedoch auf Bildaufnahmen eines realen Untersuchungsobjekts basieren, d.h. es können ein oder mehrere Bildaufnahmen eines realen Untersuchungsobjekts verwendet werden, um das synthetische Bild zu erzeugen. In der Einleitung und in der weiteren Beschreibung der vorliegenden Offenbarung sind Beispiele für synthetische Bilder beschrieben. Ein synthetisches Bild wird gemäß der vorliegenden Offenbarung von einem Modell des maschinellen Lernens erzeugt. Die Erzeugung eines synthetischen Bildes mit Hilfe eines Modells des maschinellen Lernens wird in dieser Beschreibung auch als "Vorhersage" bezeichnet. Die Begriffe "synthetisch" und "vorhergesagt" werden in dieser Offenbarung synonym verwendet. Mit anderen Worten: ein synthetisches Bild ist ein Bild, das von einem (trainierten) Modell des maschinellen Lernens auf Basis von Eingabedaten erzeugt (vorhergesagt) wird, wobei die Eingabedaten ein oder mehrere messtechnisch erzeugte Bilder umfassen können.

Das "Untersuchungsobjekt" ist vorzugsweise ein Mensch oder ein Tier, vorzugsweise ein Säugetier, ganz besonders bevorzugt ein Mensch.

Der "Untersuchungsbereich" ist ein Teil des Untersuchungsobjekts, zum Beispiel ein Organ eines Menschen oder Tieres wie beispielsweise die Leber, das Gehirn, das Herz, die Niere, die Lunge, der Magen, der Darm, die Bauchspeicheldrüse, die Schilddrüse, die Prostata, die Brust oder ein Teil der genannten Organe oder mehrere Organe oder ein anderer Teil des Untersuchungsobjekts. Der Untersuchungsbereich kann auch mehre Organe und/oder Teile von mehreren Organen umfassen.

In einer Ausführungsform umfasst der Untersuchungsbereich eine Leber oder einen Teil einer Leber oder der Untersuchungsbereich ist eine Leber oder ein Teil einer Leber eines Säugetiers, vorzugsweise eines Menschen.

In einer weiteren Ausführungsform umfasst der Untersuchungsbereich ein Gehirn oder einen Teil eines Gehirns oder der Untersuchungsbereich ist ein Gehirn oder ein Teil eines Gehirns eines Säugetiers, vorzugsweise eines Menschen.

In einer weiteren Ausführungsform umfasst der Untersuchungsbereich ein Herz oder ein Teil eines Herzes oder der Untersuchungsbereich ist ein Herz oder ein Teil eines Herzes eines Säugetiers, vorzugsweise eines Menschen.

In einer weiteren Ausführungsform umfasst der Untersuchungsbereich einen Thorax oder einen Teil eines Thorax oder der Untersuchungsbereich ist ein Thorax oder ein Teil eines Thorax eines Säugetiers, vorzugsweise eines Menschen.

In einer weiteren Ausführungsform umfasst der Untersuchungsbereich einen Magen oder einen Teil eines Magens oder der Untersuchungsbereich ist ein Magen oder ein Teil eines Magens eines Säugetiers, vorzugsweise eines Menschen.

In einer weiteren Ausführungsform umfasst der Untersuchungsbereich eine Bauchspeicheldrüse oder einen Teil einer Bauchspeicheldrüse oder der Untersuchungsbereich ist eine Bauchspeicheldrüse oder ein Teil einer Bauchspeicheldrüse eines Säugetiers, vorzugsweise eines Menschen.

In einer weiteren Ausführungsform umfasst der Untersuchungsbereich eine Niere oder einen Teil einer Niere oder der Untersuchungsbereich ist eine Niere oder ein Teil einer Niere eines Säugetiers, vorzugsweise eines Menschen.

In einer weiteren Ausführungsform umfasst der Untersuchungsbereich einen oder beide Lungenflügel oder einen Teil eines Lungenflügels Säugetiers, vorzugsweise eines Menschen.

In einer weiteren Ausführungsform umfasst der Untersuchungsbereich eine Brust oder einen Teil einer Brust oder der Untersuchungsbereich ist eine Brust oder ein Teil einer Brust eines weiblichen Säugetiers, vorzugsweise eines weiblichen Menschen.

In einer weiteren Ausführungsform umfasst der Untersuchungsbereich eine Prostata oder einen Teil einer Prostata oder der Untersuchungsbereich ist eine Prostata oder ein Teil einer Prostata eines männlichen Säugetiers, vorzugsweise eines männlichen Menschen.

Der Untersuchungsbereich, auch Aufnahmevolumen (engl.: *field of view,* FOV) genannt, stellt insbesondere ein Volumen dar, welches in radiologischen Aufnahmen abgebildet wird. Der Untersuchungsbereich wird typischerweise durch einen Radiologen, beispielsweise auf einer Übersichtsaufnahme festgelegt. Selbstverständlich kann der Untersuchungsbereich alternativ oder zusätzlich auch automatisch, beispielsweise auf Grundlage eines ausgewählten Protokolls, festgelegt werden.

Der Begriff "Bild" bezeichnet eine Datenstruktur, die eine räumliche Verteilung eines physikalischen Signals darstellt. Die räumliche Verteilung kann eine beliebige Dimension haben, z.B. 2D, 3D, 4D oder eine höhere Dimension. Die räumliche Verteilung kann eine beliebige Form haben, z.B. ein Gitter bilden und dadurch Pixel oder Voxel definieren, wobei das Gitter unregelmäßig oder regelmäßig sein kann. Das physikalische Signal kann ein beliebiges Signal sein, z.B. Protonendichte, Echogenität, Durchlässigkeit, Absorptionsvermögen, Relaxivität, Informationen über rotierende Wasserstoffkeme in einem Magnetfeld, Farbe, Graustufe, Tiefe, Oberflächen- oder Volumenbelegung.

Unter dem Begriff "Bild" wird vorzugsweise eine zwei-, drei- oder höherdimensionale visuell erfassbare Repräsentation des Untersuchungsbereichs des Untersuchungsobjekts verstanden. Üblicherweise handelt es sich bei einem solchen Bild um ein digitales Bild. Der Begriff "digital" bedeutet, dass das Bild von einer Maschine, in der Regel einem Computersystem, verarbeitet werden kann. Unter "Verarbeitung" werden die bekannten Verfahren zur elektronischen Datenverarbeitung (EDV) verstanden.

Ein digitales Bild kann mit Computersystemen und Software verarbeitet, bearbeitet und reproduziert sowie in standardisierte Datenformate konvertiert werden, wie zum Beispiel JPEG (Grafikformat der Joint Photographic Experts Group), PNG (Portable Network Graphics) oder SVG (Scalable Vector Graphics). Digitale Bilder können mit geeigneten Anzeigegeräten visualisiert werden, wie zum Beispiel mit Computermonitoren, Projektoren und/oder Druckern.

In einem digitalen Bild werden Bildinhalte üblicherweise durch ganze Zahlen repräsentiert und gespeichert. In den meisten Fällen handelt es sich um zwei- oder dreidimensionale Bilder, die binär kodiert und gegebenenfalls komprimiert sein können. Bei den digitalen Bildern handelt es sich üblicherweise um Rastergrafiken, bei denen die Bildinformation in einer gleichmäßigen Rasterung abgelegt ist. Rastergrafiken bestehen aus einer rasterförmigen Anordnung von so genannten Bildpunkten (Pixel) im Fall von zweidimensionalen Darstellungen oder Volumenelementen (Voxel) im Fall dreidimensionaler Darstellungen. Bei vierdimensionalen Darstellungen wird häufig der Begriff Doxel (*dynamic voxel*) für die Bildelemente verwendet. Bei höherdimensionalen Darstellungen oder allgemein wird im Englischen manchmal auch der Begriff "n-xel" verwendet, wobi n die jeweilige Dimension angibt. In dieser Offenbarung wird allgemein der Begriff Bildelement verwendet. Ein Bildelement kann also ein Bildpunkt (Pixel) im Fall einer zweidimensionalen Darstellung, ein Volumenelement (Voxel) im Fall einer dreidimensionalen Darstellung, ein dynamisches Voxel (Doxel) im Fall der vierdimensionalen Darstellung oder ein höherdimensionales Bildelement im Fall einer höherdimensionalen Darstellung sein.

Jedem Bildelement eines Bildes ist ein Farbwert zugeordnet. Der Farbwert gibt an, wie (z.B. in welcher Farbe) das Bildelement visuell dargestellt werden soll (z.B. auf einem Monitor).

Der einfachste Fall ist ein Binärbild, bei dem ein Bildelement entweder weiß oder schwarz dargestellt wird. Üblicherweise steht der Farbwert "0" für "schwarz" und der Farbwert "1" für "weiß".

Bei einem Graustufenbild ist jedem Bildelement eine Graustufe zugeordnet, die von schwarz über eine definierte Zahl von Grauabstufungen bis weiß reicht. Die Graustufen werden auch als Grauwerte bezeichnet. Die Zahl der Abstufungen kann beispielsweise von 0 bis 255 reichen (also 256 Graustufen/Grauwerte umfassen), wobei auch hier der Wert "0" üblicherweise für "schwarz" steht und der höchste Grauwert (in dem vorliegenden Beispiel der Wert 255) für "weiß" steht.

Bei einem Farbbild definiert sich die für ein Bildelement verwendete Kodierung der Farbe unter anderem über den Farbraum und die Farbtiefe. Bei einem Bild, dessen Farbe über den so genannten RGB-Farbraum definiert ist (RGB steht für die Grundfarben Rot, Grün und Blau), sind jedem Bildpunkt drei Farbwerte zugeordnet, ein Farbwert für die Farbe Rot, ein Farbwert für die Farbe Grün und ein Farbwert für die Farbe Blau. Die Farbe eines Bildelements ergibt sich durch Überlagerung (additives Mischen) der drei Farbwerte. Der einzelne Farbwert kann z.B. in 256 unterscheidbare Stufen diskretisiert sein, die Tonwerte genannt werden und üblicherweise von 0 bis 255 reichen. Der Tonwert "0" eines jeden Farbkanals ist üblicherweise die dunkelste Farbnuance. Haben alle drei Farbkanäle den Tonwert 0, erscheint das entsprechende Bildelement schwarz; haben alle drei Farbkanäle den Tonwert 255, erscheint das entsprechende Bildelement weiß.

Unabhängig davon, ob es sich um ein Binärbild, ein Graustufenbild oder ein Farbbild handelt, wird in dieser Offenbarung der Begriff "Farbwert" für die Information verwendet, in welcher Farbe (inkl. den "Farben" "schwarz" und "weiß" sowie allen Grautönen) ein Bildelement dargestellt werden soll. Ein Farbwert kann also beispielsweise ein Tonwert eines Farbkanals sein, ein Grauton sein oder für "schwarz" oder für "weiß" stehen.

Ein Farbwert in einem Bild (insbesondere einer medizinischen Aufnahme) repräsentiert üblicherweise eine Stärke eines physikalischen Signals (s.o.). Es sei angemerkt, dass der "Farbwert" auch ein Wert für das physikalische Signal selbst sein kann.

Es gibt eine Vielzahl an möglichen digitalen Bildformaten und Farbkodierungen. Vereinfachend wird in dieser Beschreibung davon ausgegangen, dass die vorliegenden Bilder Rastergrafiken mit einer spezifischen Zahl an Bildelementen sind. Diese Annahme soll jedoch in keiner Weise limitierend verstanden werden. Dem Fachmann der Bildbearbeitung ist klar, wie er die Lehre dieser Beschreibung auf Bilddateien, die in anderen Bildformaten vorliegen und/oder bei denen die Farbwerte anders kodiert sind, übertragen kann.

Bei einem "Bild" im Sinne der vorliegenden Offenbarung kann es sich auch um einen oder mehrere Ausschnitte

aus einer Videosequenz handeln.

In einem ersten Schritt wird mindestens ein Eingabe-Bild eines Untersuchungsbereichs eines Untersuchungsobjekts empfangen.

Der Begriff "Empfangen" umfasst sowohl das Abrufen von Bildern als auch das Entgegennehmen von Bildern, die z.B. an das Computersystem der vorliegenden Offenbarung übermittelt werden. Das mindestens eine erste Bild kann von einem Computertomografen, von einem Magnetresonanztomografen, von einem Ultraschall-Scanner, von einer Kamera und/oder von einem anderen Gerät zur Erzeugung von Bildern empfangen werden. Das mindestens eine erste Bild kann aus einem Datenspeicher ausgelesen und/oder von einem separaten Computersystem übermittelt werden.

Der Ausdruck "Empfangen mindestens eines Eingabe-Bildes" bedeutet, dass ein oder mehrere Eingabe-Bilder empfangen werden. Es kann also ein Eingabe-Bild empfangen werden; es können aber auch zwei oder drei oder vier oder mehr als vier Eingabe-Bilder empfangen werden.

Der Zusatz "Eingabe-" in dem Begriff "Eingabe-Bild" weist darauf hin, dass das Eingabe-Bild bestimmt ist, einem (trainierten) Modell des maschinellen Lernens als Eingabedaten oder zumindest als ein Teil der Eingabedaten zugeführt zu werden.

Vorzugsweise handelt es sich bei dem mindestens einen Eingabe-Bild um eine zweidimensionale oder dreidimensionale Repräsentation eines Untersuchungsbereichs eines Untersuchungsobjekts.

In einer Ausführungsform der vorliegenden Offenbarung handelt es sich bei dem mindestens einen Eingabe-Bild um eine medizinische Aufnahme.

Eine "medizinische Aufnahme" ist eine visuelle Repräsentation eines Untersuchungsbereichs eines Menschen oder eines Tieres, die für diagnostische und/oder therapeutische Zwecke verwendet werden kann.

Es gibt eine Vielzahl an Techniken, mit denen medizinische Aufnahmen erzeugt werden können; Beispiele solcher Techniken sind Röntgen, Computertomografie (CT), Fluoroskopie, Magnetresonanztomografie (MRT), Ultraschall (Sonografie), Endoskopie, Elastografie, taktile Bildgebung, Thermografie, Mikroskopie, Positronenemissionstomografie, optische Kohärenztomografie (OCT), Fundusfotografie und andere.

Beispiele für medizinische Aufnahmen sind CT-Aufnahmen, Röntgenbilder, MRT-Aufnahmen, Fluoreszenzangiografie-Bilder, OCT-Aufnahmen, histologische Aufnahmen, Ultraschallbilder, Fundusbilder und/oder andere.

Das mindestens eine Eingabe-Bild kann eine CT-Aufnahme, MRT-Aufnahme, Ultraschallaufnahme, OCT-Aufnahme, und/oder eine andere Repräsentation eines Untersuchungsbereichs eines Untersuchungsobjekts sein.

Das mindestens eine Eingabe-Bild kann auch Repräsentationen unterschiedlicher Modalitäten umfassen, z.B. eine oder mehrere CT-Aufnahme und eine oder mehrere MRT-Aufnahmen.

Vorzugsweise ist das mindestens eine Eingabe-Bild das Ergebnis einer radiologischen Untersuchung. Mit anderen Worten: Vorzugsweise ist das mindestens eine Eingabe-Bild eine radiologische Aufnahme.

Die "Radiologie" ist das Teilgebiet der Medizin, das sich mit der Anwendung elektromagnetischer Strahlen und (unter Einbezug etwa der Ultraschalldiagnostik) mechanischer Wellen zu diagnostischen, therapeutischen und/oder wissenschaftlichen Zwecken befasst. Neben Röntgenstrahlen kommen auch andere ionisierende Strahlung wie Gammastrahlung oder Elektronen zum Einsatz. Da ein wesentlicher Einsatzzweck die Bildgebung ist, werden auch andere bildgebende Verfahren wie die Sonographie und die Magnetresonanztomographie (Kernspintomographie) zur Radiologie gerechnet, obwohl bei diesen Verfahren keine ionisierende Strahlung zum Einsatz kommt. Der Begriff "Radiologie" im Sinne der vorliegenden Offenbarung umfasst damit insbesondere die folgenden Untersuchungsmethoden: Computertomographie, Magnetresonanztomographie, Sonographie.

In einer Ausführungsform der vorliegenden Offenbarung handelt es sich bei der radiologischen Untersuchung um eine magnetresonanztomographische Untersuchung, d.h., das mindestens eine Eingabe-Bild umfasst mindestens eine MRT-Aufnahme.

In einer weiteren Ausführungsform handelt es sich bei der radiologischen Untersuchung um eine computertomographische Untersuchung, d.h., das mindestens eine Eingabe-Bild umfasst mindestens eine CT-Aufnahme.

In einer weiteren Ausführungsform handelt es sich bei der radiologischen Untersuchung um eine Ultraschalluntersuchung, d.h., das mindestens eine Eingabe-Bild umfasst mindestens eine UltraschallAufnahme.

Bei radiologischen Untersuchungen werden häufig Kontrastmittel zur Kontrastverstärkung eingesetzt.

"Kontrastmittel" sind Substanzen oder Gemische von Substanzen, die die Darstellung von Strukturen und Funktionen des Körpers bei radiologischen Untersuchungen verbessern.

In der Computertomographie werden meist iodhaltige Lösungen als Kontrastmittel eingesetzt. In der Magnetresonanztomographie (MRT) werden üblicherweise superparamagnetische Substanzen (z.B. Eisenoxidnanopartikel, superparamagnetische Eisen-Platin-Partikel (SIPPs)) oder paramagnetische Substanzen (z.B. Gadolinium-Chelate, Mangan-Chelate, Hafnium-Chelate) als Kontrastmittel verwendet. Im Fall der Sonographie werden üblicherweise Flüssigkeiten, die gasgefüllte Mikrobläschen (*microbubbles*) enthalten, intravenös verabreicht. Beispiele für Kontrastmittel sind in der Literatur zu finden (siehe z.B. A. S. L. Jascinth et al.: Contrast Agents in computed tomography: A Review, Journal of Applied Dental and Medical Sciences, 2016, Vol. 2, Issue 2, 143 - 149; H. Lusic et al.: X-ray-Computed Tomography Contrast Agents, Chem. Rev. 2013, 113, 3, 1641-1666; https://www.radiology.wisc.edu/wp-content/u

ploads/2017/10/contrast-agents-tutorial.pdf, M. R. Nough et al.: Radiographie and magnetic resonances contrast agents: Essentials and tips for safe practices, World J Radiol. 2017 Sep 28; 9(9): 339-349; L. C. Abonyi et al.: Intravascular Contrast Media in Radiography: Historical Development & Review of Risk Factors for Adverse Reactions, South American Journal of Clinical Research, 2016, Vol. 3, Issue 1, 1-10; ACR Manual on Contrast Media, 2020, ISBN: 978-1-55903-012-0; A. Ignee et al.: Ultrasound contrast agents, Endosc Ultrasound. 2016 Nov-Dec; 5(6): 355-362).

MRT-Kontrastmittel entfalten in einer MRT-Untersuchung ihre Wirkung, indem sie die Relaxationszeiten der Strukturen, die Kontrastmittel aufnehmen, verändern. Es lassen sich zwei Stoffgruppen unterscheiden: para- und superparamagnetische Stoffe. Beide Stoffgruppen besitzen ungepaarte Elektronen, die ein magnetisches Feld um die einzelnen Atome bzw. Moleküle induzieren. Superparamagnetische führen zu einer überwiegenden T2-Verkürzung, während paramagnetische Kontrastmittel im Wesentlichen zu einer T1-Verkürzung führen. Die Wirkung dieser Kontrastmittel ist indirekt, da das Kontrastmittel selbst kein Signal abgibt, sondern nur die Signalintensität in seiner Umgebung beeinflusst. Ein Beispiel für ein superparamagnetisches Kontrastmittel sind Eisenoxidnanopartikel (SPIO, engl.: *superparamagnetic iron oxide*). Beispiele für paramagnetische Kontrastmittel sind Gadolinium-Chelate wie Gadopentetat-Dimeglumin (Handelsname: Magnevist® u.a.), Gadotersäure (Dotarem®, Dotagita®, Cyclolux®), Gadodiamid (Omniscan®), Gadoteridol (ProHance®), Gadobutrol (Gadovist®), Gadopiclenol (Elucirem, Vueway) und Gadoxetsäure (Primovist®/Eovist®).

In einer Ausführungsform handelt es sich bei der radiologischen Untersuchung um eine MRT-Untersuchung, bei der ein MRT-Kontrastmittel eingesetzt wird.

In einer weiteren Ausführungsform handelt es sich bei der radiologischen Untersuchung um eine CT-Untersuchung, bei der ein CT-Kontrastmittel eingesetzt wird.

In einer weiteren Ausführungsform handelt es sich bei der radiologischen Untersuchung um eine CT-Untersuchung, bei der ein MRT-Kontrastmittel eingesetzt wird.

In einer Ausführungsform der vorliegenden Offenbarung handelt es sich bei dem Kontrastmittel um ein Mittel, das Gadolinium(III) 2-[4,7,10-tris(carboxymethyl)-1,4,7,10-tetrazacyclododec-1-yl]essigsäure (auch als Gadolinium-DOTA oder Gadotersäure bezeichnet) umfasst.

In einer weiteren Ausführungsform handelt es sich bei dem Kontrastmittel um ein Mittel, das Gadolinium(III) Ethoxybenzyl-diethylenetriaminepentaessigsäure (Gd-EOB-DTPA) umfasst; vorzugsweise umfasst das Kontrastmittel das Dinatriumsalz der Gadolinium(III)-ethoxybenzyl-diethylenetriaminpentaessigsäure (auch als Gadoxetsäure bezeichnet).

In einer Ausführungsform der vorliegenden Offenbarung handelt es sich bei dem Kontrastmittel um ein Mittel, das Gadolinium(III) 2-[3,9-bis[1-carboxylato-4-(2,3-dihydroxypropylamino)-4-oxobutyl]-3,6,9,15-tetrazabicyclo[9.3.1]pentadeca-1(15),11,13-trien-6-yl]-5-(2,3-dihydroxypropylamino)-5-oxopentanoat (auch als Gadopiclenol bezeichnet), umfasst (siehe z.B. WO2007/042504 sowie WO2020/030618 und/oder WO2022/013454).

In einer Ausführungsform der vorliegenden Offenbarung handelt es sich bei dem Kontrastmittel um ein Mittel, das Dihydrogen[(±)-4-carboxy-5,8,11-tris(carboxymethyl)-1-phenyl-2-oxa-5,8,11-triazatridecan-13-oato(5-)]gadolinat(2-) (auch als Gadobensäure bezeichnet) umfasst.

In einer Ausführungsform der vorliegenden Offenbarung handelt es sich bei dem Kontrastmittel um ein Mittel, das Tetragadolinium-[4,10-bis(carboxylatomethyl)-7-{3,6,12,15-tetraoxo-16-[4,7,10-tris-(carboxylatomethyl)-1,4,7,10-tetraazacyclododecan-1 -yl]-9,9-bis({[({ 2-[4,7,10-tris-(carboxylatomethyl)-1,4,7,10-tetraazacyclododecan-1-yl]propanoyl}amino)acetyl]-amino}methyl)-4,7,11,14-tetraazahepta-decan-2-yl}-1,4,7,10-tetraazacyclododecan-1-yl]acetat (auch als Gadoquatrane bezeichnet) umfasst (siehe z.B. J. Lohrke et al.: Preclinical Profile of Gadoquatrane: A Novel Tetramerie, Macrocyclic High Relaxivity Gadolinium-Based Contrast Agent. Invest Radiol., 2022, 1, 57(10): 629-638; WO2016193190).

In einer Ausführungsform der vorliegenden Offenbarung handelt es sich bei dem Kontrastmittel um ein Mittel, das einen $Gd^{3+}$-Komplex einer Verbindung der Formel (I)

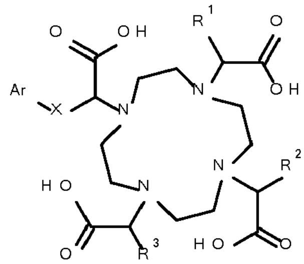

(I) ,

umfasst, wobei

Ar eine Gruppe ausgewählt aus

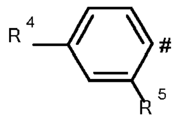

und

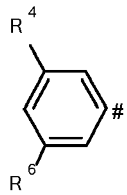

darstellt,

wobei # die Anknüpfung zu X darstellt,

X eine Gruppe darstellt, die aus

$CH_2$, $(CH_2)_2$, $(CH_2)_3$, $(CH_2)_4$ und $*\text{-}(CH_2)_2\text{-}O\text{-}CH_2\text{-}^{\#}$ ausgewählt wird,

wobei * die Anknüpfung zu Ar darstellt und # die Anknüpfung zum Essigsäurerest darstellt,

$R^1$, $R^2$ and $R^3$ unabhängig voneinander ein Wasserstoffatom oder eine Gruppe ausgewählt aus $C_1$-$C_3$-Alkyl, $-CH_2OH$, $-(CH_2)_2OH$ und $-CH_2OCH_3$ darstellen,

$R^4$ eine Gruppe ausgewählt aus $C_2$-$C_4$-Alkoxy, $(H_3C\text{-}CH_2)\text{-}O\text{-}(CH_2)_2\text{-}O\text{-}$, $(H_3C\text{-}CH_2)\text{-}O\text{-}(CH_2)_2\text{-}O\text{-}(CH_2)_2\text{-}O\text{-}$ und $(H_3C\text{-}CH_2)\text{-}O\text{-}(CH_2)_2\text{-}O\text{-}(CH_2)_2\text{-}O\text{-}(CH_2)_2\text{-}O\text{-}$ darstellt,

$R^5$ ein Wasserstoffatom darstellt,
und

$R^6$ ein Wasserstoffatom darstellt,

oder ein Stereoisomer, Tautomer, Hydrat, Solvat oder Salz davon, oder eine Mischung davon.

In einer Ausführungsform der vorliegenden Offenbarung handelt es sich bei dem Kontrastmittel um ein Mittel, das einen $Gd^{3+}$-Komplex einer Verbindung der Formel (II)

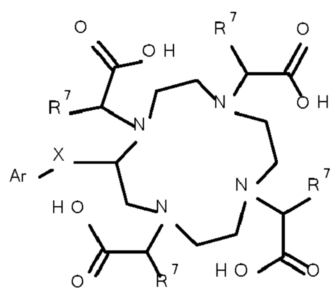

(II) ,

umfasst, wobei

Ar eine Gruppe ausgewählt aus

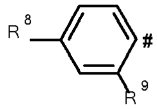 and 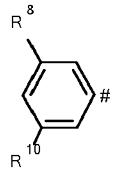

darstellt,

wobei # die Anknüpfung zu X darstellt,

X eine Gruppe darstellt, die aus $CH_2$, $(CH_2)_2$, $(CH_2)_3$, $(CH_2)_4$ and $*-(CH_2)_2-O-CH_2-^{\#}$ ausgewählt wird, wobei * die Anknüpfung zu Ar darstellt und # die Anknüpfung zum Essigsäurerest darstellt,

$R^7$ ein Wasserstoffatom oder eine Gruppe ausgewählt aus Ci-Cs-Alkyl, $-CH_2OH$, $-(CH_2)_2OH$ und $-CH_2OCH_3$ darstellt;

$R^8$ eine Gruppe ausgewählt aus $C_2$-$C_4$-Alkoxy, $(H_3C-CH_2O)-(CH_2)_2-O-$, $(H_3C-CH_2O)-(CH_2)_2-O-(CH_2)_2-O-$ und $(H_3C-CH_2O)-(CH_2)_2-O-(CH_2)_2-O-(CH_2)_2-O-$ darstellt;

$R^9$ und $R^{10}$ unabhängig voneinander ein Wasserstoffatom darstellen;

oder ein Stereoisomer, Tautomer, Hydrat, Solvat oder Salz davon, oder eine Mischung davon.

Der Begriff "$C_1$-$C_3$-Alkyl" bedeutet eine lineare oder verzweigte, gesättigte, einwertige Kohlenwasserstoffgruppe mit 1, 2 oder 3 Kohlenstoffatomen, z.B. Methyl, Ethyl, n-Propyl und Isopropyl. Der Begriff "$C_2$-$C_4$-Alkyl" bedeutet eine lineare oder verzweigte, gesättigte, einwertige Kohlenwasserstoffgruppe mit 2, 3 oder 4 Kohlenstoffatomen.

Der Begriff "$C_2$-$C_4$-Alkoxy" bedeutet eine lineare oder verzweigte, gesättigte, einwertige Gruppe der Formel ($C_2$-$C_4$-Alkyl)-O-, in der der Begriff "$C_2$-$C_4$-Alkyl" wie oben definiert ist, z. B. ein Methoxy, Ethoxy, n-Propoxy oder Isopropoxygruppe.

In einer Ausführungsform der vorliegenden Offenbarung handelt es sich bei dem Kontrastmittel um ein Mittel, das Gadolinium 2,2',2"-(10-{1-carboxy-2-[2-(4-ethoxyphenyl)ethoxy]ethyl}-1,4,7,10-tetraazacyclododecan-1,4,7-triyl) triacetat umfasst (siehe z.B. WO2022/194777, Beispiel 1).

In einer Ausführungsform der vorliegenden Offenbarung handelt es sich bei dem Kontrastmittel um ein Mittel, das Gadolinium 2,2',2"-{10-[1-carboxy-2-{4-[2-(2-ethoxyethoxy)ethoxy]phenyl}ethyl]-1,4,7,10-tetraazacyclododecan-1,4,7-triyl}triacetat umfasst (siehe z.B. WO2022/194777, Beispiel 2).

In einer Ausführungsform der vorliegenden Offenbarung handelt es sich bei dem Kontrastmittel um ein Mittel, das Gadolinium 2,2',2"-{10-[(1R)-1-carboxy-2-{4-[2-(2-ethoxyethoxy)ethoxy]phenyl}ethyl]-1,4,7,10-tetraazacyclododecan-1,4,7-triyl}triacetat umfasst (siehe z.B. WO2022/194777, Beispiel 4).

In einer Ausführungsform der vorliegenden Offenbarung handelt es sich bei dem Kontrastmittel um ein Mittel, das Gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecan-1,4,7-triyl}tris(3-hydroxypropanoat) umfasst (siehe z.B. WO2022/194777, Beispiel 15).

In einer Ausführungsform der vorliegenden Offenbarung handelt es sich bei dem Kontrastmittel um ein Mittel, das Gadolinium 2,2',2"-{10-[(1S)-4-(4-butoxyphenyl)-1-carboxybutyl]-1,4,7,10-tetraazacyclododecan-1,4,7-triyl}triacetat umfasst (siehe z.B. WO2022/194777, Beispiel 31).

In einer Ausführungsform der vorliegenden Offenbarung handelt es sich bei dem Kontrastmittel um ein Mittel, das Gadolinium-2,2',2"-{(2*S*)-10-(carboxymethyl)-2-[4-(2-ethoxyethoxy)benzyl]-1,4,7,10-tetraazacyclododecane-1,4,7-triyl}triacetat umfasst.

In einer Ausführungsform der vorliegenden Offenbarung handelt es sich bei dem Kontrastmittel um ein Mittel, das Gadolinium-2,2',2"-[10-(carboxymethyl)-2-(4-ethoxybenzyl)-1,4,7,10-tetraazacyclododecane-1,4,7-triyl]triacetat umfasst.

In einer Ausführungsform der vorliegenden Offenbarung handelt es sich bei dem Kontrastmittel um ein Mittel, das Gadolinium(III) 5,8-bis(carboxylatomethyl)-2-[2-(methylamino)-2-oxoethyl]-10-oxo-2,5,8,11-tetraazadodecan-1-carboxylat-Hydrat (auch als Gadodiamid bezeichnet) umfasst.

In einer Ausführungsform der vorliegenden Offenbarung handelt es sich bei dem Kontrastmittel um ein Mittel, das Gadolinium(III) 2-[4-(2-hydroxypropyl)-7,10-bis(2-oxido-2-oxoethyl)-1,4,7,10-tetrazacyclododec-1-yl]acetat (auch als Gadoteridol bezeichnet) umfasst.

In einer Ausführungsform der vorliegenden Offenbarung handelt es sich bei dem Kontrastmittel um ein Mittel, das Gadolinium(III) 2,2',2"-(10-((2R,3S)-1,3,4-trihydroxybutan-2-yl)-1,4,7,10-tetraazacyclododecan-1,4,7-triyl)triacetat

(auch als Gadobutrol oder Gd-DO3A-butrol bezeichnet) umfasst.

Das mindestens eine Eingabe-Bild kann auch Repräsentationen des Untersuchungsbereichs umfassen, die unter unterschiedlichen Messbedingungen erzeugt wurden, z.B. eine T1-gewichtete MRT-Aufnahme und/oder eine T2-gewichtete MRT-Aufnahme und/oder eine Diffusions-gewichtete MRT-Aufnahme und/oder eine andere MRT-Aufnahme und/oder eine oder mehrere Dual-Energy-CT-Aufnahme(n) und/oder eine oder mehrere Spectral-CT-Aufnahme(n).

Das mindestens eine Eingabe-Bild kann auch mehrere radiologische Aufnahmen umfassen, die nach Applikation unterschiedlicher Mengen eines Kontrastmittels und/oder nach Applikation unterschiedlicher Kontrastmittel erzeugt wurden, wie beispielsweise ein native radiologische Aufnahme und/oder eine radiologische Aufnahme nach Applikation einer ersten Menge eines Kontrastmittels und/oder eine oder mehrere radiologische Aufnahmen nach Applikation eines zweiten Kontrastmittels und/oder eine virtuelle Nicht-Kontrastmittel-Repräsentation (VNC-Repräsentation).

Das mindestens eine Eingabe-Bild kann auch mehrere radiologische Aufnahmen umfassen, die zu unterschiedlichen Zeitpunkten vor und/oder nach der Applikation eines oder mehrerer Kontrastmittel erzeugt wurden und/oder die den Untersuchungsbereich in unterschiedlichen Phasen und/oder Zuständen repräsentieren.

Das mindestens eine Eingabe-Bild umfasst eine Vielzahl an Bildelementen. Jedes Bildelement der Vielzahl an Bildelementen repräsentiert einen Teilbereich des Untersuchungsbereichs des Untersuchungsobjekts. Der Begriff "Vielzahl an Bildelementen" bedeutet mindestens 1000, vorzugsweise mindestens 10000, noch mehr bevorzugt mehr als 100000. Es ist denkbar, dass das mindestens eine Eingabe-Bild ein oder mehrere Bildelemente umfasst, die nicht den Untersuchungsbereich des Untersuchungsobjekts repräsentieren, sondern einen anderen Bereich wie beispielsweise einen angrenzenden und/oder umgebenden Bereich.

Das mindestens eine Eingabe-Bild repräsentiert den Untersuchungsbereich des Untersuchungsobjekts in einem ersten Zustand.

Falls mehr als ein Eingabe-Bild empfangen werden (z.B. zwei oder drei oder vier oder mehr als vier), so können die weiteren empfangenen Eingabe-Bilder den Untersuchungsbereich des Untersuchungsobjekts in demselben Zustand repräsentieren wie das erste Eingabe-Bild oder in einem oder mehreren weiteren Zustände. Ein erstes Eingabe-Bild kann den Untersuchungsbereich des Untersuchungsobjekts beispielsweise in einem ersten Zustand repräsentieren und ein zweites Eingabe-Bild kann den Untersuchungsbereich des Untersuchungsobjekts beispielsweise in einem zweiten Eingabe-Zustand repräsentieren, wobei sich der zweite Zustand von dem ersten Zustand unterscheidet. Auf Basis des mindestens einen Eingabe-Bildes wird von dem (trainierten) Modell des maschinellen Lernens ein synthetisches Bild erzeugt. Das synthetische Bild repräsentiert den Untersuchungsbereich des Untersuchungsobjekts in einem zweiten Zustand. Der erste Zustand und der zweite Zustand sind unterschiedliche Zustände.

Wenn mehr als ein erstes Eingabe-Bild empfangen wird und auf Basis der mehreren empfangenen Eingabe-Bildern ein synthetisches Bild erzeugt wird, dann repräsentiert das synthetische Bild den Untersuchungsbereich des Untersuchungsobjekts in einem anderen Zustand als jedes der empfangenen Eingabe-Bilder auf deren Basis das synthetische Bild erzeugt wird.

Es können also ein erstes Eingabe-Bild und ein zweites Eingabe-Bild empfangen werden. Das erste Eingabe-Bild kann den Untersuchungsbereich des Untersuchungsobjekts in einem ersten Zustand repräsentieren und das zweite Eingabe-Bild kann den Untersuchungsbereich des Untersuchungsobjekts in einem zweiten Zustand repräsentieren. Der erste und der zweite Zustand können gleich oder verschieden sein; vorzugsweise sind sie verschieden. Auf Basis des ersten Eingabe-Bildes und des zweiten Eingabe-Bildes kann ein synthetisches Bild erzeugt werden. Das synthetische Bild repräsentiert den Untersuchungsbereich des Untersuchungsobjekts in einem dritten Zustand; der dritte Zustand unterscheidet sich von dem ersten Zustand und von dem zweiten Zustand.

Es können auch ein erstes Eingabe-Bild, ein zweites Eingabe-Bild und ein drittes Eingabe-Bild empfangen werden. Das erste Eingabe-Bild kann den Untersuchungsbereich des Untersuchungsobjekts in einem ersten Zustand repräsentieren; das zweite Eingabe-Bild kann den Untersuchungsbereich des Untersuchungsobjekts in einem zweiten Zustand repräsentieren; das dritte Eingabe-Bild kann den Untersuchungsbereich des Untersuchungsobjekts in einem dritten Zustand repräsentieren. Der erste, zweite und der dritte Zustand können gleich oder verschieden sein; vorzugsweise sind sie verschieden. Auf Basis des ersten Eingabe-Bildes, des zweiten Eingabe-Bildes und des dritten Eingabe-Bildes kann ein synthetisches Bild erzeugt werden. Das synthetische Bild repräsentiert den Untersuchungsbereich des Untersuchungsobjekts in einem vierten Zustand; der vierte Zustand unterscheidet sich von dem ersten Zustand, von dem zweiten Zustand und von dem dritten Zustand.

Allgemein kann eine Zahl $m$ an Eingabe-Bildern empfangen werden (wobei $m$ eine ganze Zahl größer als Null ist); jedes empfangene Eingabe-Bild repräsentiert den Untersuchungsbereich des Untersuchungsobjekts in einem von $p$ Zuständen, wobei $p$ eine ganze Zahl ist, die die Werte von 1 bis $m$ annehmen kann. Auf Basis der $m$ empfangenen Eingabe-Bildern kann ein synthetisches Bild erzeugt werden, das den Untersuchungsbereich des Untersuchungsobjekts in einem Zustand repräsentiert, der sich von den $p$ Zuständen unterscheidet.

Der Zustand kann eine Menge an Kontrastmittel sein, die dem Untersuchungsobjekt verabreicht worden ist. Der Zustand kann eine Zeitspanne sein, die seit dem Applizieren eines Kontrastmittels vergangen ist. Der Zustand kann ein

spezifisches Kontrastmittel sein, das dem Untersuchungsobjekt verabreicht worden ist. Der Zustand kann auch ein Zustand ohne Kontrastmittel sein. Der Zustand kann eine Modalität (z.B. MRT-Aufnahme, CT-Aufnahme, Ultraschallaufnahme) oder ein Messprotokoll sein, das zu einem spezifischen Aussehen des Untersuchungsbereichs in einer messtechnisch erzeugten medizinischen Aufnahme führt.

Das mindestens eine Eingabe-Bild kann beispielsweise eine radiologische Aufnahme des Untersuchungsbereichs vor und/oder nach einer Applikation einer ersten Menge eines Kontrastmittels umfassen und das synthetische Bild kann eine synthetische radiologische Aufnahme des Untersuchungsbereichs nach der Applikation einer zweiten Menge eines Kontrastmittels sein, wobei die zweite Menge vorzugsweise größer als die erste Menge ist.

Das mindestens eine Eingabe-Bild kann beispielsweise eine radiologische Aufnahme des Untersuchungsbereichs ohne Kontrastmittel und/oder mit einer geringeren Menge an Kontrastmittel als die Standardmenge des Kontrastmittels umfassen und das synthetische Bild kann eine synthetische radiologische Aufnahme des Untersuchungsbereichs nach der Applikation der Standardmenge an Kontrastmittel sein (wie z.B. beschrieben in WO2019/074938A1 oder WO2022184297A1).

Die "Standardmenge" ist üblicherweise die vom Hersteller und/oder Vertreiber des Kontrastmittels empfohlene und/oder die von einer Zulassungsbehörde zugelassene und/oder die in einem Beipackzettel zum Kontrastmittel aufgeführte Menge. So beträgt die Standardmenge von Primovist® beispielsweise 0,025 mmol Gd-EOB-DTPA Dinatrium / kg Körpergewicht.

Das mindestens eine Eingabe-Bild kann beispielsweise eine oder mehrere MRT-Aufnahmen umfassen, die den Untersuchungsbereich vor und/oder nach Applikation der ersten Menge eines MRT-Kontrastmittels repräsentieren, und das synthetische Bild kann eine synthetische MRT-Aufnahme nach der Applikation der zweiten Menge des MRT-Kontrastmittels sein.

Das mindestens eine Eingabe-Bild kann auch eine CT-Aufnahme vor und/oder nach der Applikation einer ersten Menge eines MRT-Kontrastmittels umfassen, und das synthetische Bild kann eine synthetische CT-Aufnahme nach der Applikation einer zweiten Menge eines MRT-Kontrastmittels sein, wobei die zweite Menge vorzugsweise größer als die erste Menge und vorzugsweise größer als die Standardmenge des MRT-Kontrastmittels für MRT-Untersuchungen ist (wie z.B. beschrieben in WO2023161041A1).

Das mindestens eine Eingabe-Bild kann beispielsweise eine oder mehrere radiologische Aufnahmen des Untersuchungsbereichs in einer ersten Zeitspanne vor/und oder nach der Applikation eines Kontrastmittels umfassen, und das synthetische Bild kann eine synthetische radiologische Aufnahme des Untersuchungsbereichs in einer zweiten Zeitspanne nach der Applikation des Kontrastmittels sein, wobei die zweite Zeitspanne vorzugsweise chronologisch auf die erste Zeitspanne folgt (wie z.B. beschrieben in WO2021052896A1).

Das mindestens eine Eingabe-Bild kann beispielsweise eine oder mehrere radiologische Aufnahmen der Leber oder eines Teils der Leber eines Untersuchungsobjekts umfassen, die die Leber oder den Teil der Leber in der nativen Phase (d.h. ohne Kontrastmittel) und/oder in der arteriellen Phase und/oder in der portalvenösen Phase und/oder in der Übergangsphase nach der Applikation eines hepatobiliären Kontrastmittels repräsentieren, und das synthetische Bild kann eine synthetische radiologische Aufnahme sein, die die Leber oder den Teil der Leber in der hepatobiliären Phase nach der Applikation des hepatobiliären Kontrastmittels repräsentiert. Die eine oder mehreren radiologischen Aufnahmen können eine oder mehrere MRT-Aufnahmen und/oder eine oder mehrere CT-Aufnahmen sein. Das hepatobiliäre Kontrastmittel kann ein hepatobiliäres MRT-Kontrastmittel und/oder hepatobiliäres CT-Kontrastmittel sein.

Die genannten Phasen sind beispielsweise in den folgenden Publikationen näher beschrieben: J. Magn. Reson. Imaging, 2012, 35(3): 492-511, doi:10.1002/jmri.22833; Clujul Medical, 2015, Vol. 88 no. 4: 438-448, DOI: 10.15386/cjmed-414; Journal of Hepatology, 2019, Vol. 71: 534-542, http://dx.doi.org/10.1016/j.jhep.2019.05.005).

Unter einem hepatobiliären Kontrastmittel wird ein Kontrastmittel verstanden, das spezifisch von gesunden Leberzellen, den Hepatozyten, aufgenommen wird. Bespiele für hepatobiliäre Kontrastmittel sind Kontrastmittel auf der Basis der Gadoxetsäure. Sie sind beispielsweise in US 6,039,931A beschrieben. Sie sind kommerziell zum Beispiel unter den Markennamen Primovist® oder Eovist® erhältlich. Ein weiteres Kontrastmittel mit einer geringeren Aufnahme in die Hepatozyten ist Gadobenate Dimeglumine (Multihance®). Weitere hepatobiliäre Kontrastmittel sind u.a. in WO2022/194777 beschrieben.

Das mindestens eine Eingabe-Bild kann beispielsweise eine oder mehrere MRT-Aufnahmen eines Untersuchungsbereichs eines Untersuchungsobjekts umfassen, und das synthetische Bild kann eine synthetische CT-Aufnahme des Untersuchungsbereichs des Untersuchungsobjekts sein.

Das mindestens eine Eingabe-Bild kann beispielsweise eine oder mehrere CT-Aufnahmen eines Untersuchungsbereichs eines Untersuchungsobjekts umfassen, und das synthetische Bild kann eine synthetische MRT-Aufnahme des Untersuchungsbereichs des Untersuchungsobjekts sein.

Das mindestens eine Eingabe-Bild kann beispielsweise eine oder mehrere radiologische Aufnahmen (z.B. MRT- und/oder CT-Aufnahmen) eines Untersuchungsbereichs eines Untersuchungsobjekts umfassen, die unter Anwendung eines ersten Messprotokolls erzeugt worden sind, und das synthetische Bild kann eine synthetische radiologische Aufnahme des Untersuchungsbereich des Untersuchungsobjekts unter Anwendung eines zweiten Messprotokolls sein,

d.h. eine synthetische Aufnahme, die den Untersuchungsbereich des Untersuchungsobjekts so zeigt, wie er aussehen würde, wenn anstelle des ersten Messprotokolls das zweite Messprotokoll angewendet worden wäre.

Das synthetische Bild wird mittels eines trainierten Modells des maschinellen Lernens erzeugt (vorhergesagt).

Ein "Modell des maschinellen Lernens" kann als eine computerimplementierte Datenverarbeitungsarchitektur verstanden werden. Ein solches Modell kann Eingabedaten empfangen und Ausgabedaten auf der Grundlage dieser Eingabedaten und Modellparametern liefern. Ein solches Modell kann durch Training eine Beziehung zwischen den Eingabedaten und den Ausgabedaten erlernen. Beim Training können Modellparameter angepasst werden, um eine gewünschte Ausgabe für eine bestimmte Eingabe zu liefern.

Beim Trainieren eines solchen Modells werden dem Modell Trainingsdaten präsentiert, aus denen es lernen kann. Das trainierte Modell des maschinellen Lernens ist das Ergebnis des Trainingsprozesses. Die Trainingsdaten umfassen neben Eingabedaten die korrekten Ausgabedaten (Zieldaten), die das Modell auf Basis der Eingabedaten erzeugen soll. Beim Trainieren werden Muster erkannt, die die Eingabedaten auf die Zieldaten abbilden.

Im Trainingsprozess werden die Eingabedaten der Trainingsdaten in das Modell eingegeben, und das Modell erzeugt Ausgabedaten. Die Ausgabedaten werden mit den Zieldaten verglichen. Modellparameter können verändert werden, um die Abweichungen zwischen den Ausgabedaten und den Zieldaten auf ein (definiertes) Minimum zu reduzieren.

Die Trainingsdaten umfassen, für jedes Referenzobjekt einer Vielzahl an Referenzobjekten, (i) mindestens ein Eingabe-Referenzbild eines Referenzbereichs des Referenzobjekts in dem ersten Zustand und (ii) ein Ziel-Referenzbild des Referenzbereichs des Referenzobjekts in dem zweiten Zustand als Zieldaten.

Der Begriff "Referenz" wird in dieser Beschreibung verwendet, um die Phase des Trainierens des Modells des maschinellen Lernens von der Phase des Verwendens des trainierten Modells zur Vorhersage (d.h. zum Erzeugen synthetischer Bilder) zu unterscheiden. Der Begriff "Referenz" hat ansonsten keinerlei einschränkende Bedeutung. Aussagen, die in dieser Beschreibung zu dem mindestens einen Eingabe-Bild getroffen werden, treffen in analoger Weise auf jedes Eingabe-Referenzbild zu; Aussagen, die in dieser Beschreibung zu dem Untersuchungsobjekt getroffen werden, treffen in analoger Weise auf jedes Referenzobjekt zu; Aussagen, die in dieser Beschreibung zu dem Untersuchungsbereich getroffen werden, treffen in analoger Weise auf den Referenzbereich zu.

Jedes Referenzobjekt ist, wie das Untersuchungsobjekt, üblicherweise ein Lebewesen, vorzugsweise ein Säugetier, ganz besonders bevorzugt ein Mensch. Der "Referenzbereich" ist ein Teil des Referenzobjekts. Der Referenzbereich entspricht üblicherweise (aber nicht notwendigerweise) dem Untersuchungsbereich des Untersuchungsobjekts. Mit anderen Worten: für den Fall, dass der Untersuchungsbereich ein Organ oder ein Teil eines Organs (z.B. die Leber oder ein Teil der Leber) des Untersuchungsobjekts ist, ist der Referenzbereich eines jeden Referenzobjekts vorzugsweise das entsprechende Organ oder der entsprechende Teil des Organs des jeweiligen Referenzobjekts. Das mindestens eine Eingabe-Referenzbild des Referenzbereichs des Referenzobjekts in dem ersten Zustand entspricht also üblicherweise dem mindestens einen Eingabe-Bild des Untersuchungsbereichs des Untersuchungsobjekts in dem ersten Zustand - mit dem Unterschied, dass das mindestens eine Eingabe-Referenzbild vom Referenzobjekt stammt und das mindestens eine Eingabe-Bild vom Untersuchungsobjekt stammt.

Der Zusatz "Eingabe-" in dem Begriff "Eingabe-Referenzbild" weist darauf hin, dass das Eingabe-Referenzbild bestimmt ist, im Rahmen des Trainings eines Modells des maschinellen Lernens dem Modell des maschinellen Lernens als Eingabedaten oder zumindest als ein Teil der Eingabedaten zugeführt zu werden.

Der Zusatz "Ziel-" in dem Begriff "Ziel-Referenzbild" weist darauf hin, dass das Ziel-Referenzbild im Rahmen des Trainings des Modells des maschinellen Lernens als Zieldaten (*ground truth*) oder zumindest als ein Teil der Zieldaten verwendet wird.

Das Ziel-Referenzbild ist üblicherweise kein synthetisches Bild, sondern das Ergebnis einer Messung, beispielsweise einer radiologischen Untersuchung.

Das mindestens eine Eingabe-Referenzbild umfasst eine Vielzahl an Bildelementen; jedes Bildelement repräsentiert einen Teilbereich des Referenzbereichs; jedes Bildelement ist durch einen Farbwert gekennzeichnet. Ferner umfasst auch das Ziel-Referenzbild eine Vielzahl an Bildelementen; jedes Bildelement repräsentiert einen Teilbereich des Referenzbereichs; jedes Bildelement ist durch einen Farbwert gekennzeichnet.

Für jedes Referenzobjekt wird das mindestens eine Eingabe-Referenzbild dem Modell des maschinellen Lernens zugeführt. Das Modell des maschinellen Lernens ist konfiguriert, auf Basis des mindestens einen Eingabe-Referenzbildes (und ggf. weiteren Eingabedaten) ein synthetisches Referenzbild zu erzeugen. Das synthetische Referenzbild soll dem (üblicherweise gemessenen) Ziel-Referenzbild möglichst nahekommen.

Das synthetische Referenzbild umfasst eine Vielzahl an Bildelementen; jedes Bildelement repräsentiert einen Teilbereich des Referenzbereichs; jedes Bildelement ist durch einen Farbwert gekennzeichnet.

Jedes Bildelement des synthetischen Referenzbildes korrespondiert mit jeweils einem Bildelement des Ziel-Referenzbildes. Korrespondierende Bildelemente sind diejenigen Bildelemente, die denselben Referenzbereich (bzw. Untersuchungsbereich) repräsentieren; korrespondierende Bildelemente haben üblicherweise dieselben Koordinaten.

Das Modell des maschinellen Lernens ist konfiguriert und wird trainiert, für jedes Bildelement des synthetischen

Referenzbildes einen Farbwert vorherzusagen. Der vorhergesagte Farbwert eines jeden Bildelements soll dem Farbwert des korrespondierenden Bildelements des (üblicherweise gemessenen) Ziel-Referenzbild möglichst nahekommen.

Das Modell des maschinellen Lernens ist ferner konfiguriert und wird trainiert, für jeden vorhergesagten Farbwert einen Unsicherheitswert vorherzusagen. Der Unsicherheitswert spiegelt die Unsicherheit des vorhergesagten Farbwerts wider.

Das Trainieren des Modells des maschinellen Lernens umfasst das Minimieren einer Fehlerfunktion.

Die Fehlerfunktion umfasst den von dem Modell des maschinellen Lernens vorhergesagten Farbwert und/oder eine Abweichung des vorhergesagten Farbwerts von dem Farbwert des korrespondierenden Bildelements des Ziel-Referenzbildes als Parameter. Die Fehlerfunktion umfasst ferner den von dem Modell des maschinellen Lernens vorhergesagten Unsicherheitswert als weiteren Parameter.

Die Fehlerfunktion L kann beispielsweise die folgende Gleichung (1) aufweisen:

$$\mathcal{L} = \frac{1}{N}\sum_{i=1}^{N}\frac{1}{2\hat{\sigma}(x_i)^2}\|y_i - \hat{y}_i\|^2 + \frac{1}{2}\log\hat{\sigma}(x_i)^2 \qquad (1)$$

Dabei ist $\hat{y}_i$ der vorhergesagte Farbwert des Bildelements i des synthetischen Referenzbildes und $y_i$ der Farbwert des korrespondierenden Bildelements i des (üblicherweise gemessenen) Ziel-Referenzbildes. *N* gibt die Zahl der Bildelemente des synthetischen Referenzbildes an. $\hat{\sigma}(x_i)$ ist der vorhergesagte Unsicherheitswert; er ist abhängig von den Farbwerten $x_i$ der korrespondierenden Bildelemente des mindestens einen Eingabe-Referenzbildes.

Die hier beispielhaft aufgeführte Fehlerfunktion L basiert auf der von A. Kendall und Y. Gal vorgeschlagenen Fehlerfunktion (siehe: A. Kendall, Y. Gal: *What Uncertainties Do We Need in Bayesian Deep Learning for Computer Vision?,* Computer Vision and Pattern Recognition, 2017, https://arxiv.org/abs/1703.04977).

Die Fehlerfunktion $\mathcal{L}$ umfasst einen Term, der der L2-Fehlerfunktion (Euklidischer Abstand) entspricht:

$$\sum_{i=1}^{N}\|y_i - \hat{y}_i\|^2$$

Werden von dem Modell des maschinellen Lernens während das Trainierens Farbwerte vorhergesagt, die den Farbwerten der Zieldaten nahekommen (im Idealfall mit diesen übereinstimmen), dann ist der Euklidische Abstand gering (bei Übereinstimmung der Farbwerte ist er gleich Null) und der mit der Fehlerfunktion L berechnete Fehler ist ebenfalls gering.

Weichen die vorhergesagten Farbwerte von den Zieldaten ab, dann ist der Euklidische Abstand groß.

Der Euklidische Abstand wird in der Fehlerfunktion $\mathcal{L}$ durch das Quadrat des Unsicherheitswerts $\hat{\sigma}(x_i)$ geteilt. Dies führt dazu, dass der erste Term der Fehlerfunktion $\mathcal{L}$ auch bei einem großen Euklidischen Abstand der vorhergesagten Farbwerte von den Farbwerten der Zieldaten klein bleibt. Mit anderen Worten: weichen die vorhergesagten Farbwerte von den Farbwerten der Zieldaten ab, kann die Fehlerfunktion $\mathcal{L}$ dennoch minimiert werden, wenn die Unsicherheitswerte groß sind. Mit anderen Worten: eine hohe Abweichung bei der Vorhersage der Farbwerte führt zu einem hohen Unsicherheitswert.

Damit während des Trainierens des Modells des maschinellen Lernens die Modellparameter nicht so verändert werden, dass die Unsicherheitswerte immer größer werden (um die Fehlerfunktion zu minimieren), gibt es einen zweiten Term zur Regularisierung:

$$\frac{1}{2}\log\hat{\sigma}(x_i)^2$$

Das Modell des maschinellen Lernens lernt die Unsicherheitswerte also implizit aus der Fehlerfunktion; die Unsicherheitswerte sind nicht Bestandteil der Zieldaten; die Unsicherheitswerte spiegeln die Unsicherheit der Eingabedaten wider, auch als heteroskedastische aleatorische Ungewissheit bezeichnet.

Die oben angegebene Gleichung (1) ist nur ein Beispiel für eine Fehlerfunktion; andere Gleichungen sind denkbar.

In einer Gleichung für eine geeignete Fehlerfunktion sind neben den vorhergesagten Farbwerten bzw. Abweichungen der vorhergesagten Farbwerte von den Farbwerten der Zieldaten ein oder mehrere Unsicherheitswerte als Parameter enthalten. Ein solcher Unsicherheitswert dient als Komplementär zu den Abweichungen der vorhergesagten

Farbwerte von den Farbwerten der Zieldaten. Er sorgt dafür, dass einer Zunahme der Abweichungen der vorhergesagten Farbwerte von den Farbwerten der Zieldaten etwas entgegengesetzt wird, um die Fehlerfunktion auch bei Abweichungen, die von Null verschieden sind, minimieren zu können.

Das Trainieren des Modells des maschinellen Lernens ist beispielhaft und schematisch in Fig. 1 dargestellt.

Fig. 1 zeigt beispielhaft und schematisch ein Verfahren zum Trainieren des Modells des maschinellen Lernens.

Das Trainieren des Modells MLM des maschinellen Lernens erfolgt mit Trainingsdaten. Die Trainingsdaten umfassen, für jedes Referenzobjekt einer Vielzahl von Referenzobjekten, (i) mindestens ein Eingabe-Referenzbild $RI_1(x_i)$ eines Referenzbereichs des Referenzobjekts in einem ersten Zustand als Eingabedaten und (ii) ein Ziel-Referenzbild $RI_2(y_i)$ des Referenzbereichs des Referenzobjekts in einem zweiten Zustand als Zieldaten.

In dem in Fig. 1 dargestellten Beispiel ist nur ein Satz an Trainingsdaten TD eines Referenzobjekts gezeigt; üblicherweise umfassen die Trainingsdaten eine Vielzahl dieser Datensätze für eine Vielzahl an Referenzobjekten.

Das mindestens eine Eingabe-Referenzbild $RI_1(x_i)$ repräsentiert den Referenzbereich des Referenzobjekts in einem ersten Zustand; das Ziel-Referenzbild $RI_2(y_i)$ repräsentiert den Referenzbereich des Referenzobjekts in einem zweiten Zustand. Der erste Zustand und der zweite Zustand sind verschiedene Zustände. Zum Beispiel kann der Zustand eine Menge an Kontrastmittel repräsentieren, die in den Referenzbereich eingebracht wird oder worden ist. Zum Beispiel kann der Zustand ein spezifisches Kontrastmittel repräsentieren. Zum Beispiel kann der Zustand einen Zeitpunkt vor und/oder nach einer Applikation eines Kontrastmittels repräsentieren. Zum Beispiel kann der Zustand eine Modalität und/oder ein Messprotokoll repräsentieren. Weitere Beispiele sind in dieser Offenbarung genannt.

Das mindestens eine Eingabe-Referenzbild $RI_1(x_i)$ dient in dem in Fig. 1 gezeigten Beispiel als Eingabedaten; es wird dem Modell MLM des maschinellen Lernens zugeführt. Das Modell MLM des maschinellen Lernens ist konfiguriert, auf Basis des mindestens einen Eingabe-Referenzbildes $RI_1(x_i)$ und auf Basis von Modellparametern MP ein synthetisches Referenzbild $RI_2^*(\hat{y}_i)$ zu erzeugen. Das synthetische Referenzbild $RI_2^*(\hat{y}_i)$ ist ein synthetisches (vorhergesagtes) Bild. Das synthetische Referenzbild $RI_2^*(\hat{y}_i)$ soll dem Ziel-Referenzbild $RI_2(y_i)$ möglichst nahekommen. Das heißt, das Ziel-Referenzbild $RI_2(y_i)$ fungiert in dem in Fig. 1 gezeigten Beispiel als Zieldaten (*ground truth*). Das synthetische Referenzbild $RI_2^*(\hat{y}_i)$ soll den Referenzbereich des Referenzobjekts in dem zweiten Zustand repräsentieren.

Das mindestens eine Eingabe-Referenzbild $RI_1(x_i)$ umfasst eine Vielzahl an Bildelementen (in Fig. 1 nicht explizit gezeigt). Jedes Bildelement *i* des mindestens einen Eingabe-Referenzbildes $RI_1(x_i)$ ist durch einen Farbwert $x_i$ gekennzeichnet. Das Ziel-Referenzbild $RI_2(y_i)$ umfasst ebenfalls eine Vielzahl an Bildelementen (in Fig. 1 nicht explizit gezeigt). Jedes Bildelement *i* des Ziel-Referenzbildes $RI_2(y_i)$ ist durch einen Farbwert $y_i$ gekennzeichnet.

Das Modell MLM des maschinellen Lernens wird trainiert, für jedes Bildelement *i* des synthetischen Referenzbildes $RI_2^*(\hat{y}_i)$ einen Farbwert $\hat{y}_i$ vorherzusagen. Die Vorhersage erfolgt auf Basis der Farbwerte des mindestens einen Eingabe-Referenzbild $RI_1(x_i)$.

Jeder vorhergesagte Farbwert $\hat{y}_i$ eines jeden Bildelements des synthetischen Referenzbildes $RI_2^*(\hat{y}_i)$ soll dem jeweiligen Farbwert $y_i$ des korrespondierenden Bildelements des Ziel-Referenzbildes $RI_2(y_i)$ möglichst nahekommen (im Idealfall entsprechen). Mit anderen Worten: Abweichungen zwischen dem vorhergesagten Farbwert $\hat{y}_i$ eines Bildelements *i* des synthetischen Referenzbildes $RI_2^*(\hat{y}_i)$ und dem jeweiligen Farbwert $y_i$ des korrespondierenden Bildelements *i* des Ziel-Referenzbildes $RI_2(y_i)$ sollen möglichst klein sein (im Idealfall Null sein).

Das Modell MLM des maschinellen Lernens wird ferner trainiert, für jeden vorhergesagten Farbwert $\hat{y}_i$ einen Unsicherheitswert $\hat{\sigma}(x_i)$ vorherzusagen. Der Unsicherheitswert $\hat{\sigma}(x_i)$ ist abhängig von den Farbwerten $x_i$ der Bildelemente *i* des mindestens einen Eingabe-Referenzbildes $RI_1(x_i)$.

Das Trainieren des Modells MLM des maschinellen Lernens umfasst ein Minimieren einer Fehlerfunktion $\mathcal{L}$. Mit anderen Worten: für jedes Tripel aus einem vorhergesagten Farbwert $\hat{y}_i$, dem Farbwert $y_i$ und dem vorhergesagten Unsicherheitswert $\hat{\sigma}(x_i)$ kann mittels der Fehlerfunktion $\mathcal{L}$ ein Fehler berechnet werden; durch Modifizieren von Modellparametern MP kann der ermittelte Fehler reduziert werden; Ziel kann es sein, die Modellparameter so zu modifizieren, dass der mittels der Fehlerfunktion $\mathcal{L}$ ermittelte Fehler für alle Tripel einen minimalen Wert annimmt, und/oder sich der ermittelte Fehler durch Modifizieren der Modellparameter nicht mehr reduzieren lässt. Das Modifizieren der Modellparameter MP und das Minimieren der Fehlerfunktion $\mathcal{L}$ kann in einem Optimierungsverfahren erfolgen. Die Fehlerfunktion $\mathcal{L}$ kann die oben genannte Gleichung (1) oder eine andere Gleichung haben.

Das trainierte Modell des maschinellen Lernens kann nach dem Trainieren in einem Datenspeicher gespeichert und/oder ausgegeben und/oder an ein anderes Computersystem übermittelt werden. Das trainierte Modell des maschinellen Lernens kann nach dem Trainieren zum Erzeugen synthetischer Bilder genutzt werden.

Fig. 2 zeigt beispielhaft und schematisch ein computer-implementiertes Verfahren zum Erzeugen eines synthetischen Bildes eines Untersuchungsbereichs eines Untersuchungsobjekts.

Das Erzeugen des synthetischen Bildes erfolgt mit einem trainierten Modell $MLM^t$ des maschinellen Lernens. Das trainierte Modell $MLM^t$ des maschinellen Lernens kann wie in Bezug zu Fig. 1 beschrieben trainiert worden sein.

Dem trainierten Modell $MLM^t$ des maschinellen Lernens wird mindestens ein Eingabe-Bild $I_1(x_i)$ eines Untersuchungsbereichs eines Untersuchungsobjekts zugeführt. Das mindestens eine Eingabe-Bild $I_1(x_i)$ repräsentiert den

Untersuchungsbereich des Untersuchungsobjekts in einem ersten Zustand. Auf Basis des mindestens einen Eingabe-Bildes $I_1(x_i)$ erzeugt das trainierte Modell $MLM^t$ des maschinellen Lernens ein synthetisches Bild $I_2^*(\hat{y}_i)$. Das synthetische Bild $I_2^*(\hat{y}_i)$ repräsentiert den Untersuchungsbereich des Untersuchungsobjekts in einem zweiten Zustand. Der zweite Zustand ist verschieden von dem ersten Zustand.

Zum Beispiel kann der Zustand eine Menge an Kontrastmittel repräsentieren, die in den Untersuchungsbereich eingebracht wird oder worden ist. Zum Beispiel kann der Zustand ein spezifisches Kontrastmittel repräsentieren. Zum Beispiel kann der Zustand einen Zeitpunkt vor und/oder nach einer Applikation eines Kontrastmittels repräsentieren. Zum Beispiel kann der Zustand eine Modalität und/oder ein Messprotokoll repräsentieren. Weitere Beispiele sind in dieser Offenbarung genannt. Die Zustände bei der Vorhersage entsprechen üblicherweise den Zuständen beim Trainieren des Modells des maschinellen Lernens.

Das mindestens eine Eingabe-Bild $I_1(x_i)$ umfasst eine Vielzahl an Bildelementen (in Fig. 2 nicht explizit dargestellt); jedes Bildelement *i* des mindestens einen Eingabe-Bildes $I_1(x_i)$ repräsentiert einen Teilbereich des Untersuchungsbereichs; jedem Bildelement i des mindestens einen Eingabe-Bildes $I_1(x_i)$ ist ein Farbwert x, zugeordnet.

Das synthetische Bild $I_2^*(\hat{y}_i)$ umfasst eine Vielzahl an Bildelementen (in Fig. 2 nicht explizit dargestellt); jedes Bildelement *i* des syntehtsichen Bildes $I_2^*(\hat{y}_i)$ repräsentiert einen Teilbereich des Untersuchungsbereichs

Das trainierte Modell $MLM^t$ des maschinellen Lernens ist konfiguriert und wurde trainiert, auf Basis der mindestens einen Eingabe-Bildes $I_1(x_i)$ für jedes Bildelement *i* des synthetischen Bildes $I_2^*(\hat{y}_i)$ einen Farbwert $\hat{y}_i$ vorauszusagen. Mit anderen Worten: jedem Bildelement *i* des synthetischen Bildes $I_2^*(\hat{y}_i)$ ist ein vorhergesagter Farbwert $\hat{y}_i$ zugeordnet.

Das synthetische Bild $I_2^*(\hat{y}_i)$ kann ausgegeben (d.h. zum Beispiel auf einem Monitor angezeigt oder mit einem Drucker ausgedruckt) und/oder in einem Datenspeicher gespeichert und/oder an ein separates Computersystem übermittelt werden.

Das trainierte Modell $MLM^t$ des maschinellen Lernens ist ferner konfiguriert und wurde ferner trainiert, für jeden vorhergesagten Farbwert $\hat{y}_i$ einen Unsicherheitswert $\hat{\sigma}(x_i)$ vorherzusagen.

Auf Basis der Unsicherheitswerte, die für die vorhergesagten Farbwerte der Bildelemente eines synthetischen) Bildes vorhergesagte wurden, lässt sich mindestens ein Vertrauenswert ermitteln.

Der mindestens eine Vertrauenswert kann ein Wert sein, der angibt, inwieweit man einem synthetischen Bild (z.B. synthetischen Bild $I_2^*(\hat{y}_i)$ in Fig. 2 (oder dem synthetischen Bild $I_3^*(\hat{y}_i)$ in Fig. 6) vertrauen kann. Der Vertrauenswert kann positiv mit der Vertrauenswürdigkeit des synthetischen Bildes korrelieren, d.h., wenn der Vertrauenswert gering ist, ist auch die Vertrauenswürdigkeit gering und wenn der Vertrauenswert hoch ist, ist auch die Vertrauenswürdigkeit hoch. Es ist aber auch möglich, dass der Vertrauenswert negativ mit der Vertrauenswürdigkeit korreliert; d.h., wenn der Vertrauenswert gering ist, ist die Vertrauenswürdigkeit hoch und wenn der Vertrauenswert hoch ist, ist die Vertrauenswürdigkeit gering.

Ist die Vertrauenswürdigkeit gering, dann geht von dem synthetischen Bild eine hohe Unsicherheit aus; es ist möglich, dass das synthetische Bild ein oder mehrere Artefakte aufweist; es ist möglich, dass für Strukturen und/oder Morphologien und/oder Texturen in dem synthetischen Bild keine Entsprechung in der Realität existiert, d.h., dass Strukturen und/oder Morphologien und/oder Texturen in dem synthetischen Bild nicht auf reale Strukturen und/oder reale Morphologien und/oder reale Texturen im Untersuchungsbereich zurückgeführt werden können.

Ein hoher Vertrauenswert hingegen zeigt an, dass das synthetische Bild eine geringe Unsicherheit aufweist; Merkmale in dem synthetischen Bild haben eine Entsprechung in der Realität; dem synthetischen Bild kann vertraut werden; eine medizinische Diagnose kann auf Basis des synthetischen Bildes gestellt und/oder eine medizinische Therapie kann auf Basis des synthetischen Bildes initiiert werden.

Ein Vertrauenswert, der positiv mit der Vertrauenswürdigkeit korreliert, kann prinzipiell in einen Vertrauenswert, der negativ mit der Vertrauenswürdigkeit korreliert, umgewandelt werden, beispielsweise, indem man das Reziproke (den Kehrwert) bildet. Umgekehrt kann entsprechend auch ein Vertrauenswert, der negativ mit der Vertrauenswürdigkeit korreliert, in einen Vertrauenswert, der positiv mit der Vertrauenswürdigkeit korreliert, umgewandelt werden.

Die von dem trainierten Modell des maschinellen Lernens vorhergesagten Unsicherheitswerte geben für jedes einzelne Bildelement an, wie unsicher der jeweilige vorhergesagte Farbwert ist. Dementsprechend können diese Unsicherheitswerte direkt als Vertrauenswerte verwendet werden. Sie korrelieren negativ mit der Vertrauenswürdigkeit; sie korrelieren positiv mit der Unsicherheit.

Der mindestens eine Vertrauenswert kann auch ein Wert sein, der von den Unsicherheitswerten abgeleitet ist.

Unter der Annahme, dass die vorhergesagten Farbwerte einer Gaußschen Wahrscheinlichkeitsverteilung gehorchen, entspricht der vorhergesagte Farbwert $\hat{y}_i$ in Gleichung (1) dem Mittelwert, der Unsicherheitswert $\hat{\sigma}(x_i)$ der Standardabweichung und das Quadrat $\hat{\sigma}(x_i)^2$ des Unsicherheitswerts der Varianz der Gaußschen Verteilungsfunktion. Anstelle des vorhergesagten Unsicherheitswerts $\hat{\sigma}(x_i)$ kann also auch das Quadrat $\hat{\sigma}(x_i)^2$ oder eine andere abgeleitete Größe als der mindestens eine Vertrauenswert verwendet werden.

Es ist auch denkbar, dass das Modell des maschinellen Lernens konfiguriert ist und trainiert wird, einen Vertrauenswert (anstelle des Unsicherheitswerts oder in Ergänzung zum Unsicherheitswert) vorherzusagen. In einem

solchen Fall ist der Vertrauenswert (oder eine davon abgeleitete Größe) ein (weiterer) Parameter der Fehlerfunktion.

Es lässt sich also für jedes einzelne Bildelement des synthetischen Bildes ein Vertrauenswert ermitteln, der angibt, wie sehr man dem Farbwert des Bildelements vertrauen kann.

Gibt es mehr als einen Farbwert (zum Beispiel drei Farbwerte, wie im Fall von Bildern, deren Farbwerte gemäß dem RGB-Farbmodell spezifiziert sind), kann für jeden Farbkanal ein Unsicherheitswert vorhergesagt werden und es kann für jeden Farbkanal ein Vertrauenswert ermittelt werden.

Es ist aber auch möglich, die Unsicherheitswerte der Farbkanäle zu einem einzigen Wert zu kombinieren und einen Vertrauenswert auf Basis des kombinierten Werts zu ermitteln; es ist möglich, auf Basis des maximalen Unsicherheitswert der Farbkanäle einen Vertrauenswert zu ermitteln; es ist möglich, auf Basis eines Mittelwerts (z.B. des arithmetischen Mittel, des geometrischen Mitteln, des quadratischen Mittels oder eines anderen Mittelwerts) der Unsicherheitswerte der Farbkanäle einen Vertrauenswert zu ermitteln; es ist möglich, auf Basis der Länge desjenigen Vektors, den die Unsicherheitswerte der Farbkanäle in einem dreidimensionalen Raum (oder einem höherdimensionalen Raum bei Verwendung von mehr als drei Farbkanälen) spezifizieren, einen Vertrauenswerts zu ermitteln; weitere Möglichkeiten sind denkbar.

Es ist möglich, dass für verschiedene Teilbereiche des Untersuchungsobjekts verschiedene Methoden zur Berechnung eines Vertrauenswertes verwendet werden. Es ist zum Beispiel möglich, dass für Bildelemente, die ein spezifisches Gewebe und/oder ein Organ und/oder eine Läsion repräsentieren, eine andere Methode zur Berechnung eines Vertrauenswerts verwendet wird als für Bildelemente, die ein anderes Gewebe und/oder ein anderes Organ und/oder einen anderen Teilbereich repräsentieren. Teilbereiche, für die verschiedene Rechenvorschriften für die Vertrauenswerte gelten, können beispielsweise mit Hilfe einer Segmentierung identifiziert werden.

Der Begriff "Segmentierung" bezieht sich auf den Prozess der Aufteilung eines Bildes in mehrere Segmente, die auch als Bildsegmente, Bildregionen oder Bildobjekte bezeichnet werden. Die Segmentierung wird in der Regel verwendet, um Objekte und Grenzen (Linien, Kurven usw.) in Bildern zu lokalisieren. In einem segmentierten Bild können die lokalisierten Objekte vom Hintergrund getrennt, visuell hervorgehoben (z.B. farbig), gemessen, gezählt und/oder anderweitig quantifiziert werden. Bei der Segmentierung wird jedem Bildelement eines Bildes ein Kennzeichen (z.B. eine Zahl) zugewiesen, so dass Bildelemente mit demselben Kennzeichen bestimmte gemeinsame Merkmale aufweisen, z.B. das gleiche Gewebe (z.B. Knochengewebe oder Fettgewebe oder gesundes Gewebe oder erkranktes Gewebe (z.B. Tumorgewebe) oder Muskelgewebe und/oder dergleichen) und/oder dasselbe Organ repräsentieren. Für Bildelemente mit einem spezifischen Kennzeichen kann dann eine spezifische Rechenvorschrift zur Berechnung eines Vertrauenswerts verwendet werden; für Bildelemente mit einem anderen (spezifischen) Kennzeichen kann eine andere (spezifische) Rechenvorschrift zur Berechnung eines Vertrauenswerts verwendet werden.

Die für Bildelemente ermittelten Vertrauenswerte können ausgegeben (z.B. auf einem Monitor angezeigt oder auf einem Drucker ausgedruckt), in einem Datenspeicher gespeichert und/oder an ein separates Computersystem z.B. über ein Netzwerk übermittelt werden.

Die für Bildelemente ermittelten Vertrauenswerte können auch bildhaft dargestellt werden.

Neben dem synthetischen Bild, das auf Basis des mindestens einen Eingabe-Bildes erzeugt wurde, kann also eine weitere Repräsentation des Untersuchungsbereichs ausgegeben (z.B. auf einem Monitor angezeigt) werden, in der für jedes Bildelement angezeigt wird, wie vertrauenswürdig es ist. Eine solche Repräsentation wird in dieser Beschreibung auch als Vertrauensrepräsentation bezeichnet. Die Vertrauensrepräsentation hat vorzugsweise die gleiche Dimension und Größe wie das synthetische Bild; jedem Bildelement des synthetischen Bildes ist vorzugsweise ein Bildelement in der Vertrauensrepräsentation zugeordnet.

Anhand einer solchen Vertrauensrepräsentation kann ein Nutzer (z.B. ein Arzt) für jedes einzelne Bildelement erkennen, wie weit er dem vorhergesagten Farbwert des Bildelements vertrauen kann. Es ist möglich, die Vertrauensrepräsentation mit dem synthetischen Bild ganz oder teilweise überlagert darzustellen. Es ist möglich, die überlagerte Darstellung so zu gestalten, dass der Nutzer sie ein- und ausblenden kann. Der Nutzer kann sich das synthetische Bild beispielsweise Schicht für Schicht anzeigen lassen, so wie es für computertomografische, magnetresonanztomografische und andere drei- oder höherdimensionale Repräsentationen üblich ist. Bei jeder Schicht kann er die entsprechende Schicht der Vertrauensrepräsentation einblenden lassen, um zu prüfen, ob die vorhergesagten Farbwerte der Bildelemente in der Schicht, die Strukturen, Morphologien und/oder Texturen zeigen, vertrauenswürdig oder unsicher sind. Auf diese Weise kann der Nutzer erkennen, wie hoch das Risiko ist, dass es sich bei den Strukturen, Morphologien und/oder Texturen um reale Eigenschaften des Untersuchungsbereichs oder um Artefakte handelt.

Bildelemente mit einer geringen Vertrauenswürdigkeit (mit einer hohen Unsicherheit) können beispielsweise hell und/oder mit einer Signalfarbe (z.B. rot oder orange oder gelb) dargestellt werden, während Bildelemente mit einer hohen Vertrauenswürdigkeit (mit einer geringen Unsicherheit) dunkel oder mit einer unauffälligeren oder beruhigenden Farbe (z.B. grün oder blau) dargestellt werden können. Es ist auch möglich, dass nur diejenigen Bildelemente bei einer Überlagerung dargestellt werden, bei denen der Vertrauenswert einen vordefinierten Grenzwert über- oder unterschreitet. Korreliert der Vertrauenswert positiv mit der Vertrauenswürdigkeit können beispielsweise nur diejenigen Bildelemente der Vertrauensrepräsentation dargestellt werden, deren Vertrauenswert unterhalb eines vordefinierten Grenzwerts liegt;

in einem solchen Fall werden einem Nutzer (z.B. einem Arzt) explizit diejenigen Bildelemente zur Kenntnis gegeben, denen er eher nicht vertrauen sollte.

Es ist auch möglich, dass Vertrauenswerte für Teilbereiche des synthetischen Bildes (z.B. für Schichten innerhalb eines drei- oder höherdimensionalen synthetischen Bildes) und/oder für das gesamte synthetische Bild ermittelt werden. Eine Ermittlung solcher Vertrauenswerte für Teilbereiche oder gesamte Bilder kann auf Basis der Vertrauenswerte derjenigen Bildelemente erfolgen, aus denen sie zusammengesetzt sind. Zur Ermittlung eines Vertrauenswerts einer Schicht können beispielsweise alle Vertrauenswerte derjenigen Bildelemente berücksichtigt werden, die in dieser Schicht liegen. Es ist aber auch möglich, auch benachbarte Bildelemente (zum Beispiel Bildelemente der Schicht über und/oder unter der betrachteten Schicht) zu berücksichtigen. Ein Vertrauenswert für einen Teilbereich oder den gesamten Bereich kann beispielsweise durch Mittelwertbildung (z.B. arithmetischer Mittelwert, geometrisches Mittel, quadratische Mittel oder einen anderen Mittelwert) ermittelt werden. Es ist auch möglich, den Maximalwert (z.B. bei einem Vertrauenswert, der negativ mit der Vertrauenswürdigkeit korreliert) oder den Minimalwert (z.B. bei einem Vertrauenswert, der negativ mit der Vertrauenswürdigkeit korreliert) der Vertrauenswerte der Bildelemente eines Teilbereichs oder des Gesamtbereichs zu ermitteln und diesen als Vertrauenswert des Teilbereichs oder des Gesamtbereichs zu verwenden. Weitere Möglichkeiten, einen Vertrauenswert für einen Teilbereich oder den Gesamtbereich auf Basis der Vertrauenswerte einzelner Bildelemente zu ermitteln, sind denkbar.

Eine solcher Vertrauenswert für einen Teilbereich oder den Gesamtbereich kann ebenfalls ausgegeben (z.B. auf einem Monitor angezeigt oder auf einem Drucker ausgegeben), in einem Datenspeicher gespeichert und/oder an ein separates Computersystem übermittelt werden. Er kann auch, wie für die einzelnen Vertrauenswerte beschrieben, bildhaft (z.B. farblich) dargestellt werden.

Ist ein positiv mit der Vertrauenswürdigkeit korrelierender Vertrauenswert für einen Teilbereich oder den Gesamtbereich geringer als ein vordefinierter Grenzwert, dann ist es möglich, dass dem entsprechenden Teilbereich oder Gesamtbereich kein Vertrauen geschenkt werden sollte. Es ist möglich, dass ein solcher Teilbereich oder der entsprechende Gesamtbereich gar nicht ausgegeben (z.B. gar nicht angezeigt) wird, oder dass er mit einem Warnhinweis angezeigt wird, dass ein Nutzer bei der Interpretation der angezeigten Daten vorsichtig sein sollte, da die angezeigten Daten unsicher sind.

Es ist auch möglich, dass dem Nutzer des Computersystems/Computerprogramms der vorliegenden Offenbarung über eine Nutzerschnittstelle die Möglichkeit gegeben wird, in dem synthetischen Bild zu Teilbereichen zu navigieren, die eine niedrige Vertrauenswürdigkeit aufweisen. Dem Nutzer können beispielsweise die Teilbereiche mit der niedrigsten Vertrauenswürdigkeit in einer Liste angezeigt werden (z.B. in Form einer Liste mit einer Zahl $q$ an Teilbereichen, die den niedrigsten positiv mit der Vertrauenswürdigkeit korrelierenden Vertrauenswert aufweisen, wobei q eine positive ganze Zahl ist). Durch ein Anklicken eines Listeneintrags kann dem Nutzer der entsprechende Teilbereich in Form eines synthetischen Bildes, einer Vertrauensrepräsentation und/oder einem Eingabe-Bild und/oder einem Ausschnitt davon angezeigt werden.

Das Modell des maschinellen Lernens kann beispielsweise ein künstliches neuronales Netz sein oder ein solches umfassen.

Ein künstliches neuronales Netzwerk umfasst mindestens drei Schichten von Verarbeitungselementen: eine erste Schicht mit Eingangsneuronen (Knoten), eine $N$-te Schicht mit mindestens einem Ausgangsneuron (Knoten) und $N$-2 innere Schichten, wobei $N$ eine natürliche Zahl und größer als 2 ist.

Die Eingangsneuronen dienen zum Empfangen der Eingabedaten, insbesondere zum Empfangen des mindestens einen Eingabe-Bildes. Beispielsweise kann es mindestens ein Eingangsneuron für jedes Bildelement des mindestens einen Eingabe-Bildes geben. Es können zusätzliche Eingangsneuronen für zusätzliche Eingabedaten (z.B. Informationen zum Untersuchungsbereich, zum Untersuchungsobjekt, zu Bedingungen, die bei der Erzeugung des mindestens einen Eingabe-Bildes herrschten, Informationen zu dem ersten und/oder zweiten Zustand, und/oder Informationen zu dem Zeitpunkt oder der Zeitspanne zu/in der das mindestens eine Eingabe-Bild erzeugt worden ist) vorhanden sein.

Die Ausgangsneuronen können dazu dienen, ein synthetisches Bild auszugeben.

Die Verarbeitungselemente der Schichten zwischen den Eingangsneuronen und den Ausgangsneuronen sind in einem vorbestimmten Muster mit vorbestimmten Verbindungsgewichten miteinander verbunden.

Vorzugsweise handelt es sich bei dem künstlichen neuronalen Netz um ein so genanntes Convolutional Neural Network (kurz: CNN) oder es umfasst ein solches.

Ein CNN besteht üblicherweise im Wesentlichen aus Filtern (Convolutional Layer) und Aggregations-Schichten (Pooling Layer), die sich abwechselnd wiederholen, und am Ende aus einer Schicht oder mehreren Schichten von vollständig verbundenen Neuronen (Dense / Fully Connected Layer).

Das Modell des maschinellen Lernens kann beispielsweise eine Architektur wie das U-Net aufweisen (siehe z.B. O. Ronneberger et al.: U-Net: Convolutional Networks for Biomedical Image Segmentation, arXiv:1505.04597v1). Das Modell des maschinellen Lernens kann eine Architektur aufweisen wie beschrieben in: V. P. Sudarshan et al.: Towards lower-dose PET usingphysics-based uncertainty-aware multimodal learning with robustness to out-of-distribution data, Medical Image Analysis 73 (2021) 102187.

Das Trainieren des neuronalen Netzes kann beispielsweise mittels eines Backpropagation-Verfahrens durchgeführt werden. Dabei wird für das Netz eine möglichst zuverlässige Abbildung des mindestens einen Eingabe-Referenzbildes auf das Ziel-Referenzbild angestrebt. Die Qualität der Vorhersage wird durch eine Fehlerfunktion beschrieben. Das Ziel ist die Minimierung der Fehlerfunktion. Das Einlernen eines künstlichen neuronalen Netzes erfolgt bei dem Backpropagation-Verfahren durch die Änderung der Verbindungsgewichte.

Im trainierten Zustand enthalten die Verbindungsgewichte zwischen den Verarbeitungselementen Informationen bezüglich der Zusammenhänge zwischen einer Vielzahl an Eingabe-Referenzbildern und den korrespondierenden Ziel-Referenzbildern, die für Vorhersagezwecke verwendet werden können.

Eine Kreuzvalidierungsmethode kann verwendet werden, um die Daten in Trainings- und Validierungsdatensätze aufzuteilen. Der Trainingsdatensatz wird beim Backpropagation-Training der Netzwerkgewichte verwendet. Der Validierungsdatensatz wird verwendet, um zu überprüfen, mit welcher Vorhersagegenauigkeit sich das trainierte Netzwerk auf unbekannte Daten anwenden lässt.

Fig. 3 zeigt eine Ausführungsform des Verfahrens zum Trainieren des Modells des maschinellen Lernens in Form eines Ablaufschemas.

Das computer-implementierte Trainingsverfahren (100) umfasst die Schritte:

(110) Empfangen von Trainingsdaten,

o wobei die Trainingsdaten für jedes Referenzobjekt einer Vielzahl von Referenzobjekten (i) mindestens ein Eingabe-Referenzbild eines Referenzbereichs des Referenzobjekts in einem ersten Zustand und (ii) ein Ziel-Referenzbild des Referenzbereichs des Referenzobjekts in einem zweiten Zustand umfassen,

o wobei sich der zweite Zustand von dem ersten Zustand unterscheidet,

o wobei das mindestens eine Eingabe-Referenzbild eine Vielzahl an Bildelementen umfasst, wobei jedes Bildelement des mindestens einen Eingabe-Referenzbildes einen Teilbereich des Referenzbereichs repräsentiert, wobei jedes Bildelement des mindestens einen Eingabe-Referenzbildes durch einen Farbwert gekennzeichnet ist,

o wobei das Ziel-Referenzbild eine Vielzahl an Bildelementen umfasst, wobei jedes Bildelement des Ziel-Referenzbildes einen Teilbereich des Referenzbereich repräsentiert, wobei jedes Bildelement des Ziel-Referenzbildes durch einen Farbwert gekennzeichnet ist,

(120) Bereitstellen eines Modells des maschinellen Lernens,

o wobei das Modell des maschinellen Lernens konfiguriert ist, auf Basis des mindestens einen Eingabe-Referenzbildes des Referenzbereichs eines Referenzobjekts und Modellparametern ein synthetisches Referenzbild des Referenzbereichs des Referenzobjekts zu erzeugen, wobei das synthetische Referenzbild eine Vielzahl an Bildelementen umfasst, wobei jedes Bildelement des synthetischen Referenzbildes mit einem Bildelement des Ziel-Referenzbildes korrespondiert, wobei jedem Bildelement des synthetischen Referenzbildes ein vorhergesagter Farbwert zugeordnet ist, wobei das Modell des maschinellen Lernens konfiguriert ist für jeden vorhergesagten Farbwert einen Unsicherheitswert vorherzusagen,

(130) Trainieren des Modells des maschinellen Lernens, wobei das Trainieren für jedes Referenzobjekt der Vielzahl an Referenzobjekten umfasst:

(131) Eingeben des mindestens einen Eingabe-Referenzbildes in das Modell des maschinellen Lernens,

(132) Empfangen eines synthetischen Referenzbildes von dem Modell des maschinellen Lernens,

(133) Empfangen eines Unsicherheitswerts für jeden vorhergesagten Farbwert des vorhergesagten zweiten Referenzbildes,

(134) Berechnen eines Fehlers mittels einer Fehlerfunktion, wobei die Fehlerfunktion (i) den vorhergesagten Farbwert und/oder eine Abweichung zwischen dem vorhergesagten Farbwert und einem Farbwert des korrespondierenden Bildelements des Ziel-Referenzbildes und (ii) den vorhergesagten Unsicherheitswert als Parameter umfassen,

(135) Reduzieren des Fehlers durch Modifizieren von Modellparametern,

(140) Ausgeben und/oder Speichern des trainierten Modells des maschinellen Lernens und/oder Übermitteln des Modells des maschinellen Lernens an ein separates Computersystem und/oder Verwenden des trainierten Modells des maschinellen Lernens zum Erzeugen eines synthetischen Bildes und/oder zum Erzeugen mindestens eines Vertrauenswertes für ein synthetisches Bild.

Fig. 4 zeigt schematisch eine weitere Ausführungsform des computer-implementierten Verfahrens zum Trainieren des Modells des maschinellen Lernens.

Das Trainieren des Modells MLM des maschinellen Lernens erfolgt mit Trainingsdaten. Die Trainingsdaten umfassen, für jedes Referenzobjekt einer Vielzahl von Referenzobjekten, (i) mindestens ein Eingabe-Referenzbild eines Referenzbereichs des Referenzobjekts in einem ersten Zustand und (ii) ein Ziel-Referenzbild des Referenzbereichs des Referenzobjekts in einem zweiten Zustand.

In dem in Fig. 4 dargestellten Beispiel ist nur ein Satz an Trainingsdaten TD eines Referenzobjekts gezeigt; üblicherweise umfassen die Trainingsdaten eine Vielzahl dieser Datensätze für eine Vielzahl an Referenzobjekten. In dem in Fig. 4 dargestellten Beispiel umfassen die Trainingsdaten TD ein erstes Eingabe-Referenzbild $RI_1(x_{1i})$ und ein zweitens Eingabe-Referenzbild $RI_2(x_{2i})$ als Eingabedaten und ein Ziel-Referenzbild $RI_3(y_i)$ als Zieldaten.

Das erste Eingabe-Referenzbild $RI_1(x_{1i})$ repräsentiert den Referenzbereich des Referenzobjekts in einem ersten Zustand; das zweite Eingabe-Referenzbild $RI_2(x_{2i})$ repräsentiert den Referenzbereich des Referenzobjekts in einem zweiten Zustand; das Ziel-Referenzbild $RI_3(y_i)$ repräsentiert den Referenzbereich des Referenzobjekts in einem dritten Zustand. Der erste Zustand und der zweite Zustand können gleich oder verschieden sein; vorzugsweise sind die Zustände verschieden. Der dritte Zustand unterscheidet sich von dem ersten Zustand und dem zweiten Zustand. Zum Beispiel kann der Zustand eine Menge an Kontrastmittel repräsentieren, die in den Referenzbereich eingebracht wird oder worden ist. Zum Beispiel kann der Zustand einen Zeitpunkt vor und/oder nach einer Applikation eines Kontrastmittels repräsentieren.

Zum Beispiel kann das erste Eingabe-Referenzbild $RI_1(x_{1i})$ den Referenzbereich ohne oder nach Applikation einer ersten Menge eines Kontrastmittels, das zweite Eingabe-Referenzbild $RI_2(x_{2i})$ den Referenzbereich nach Applikation einer zweiten Menge des Kontrastmittels und das Ziel-Referenzbild $RI_3(y_i)$ den Referenzbereich nach Applikation einer dritten Menge des Kontrastmittels repräsentieren. Dabei kann die erste Menge kleiner als die zweite Menge und die zweite Menge kleiner als die dritte Menge sein (siehe z.B. WO2019/074938A1, WO2022184297A1).

Zum Beispiel kann das erste Eingabe-Referenzbild $RI_1(x_{1i})$ den Referenzbereich vor oder in einer ersten Zeitspanne nach Applikation eines Kontrastmittels, das zweite Eingabe-Referenzbild $RI_2(x_{2i})$ den Referenzbereich in einer zweiten Zeitspanne nach Applikation des Kontrastmittels und das Ziel-Referenzbild $RI_3(y_i)$ den Referenzbereich in einer dritten Zeitspanne nach Applikation des Kontrastmittels repräsentieren (siehe z.B. WO2021052896A1, WO2021069338A1). Dabei kann die zweite Zeitspanne zeitlich auf die erste Zeitspanne folgen und die dritte Zeitspanne kann zeitlich auf die zweite Zeitspanne folgen.

Weitere Beispiele sind in dieser Offenbarung genannt.

Das erste Eingabe-Referenzbild $RI_1(x_{1i})$ und das zweite Eingabe-Referenzbild $RI_2(x_{2i})$ dienen in dem in Fig. 4 gezeigten Beispiel als Eingabedaten; sie werden dem Modell MLM des maschinellen Lernens zugeführt. Das Modell MLM des maschinellen Lernens ist konfiguriert, auf Basis des ersten Eingabe-Referenzbildes $RI_1(x_{1i})$ und des zweiten Eingabe-Referenzbildes $RI_2(x_{2i})$ und auf Basis von Modellparametern MP ein synthetisches Referenzbild $RI_3{}^*(\hat{y}_i)$ zu erzeugen. Das synthetische Referenzbild $RI_3{}^*(\hat{y}_i)$ soll dem Ziel-Referenzbild $RI_3(y_i)$ möglichst nahekommen. Das heißt, das Ziel-Referenzbild $RI_3(y_i)$ fungiert in dem in Fig. 4 gezeigten Beispiel als Zieldaten *(ground truth)*. Das synthetische Referenzbild $RI_3{}^*(\hat{y}_i)$ soll den Referenzbereich des Referenzobjekts in dem dritten Zustand repräsentieren.

Das erste Eingabe-Referenzbild $RI_1(x_{1i})$ umfasst eine Vielzahl an Bildelementen (in Fig. 4 nicht explizit gezeigt). Jedes Bildelement *i* des ersten Eingabe-Referenzbildes $RI_1(x_{1i})$ ist durch einen Farbwert $x_{1i}$ gekennzeichnet. Das zweite Eingabe-Referenzbild $RI_2(x_{2i})$ umfasst ebenfalls eine Vielzahl an Bildelementen (in Fig. 4 nicht explizit gezeigt). Jedes Bildelement *i* des zweiten Eingabe-Referenzbildes $RI_2(x_{2i})$ ist durch einen Farbwert $x_{2i}$ gekennzeichnet. Das Ziel-Referenzbild $RI_3(y_i)$ umfasst eine Vielzahl an Bildelementen (in Fig. 4 nicht explizit gezeigt). Jedes Bildelement *i* des Ziel-Referenzbildes $RI_3(y_i)$ ist durch einen Farbwert $y_i$ gekennzeichnet.

Das Modell MLM des maschinellen Lernens wird trainiert, für jedes Bildelement *i* des synthetischen Referenzbildes $RI_3{}^*(\hat{y}_i)$ einen Farbwert $\hat{y}_i$ vorherzusagen. Die Vorhersage erfolgt auf Basis der Farbwerte $x_{1i}$ des ersten Eingabe-Referenzbildes $RI_1(x_{1i})$ und der Farbwerte $x_{2i}$ des zweiten Eingabe-Referenzbildes $RI_2(x_{2i})$.

Jeder vorhergesagte Farbwert $\hat{y}_i$ eines jeden Bildelements *i* des synthetischen Referenzbildes $PI_3{}^*(\hat{y}_i)$ soll dem jeweiligen Farbwert $y_i$ des korrespondierenden Bildelements *i* des Ziel-Referenzbildes $RI_3(y_i)$ möglichst nahekommen (im Idealfall entsprechen). Mit anderen Worten: Abweichungen zwischen dem vorhergesagten Farbwert $\hat{y}_i$ und dem jeweiligen Farbwert $y_i$ des korrespondierenden Bildelements des Ziel-Referenzbildes $RI_3(y_i)$ sollen möglichst klein sein (im Idealfall Null sein).

Das Modell MLM des maschinellen Lernens wird ferner trainiert, für jeden vorhergesagten Farbwert $\hat{y}_i$ einen Unsicherheitswert $\hat{\sigma}(x_{1i}, x_{2i})$ vorherzusagen. Der Unsicherheitswert $\hat{\sigma}(x_{1i}, x_{2i})$ ist abhängig von den Farbwerten $x_{1i}$ und $x_{2i}$ der Bildelemente des ersten Eingabe-Referenzbildes $RI_1(x_{1i})$ und des zweiten Eingabe-Referenzbildes $RI_2(x_{2i})$.

Das Trainieren des Modells MLM des maschinellen Lernens umfasst ein Minimieren einer Fehlerfunktion $\mathcal{L}$. Mit anderen Worten: für jedes Tripel aus einem vorhergesagten Farbwert $\hat{y}_i$, dem Farbwert $y_i$ und dem vorhergesagten Unsicherheitswert $\hat{\sigma}(x_{1i}, x_{2i})$ kann mittels der Fehlerfunktion $\mathcal{L}$ ein Fehler berechnet werden; durch Modifizieren von Modellparametern MP kann der ermittelte Fehler reduziert werden; Ziel kann es sein, die Modellparameter so zu modifizieren, dass der mittels der Fehlerfunktion $\mathcal{L}$ ermittelte Fehler für alle Tripel einen minimalen Wert annimmt, und/oder sich der ermittelte Fehler durch Modifizieren der Modellparameter nicht mehr reduzieren lässt. Das Modifizieren der Modellparameter MP und das Minimieren der Fehlerfunktion $\mathcal{L}$ kann in einem Optimierungsverfahren erfolgen. Die Fehlerfunktion $\mathcal{L}$ kann eine analoge Form wie die oben genannte Gleichung (1) haben oder eine andere Gleichung sein.

Das trainierte Modell des maschinellen Lernens kann nach dem Trainieren in einem Datenspeicher gespeichert und/oder ausgegeben und/oder an ein anderes Computersystem übermittelt werden. Das trainierte Modell des maschinellen Lernens kann nach dem Trainieren zum Erzeugen synthetischer Bilder genutzt werden.

Das in Fig. 4 gezeigte computer-implementierte Trainingsverfahren umfasst somit die folgenden Schritte:

- Empfangen von Trainingsdaten,

  o wobei die Trainingsdaten für jedes Referenzobjekt einer Vielzahl von Referenzobjekten (i) ein erstes Eingabe-Referenzbild eines Referenzbereichs des Referenzobjekts in einem ersten Zustand und ein zweites Eingabe-Referenzbild des Referenzobjekts in einem zweiten Zustand und (ii) ein Ziel-Referenzbild des Referenzbereichs des Referenzobjekts in einem dritten Zustand umfassen,

  o wobei sich der zweite Zustand vorzugsweise von dem ersten Zustand unterscheidet,

  o wobei sich der dritte Zustand von dem ersten Zustand und von dem zweiten Zustand unterscheidet,

  o wobei das erste Eingabe-Referenzbild eine Vielzahl an Bildelementen umfasst, wobei jedes Bildelement des ersten Eingabe-Referenzbildes einen Teilbereich des Referenzbereichs repräsentiert, wobei jedes Bildelement des ersten Eingabe-Referenzbildes durch einen Farbwert gekennzeichnet ist,

  o wobei das zweite Eingabe-Referenzbild eine Vielzahl an Bildelementen umfasst, wobei jedes Bildelement des zweiten Eingabe-Referenzbildes einen Teilbereich des Referenzbereichs repräsentiert, wobei jedes Bildelement des zweiten Eingabe-Referenzbildes durch einen Farbwert gekennzeichnet ist,

  o wobei das Ziel-Referenzbild eine Vielzahl an Bildelementen umfasst, wobei jedes Bildelement des Ziel-Referenzbildes einen Teilbereich des Referenzbereich repräsentiert, wobei jedes Bildelement des Ziel-Referenzbildes durch einen Farbwert gekennzeichnet ist,

- Bereitstellen eines Modells des maschinellen Lernens,

  o wobei das Modell des maschinellen Lernens konfiguriert ist, auf Basis eines ersten Eingabe-Referenzbildes des Referenzbereichs eines Referenzobjekts, eines zweiten Eingabe-Referenzbildes des Referenzbereichs des Referenzobjekts und Modellparametern ein synthetisches Referenzbild des Referenzbereichs des Referenzobjekts zu erzeugen, wobei das synthetische Referenzbild eine Vielzahl an Bildelementen umfasst, wobei jedes Bildelement des synthetischen Referenzbildes mit einem Bildelement des Ziel-Referenzbildes korrespondiert, wobei jedem Bildelement des synthetischen Referenzbildes ein vorhergesagter Farbwert zugeordnet ist, wobei das Modell des maschinellen Lernens konfiguriert ist für jeden vorhergesagten Farbwert einen Unsicherheitswert vorherzusagen,

- Trainieren des Modells des maschinellen Lernens, wobei das Trainieren für jedes Referenzobjekt der Vielzahl an Referenzobjekten umfasst:

  o Eingeben des ersten Eingabe-Referenzbildes und des zweiten Eingabe-Referenzbildes in das Modell des maschinellen Lernens,

  o Empfangen eines synthetischen Referenzbildes von dem Modell des maschinellen Lernens,

o Empfangen eines Unsicherheitswerts für jeden vorhergesagten Farbwert des vorhergesagten dritten Referenzbildes,

o Berechnen eines Fehlers mittels einer Fehlerfunktion, wobei die Fehlerfunktion (i) den vorhergesagten Farbwert und/oder eine Abweichung zwischen dem vorhergesagten Farbwert und einem Farbwert des korrespondierenden Bildelements des Ziel-Referenzbildes und (ii) den vorhergesagten Unsicherheitswert als Parameter umfassen,

o Reduzieren des Fehlers durch Modifizieren von Modellparametern,

- Ausgeben und/oder Speichern des trainierten Modells des maschinellen Lernens und/oder Übermitteln des Modells des maschinellen Lernens an ein separates Computersystem und/oder Verwenden des trainierten Modells des maschinellen Lernens zum Erzeugen eines synthetischen Bildes und/oder zum Erzeugen mindestens eines Vertrauenswertes für ein synthetisches Bild.

Fig. 5 zeigt eine Ausführungsform des Verfahrens zum Erzeugen mindestens eines Vertrauenswertes für ein synthetisches Bild in Form eines Ablaufdiagramms.
Das computer-implementierte Verfahren (200) umfasst die Schritte:

(210) Bereitstellen eines trainierten Modells des maschinellen Lernens,

o wobei das trainierte Modell des maschinellen Lernens anhand von Trainingsdaten trainiert wurde,

o wobei die Trainingsdaten für jedes Referenzobjekt einer Vielzahl von Referenzobjekten (i) mindestens ein Eingabe-Referenzbild eines Referenzbereichs des Referenzobjekts in einem ersten Zustand und (ii) ein Ziel-Referenzbild des Referenzbereichs des Referenzobjekts in einem zweiten Zustand umfassen, wobei das mindestens eine Eingabe-Referenzbild und das Ziel-Referenzbild jeweils eine Vielzahl an Bildelementen umfassen,

o wobei das Modell des maschinellen Lernens konfiguriert ist und trainiert wurde, für jedes Referenzobjekt auf Basis des mindestens einen Eingabe-Referenzbildes ein synthetisches Referenzbild zu erzeugen,

▪ wobei das synthetische Referenzbild eine Vielzahl an Bildelementen umfasst, wobei jedes Bildelement des synthetischen Referenzbildes mit jeweils einem Bildelement des Ziel-Referenzbildes korrespondiert,

▪ wobei das Modell des maschinellen Lernens trainiert wurde, für jedes Bildelement des synthetischen Referenzbildes einen Farbwert und einen Unsicherheitswert für den vorhergesagten Farbwert vorherzusagen,

▪ wobei das Trainieren ein Minimieren einer Fehlerfunktion umfasst, wobei die Fehlerfunktion (i) den vorhergesagten Farbwert und/oder eine Abweichung des vorhergesagten Farbwerts von einem Farbwert des korrespondierenden Bildelements des Ziel-Referenzbildes und (ii) den vorhergesagten Unsicherheitswert als Parameter umfasst,

(220) Empfangen mindestens eines Eingabe-Bildes eines Untersuchungsbereichs eines Untersuchungsobjekts, wobei das mindestens eine Eingabe-Bild den Untersuchungsbereich des Untersuchungsobjekts in dem ersten Zustand repräsentiert,

(230) Zuführen des mindestens einen Eingabe-Bildes dem trainierten Modell des maschinellen Lernens,

(240) Empfangen eines synthetischen Bildes von dem trainierten Modell des maschinellen Lernens, wobei das synthetische Bild den Untersuchungsbereich des Untersuchungsobjekts in dem zweiten Zustand repräsentiert,

(250) Empfangen eines Unsicherheitswertes für jedes Bildelement des synthetischen Bildes,

(260) Ermitteln mindestens eines Vertrauenswertes auf Basis der empfangenen Unsicherheitswerte,

(270) Ausgeben des mindestens einen Vertrauenswertes.

Fig. 6 zeigt schematisch eine weitere Ausführungsform des computer-implementiertes Verfahren zum Erzeugen eines synthetischen Bildes eines Untersuchungsbereichs eines Untersuchungsobjekts.

Das Erzeugen des synthetischen Bildes erfolgt mit einem trainierten Modell $MLM^t$ des maschinellen Lernens. Das Modell $MLM^t$ des maschinellen Lernens kann wie in Bezug zu Fig. 4 beschrieben trainiert worden sein.

Dem trainierten Modell $MLM^t$ des maschinellen Lernens wird mindestens ein Eingabe-Bild eines Untersuchungsbereichs eines Untersuchungsobjekts zugeführt. Das mindestens eine Eingabe-Bild repräsentiert den Untersuchungsbereich des Untersuchungsobjekts in einem ersten Zustand.

In dem in Fig. 6 gezeigten Beispiel handelt es sich bei dem mindestens einen Eingabe-Bild um ein erstes Eingabe-Bild $I_1(x_{1i})$ und ein zweites Eingabe-Bild $I_2(x_{2i})$. Das erste Eingabe-Bild $I_1(x_{1i})$ repräsentiert den Untersuchungsbereich des Untersuchungsobjekts in einem ersten Zustand und das zweite Eingabe-Bild $I_2(x_{2i})$ repräsentiert den Untersuchungsbereich des Untersuchungsobjekts in dem ersten Zustand oder in einem zweiten Zustand.

Zum Beispiel kann das erste Eingabe-Bild $I_1(x_{1i})$ den Untersuchungsbereich ohne oder nach Applikation einer ersten Menge eines Kontrastmittels und das zweite Eingabe-Bild $I_2(x_{2i})$ den Untersuchungsbereich nach Applikation einer zweiten Menge des Kontrastmittels repräsentieren. Dabei kann die erste Menge kleiner als die zweite Menge sein (siehe z.B. WO2019/074938A1, WO2022184297A1).

Zum Beispiel kann das erste Eingabe-Bild $I_1(x_{1i})$ den Untersuchungsbereich vor oder in einer ersten Zeitspanne nach Applikation eines Kontrastmittels und das zweite Eingabe-Bild $I_2(x_{2i})$ den Untersuchungsbereich in einer zweiten Zeitspanne nach Applikation des Kontrastmittels repräsentieren. Dabei kann die zweite Zeitspanne zeitlich auf die erste Zeitspanne folgen (siehe z.B. WO2021052896A1, WO2021069338A1).

Weitere Beispiele sind in dieser Offenbarung genannt.

Das erste Eingabe-Bild $I_1(x_{1i})$ umfasst eine Vielzahl an Bildelementen (in Fig. 6 nicht explizit dargestellt); jedes Bildelement *i* des ersten Eingabe-Bildes $I_1(x_{1i})$ repräsentiert einen Teilbereich des Untersuchungsbereichs; jedem Bildelement *i* des ersten Eingabe-Bildes $I_1(x_{1i})$ ist ein Farbwert $x_{1i}$ zugeordnet.

Das zweite Eingabe-Bild $I_2(x_{2i})$ umfasst ebenfalls eine Vielzahl an Bildelementen (in Fig. 6 nicht explizit dargestellt); jedes Bildelement *i* des zweiten Eingabe-Bildes $I_2(x_{2i})$ repräsentiert einen Teilbereich des Untersuchungsbereichs; jedem Bildelement *i* des zweiten Eingabe-Bildes $I_2(x_{2i})$ ist ein Farbwert $x_{2i}$ zugeordnet.

Das trainierte Modell $MLM^t$ des maschinellen Lernens ist konfiguriert und wurde trainiert, auf Basis der ersten Eingabe-Bildes $I_1(x_{1i})$ und des zweiten Eingabe-Bildes $I_2(x_{2i})$ ein synthetisches Bild $I_3^*(\hat{y}_i)$ zu erzeugen.

Das synthetische Bild $I_3^*(\hat{y}_i)$ repräsentiert den Untersuchungsbereich des Untersuchungsobjekts in einem dritten Zustand. Zum Beispiel kann das synthetische Bild $I_3^*(\hat{y}_i)$ den Untersuchungsbereich nach Applikation einer dritten Menge des Kontrastmittels repräsentieren. Dabei kann die dritte Menge größer als die zweite Menge sein (siehe z.B. WO2019/074938A1, WO2022184297A1). Zum Beispiel kann das synthetische Bild $I_3^*(\hat{y}_i)$ den Untersuchungsbereich in einer dritten Zeitspanne nach Applikation des Kontrastmittels repräsentieren. Dabei kann die dritte Zeitspanne zeitlich auf die zweite Zeitspanne folgen (siehe z.B. WO2021052896A1, WO2021069338A1). Weitere Beispiele sind in dieser Offenbarung genannt.

Das synthetische Bild $I_3^*(\hat{y}_i)$ kann ausgegeben (d.h. zum Beispiel auf einem Monitor angezeigt oder mit einem Drucker ausgedruckt) und/oder in einem Datenspeicher gespeichert und/oder an ein separates Computersystem übermittelt werden.

Das synthetische Bild $I_3^*(\hat{y}_i)$ umfasst eine Vielzahl an Bildelementen (in Fig. 6 nicht explizit dargestellt); jedes Bildelement *i* des synthetischen Bildes $I_3^*(\hat{y}_i)$ repräsentiert einen Teilbereich des Untersuchungsbereichs

Das trainierte Modell $MLM^t$ des maschinellen Lernens ist konfiguriert und wurde trainiert, auf Basis der ersten Eingabe-Bildes $I_1(x_{1i})$ und des zweiten Eingabe-Bildes $I_2(x_{2i})$ für jedes Bildelement *i* des synthetischen Bildes $I_3^*(\hat{y}_i)$ einen Farbwert $\hat{y}_i$ vorauszusagen. Mit anderen Worten: jedem Bildelement *i* des synthetischen Bildes $I_3^*(\hat{y}_i)$ ist ein vorhergesagter Farbwert $\hat{y}_i$ zugeordnet.

Das trainierte Modell $MLM^t$ des maschinellen Lernens ist ferner konfiguriert und wurde ferner trainiert, für jeden vorhergesagten Farbwert $\hat{y}_i$ einen Unsicherheitswert $\hat{\sigma}(x_{1i}, x_{2i})$ vorherzusagen.

Auf Basis der Unsicherheitswerte, die für die vorhergesagten Farbwerte der Bildelemente eines synthetischen Bildes vorhergesagte wurden, lässt sich mindestens ein Vertrauenswert ermitteln.

Das in Fig. 6 dargestellte Verfahren zum Erzeugen mindestens eines Vertrauenswertes für ein synthetisches Bild umfasst somit die folgenden Schritte:

- Bereitstellen eines trainierten Modells des maschinellen Lernens,

  o wobei das trainierte Modell des maschinellen Lernens anhand von Trainingsdaten trainiert wurde,

  o wobei die Trainingsdaten für jedes Referenzobjekt einer Vielzahl von Referenzobjekten (i) ein erstes Eingabe-Referenzbild eines Referenzbereichs des Referenzobjekts in einem ersten Zustand und ein zweites Eingabe-Referenzbild des Referenzbereichs des Referenzobjekts in einem zweiten Zustand und (ii) ein Ziel-Referenzbild

des Referenzbereichs des Referenzobjekts in einem dritten Zustand umfassen, wobei das erste Eingabe-Referenzbild, das zweite Eingabe-Referenzbild und das Ziel-Referenzbild jeweils eine Vielzahl an Bildelementen umfassen,

o wobei das Modell des maschinellen Lernens konfiguriert ist und trainiert wurde, für jedes Referenzobjekt auf Basis des ersten Eingabe-Referenzbildes und des zweiten Eingabe-Referenzbildes ein synthetisches Referenzbild zu erzeugen,

▪ wobei das synthetische Referenzbild eine Vielzahl an Bildelementen umfasst, wobei jedes Bildelement des synthetischen Referenzbildes mit jeweils einem Bildelement des Ziel-Referenzbildes korrespondiert,

▪ wobei das Modell des maschinellen Lernens trainiert wurde, für jedes Bildelement des synthetischen Referenzbildes einen Farbwert und einen Unsicherheitswert für den vorhergesagten Farbwert vorherzusagen,

▪ wobei das Trainieren ein Minimieren einer Fehlerfunktion umfasst, wobei die Fehlerfunktion (i) den vorhergesagten Farbwert und/oder eine Abweichung des vorhergesagten Farbwerts von einem Farbwert des korrespondierenden Bildelements des Ziel-Referenzbildes und (ii) den vorhergesagten Unsicherheitswert als Parameter umfasst,

- Empfangen eines ersten Eingabe-Bildes und eines zweiten Eingabe-Bildes eines Untersuchungsbereichs eines Untersuchungsobjekts, wobei das erste Eingabe-Bild den Untersuchungsbereich des Untersuchungsobjekts in dem ersten Zustand und das zweite Eingabe-Bild den Untersuchungsbereich des Untersuchungsobjekts in dem zweiten Zustand repräsentiert,
- Zuführen des ersten Eingabe-Bildes und des zweiten Eingabe-Bildes dem trainierten Modell des maschinellen Lernens,
- Empfangen eines synthetischen Bildes von dem trainierten Modell des maschinellen Lernens, wobei das synthetische Bild den Untersuchungsbereich des Untersuchungsobjekts in dem dritten Zustand repräsentiert,
- Empfangen eines Unsicherheitswertes für jedes Bildelement des synthetischen Bildes,
- Ermitteln mindestens eines Vertrauenswertes auf Basis der empfangenen Unsicherheitswerte,
- Ausgeben des mindestens einen Vertrauenswertes.

Weitere Ausführungsformen der vorliegenden Offenbarung sind:

1. Ein computer-implementiertes Verfahren umfassend:

- Bereitstellen eines trainierten Modells des maschinellen Lernens,

o wobei das trainierte Modell des maschinellen Lernens anhand von Trainingsdaten trainiert wurde,

o wobei die Trainingsdaten für jedes Referenzobjekt einer Vielzahl von Referenzobjekten (i) mindestens ein Eingabe-Referenzbild eines Referenzbereichs des Referenzobjekts in einem ersten Zustand und (ii) ein Ziel-Referenzbild des Referenzbereichs des Referenzobjekts in einem zweiten Zustand umfassen, wobei das mindestens eine Eingabe-Referenzbild und das Ziel-Referenzbild jeweils eine Vielzahl an Bildelementen umfassen,

o wobei das Modell des maschinellen Lernens konfiguriert ist und trainiert wurde, für jedes Referenzobjekt auf Basis des mindestens einen Eingabe-Referenzbildes ein synthetisches Referenzbild zu erzeugen,

▪ wobei das synthetische Referenzbild eine Vielzahl an Bildelementen umfasst, wobei jedes Bildelement des synthetischen Referenzbildes mit jeweils einem Bildelement des Ziel-Referenzbildes korrespondiert,

- wobei das Modell des maschinellen Lernens trainiert wurde, für jedes Bildelement des synthetischen Referenzbildes einen Farbwert und einen Unsicherheitswert für den vorhergesagten Farbwert vorherzusagen,

- wobei das Trainieren ein Minimieren einer Fehlerfunktion umfasst, wobei die Fehlerfunktion (i) den vorhergesagten Farbwert und/oder eine Abweichung des vorhergesagten Farbwerts von einem Farbwert des korrespondierenden Bildelements des Ziel-Referenzbildes und (ii) den vorhergesagten Unsicherheitswert als Parameter umfasst,

- Empfangen mindestens eines Eingabe-Bildes eines Untersuchungsbereichs eines Untersuchungsobjekts, wobei das mindestens eine Eingabe-Bild den Untersuchungsbereich des Untersuchungsobjekts in dem ersten Zustand repräsentiert,

- Zuführen des mindestens einen Eingabe-Bildes dem trainierten Modell des maschinellen Lernens,

- Empfangen eines synthetischen Bildes von dem trainierten Modell des maschinellen Lernens, wobei das synthetische Bild den Untersuchungsbereich des Untersuchungsobjekts in dem zweiten Zustand repräsentiert,

- Empfangen eines Unsicherheitswertes für jedes Bildelement des synthetischen Bildes,

- Ermitteln mindestens eines Vertrauenswertes auf Basis der empfangenen Unsicherheitswerte,

- Ausgeben des mindestens einen Vertrauenswertes.

2. Das Verfahren gemäß der Ausführungsform 1, ferner umfassend:

- Erzeugen einer Vertrauensrepräsentation, wobei die Vertrauensrepräsentation eine Vielzahl an Bildelementen umfasst, wobei jedes Bildelement der Vielzahl an Bildelementen einen Teilbereich des Untersuchungsbereichs repräsentiert, wobei jedes Bildelement der Vielzahl an Bildelementen mit jeweils einem Bildelement des synthetischen Bildes korrespondiert, wobei jedes Bildelement der Vielzahl an Bildelementen einen Farbwert aufweist, wobei der Farbwert mit dem jeweiligen Unsicherheitswert des vorhergesagten Farbwerts des korrespondierenden Bildelements des synthetischen Bildes korreliert,

- Ausgeben und/oder Speichern der Vertrauensrepräsentation und/oder Übermitteln der Vertrauensrepräsentation an ein separates Computersystem, vorzugsweise in einer überlagerten Darstellung mit dem synthetischen Bild.

3. Das Verfahren gemäß einer der Ausführungsformen 1 oder 2, ferner umfassend:

- Ermitteln eines Vertrauenswertes für einen oder mehrere Teilbereiche des synthetischen Bildes,

- Ausgeben des Vertrauenswertes für den einen oder die mehreren Teilbereiche des synthetischen Bildes.

4. Das Verfahren gemäß einer der Ausführungsformen 1 bis 3, ferner umfassend:

- Prüfen, ob der positiv mit der Vertrauenswürdigkeit korrelierende Vertrauenswert unterhalb eines vordefinierten Schwellenwerts liegt oder ob der negativ mit der Vertrauenswürdigkeit korrelierende Vertrauenswert oberhalb eines vordefinierten Schwellenwerts liegt;

- für den Fall, dass der positiv mit der Vertrauenswürdigkeit korrelierende Vertrauenswert unterhalb eines vordefinierten Schwellenwerts liegt oder ob der negativ mit der Vertrauenswürdigkeit korrelierende Vertrauenswert oberhalb eines vordefinierten Schwellenwerts liegt: Ausgeben einer Mitteilung, dass das synthetische Bild eine Unsicherheit aufweist.

5. Ein Computersystem umfassend Mittel zur Ausführung des Verfahrens gemäß einer der Ausführungsformen 1 bis 4.

6. Ein Computerprogramm umfassend Befehle, die bei der Ausführung des Computerprogramms durch ein Computersystem dieses veranlassen, das Verfahren gemäß einer der Ausführungsformen 1 bis 4 auszuführen.

7. Computerlesbares Speichermedium umfassend Befehle, die bei der Ausführung durch ein Computersystem dieses veranlassen, das Verfahren gemäß einer der Ausführungsformen 1 bis 4 auszuführen.

8. Computersystem umfassend eine Empfangseinheit, eine Steuer- und Recheneinheit und eine Ausgabeeinheit, wobei die Steuer- und Recheneinheit konfiguriert ist,

- ein trainiertes Modell des maschinellen Lernens bereitzustellen,

  o wobei das trainierte Modell des maschinellen Lernens anhand von Trainingsdaten trainiert wurde,

  o wobei die Trainingsdaten für jedes Referenzobjekt einer Vielzahl von Referenzobjekten (i) mindestens ein Eingabe-Referenzbild eines Referenzbereichs des Referenzobjekts in einem ersten Zustand und (ii) ein Ziel-Referenzbild des Referenzbereichs des Referenzobjekts in einem zweiten Zustand umfassen, wobei das mindestens eine Eingabe-Referenzbild und das Ziel-Referenzbild jeweils eine Vielzahl an Bildelementen umfassen,

  o wobei das Modell des maschinellen Lernens konfiguriert ist und trainiert wurde, für jedes Referenzobjekt auf Basis des mindestens einen Eingabe-Referenzbildes ein synthetisches Referenzbild zu erzeugen,

    ▪ wobei das synthetische Referenzbild eine Vielzahl an Bildelementen umfasst, wobei jedes Bildelement des synthetischen Referenzbildes mit jeweils einem Bildelement des Ziel-Referenzbildes korrespondiert,

    ▪ wobei das Modell des maschinellen Lernens trainiert wurde, für jedes Bildelement des synthetischen Referenzbildes einen Farbwert und einen Unsicherheitswert für den vorhergesagten Farbwert vorherzusagen,

    ▪ wobei das Trainieren ein Minimieren einer Fehlerfunktion umfasst, wobei die Fehlerfunktion (i) den vorhergesagten Farbwert und/oder eine Abweichung des vorhergesagten Farbwerts von einem Farbwert des korrespondierenden Bildelements des Ziel-Referenzbildes und (ii) den vorhergesagten Unsicherheitswert als Parameter umfasst,

- die Empfangseinheit zu veranlassen, mindestens ein Eingabe-Bild eines Untersuchungsbereichs eines Untersuchungsobjekts zu empfangen, wobei das mindestens eine Eingabe-Bild einen Untersuchungsbereich eines Untersuchungsobjekts in dem ersten Zustand repräsentiert,

- das mindestens eine Eingabe-Bild einem trainierten Modell des maschinellen Lernens zuzuführen,

- von dem trainierten Modell des maschinellen Lernens ein synthetisches Bild zu empfangen, wobei das synthetische Bild den Untersuchungsbereich des Untersuchungsobjekts in dem zweiten Zustand repräsentiert,

- von dem trainierten Modell des maschinellen Lernens einen Unsicherheitswert für jedes Bildelement des synthetischen Bildes zu empfangen,

- mindestens einen Vertrauenswert auf Basis der empfangenen Unsicherheitswerte zu ermitteln,

die Ausgabeeinheit zu veranlassen, den mindestens einen Vertrauenswert auszugeben, in einem Datenspeicher zu speichern und/oder an ein separates Computersystem zu übermitteln.

9. Ein computerlesbares Speichermedium umfassend ein Computerprogramm, das, wenn es in einen Arbeitsspeicher eines Computersystem geladen wird, das Computersystem veranlasst, folgende Schritte auszuführen:

- Bereitstellen eines trainierten Modells des maschinellen Lernens,

  o wobei das trainierte Modell des maschinellen Lernens anhand von Trainingsdaten trainiert wurde,

o wobei die Trainingsdaten für jedes Referenzobjekt einer Vielzahl von Referenzobjekten (i) mindestens ein Eingabe-Referenzbild eines Referenzbereichs des Referenzobjekts in einem ersten Zustand und (ii) ein Ziel-Referenzbild des Referenzbereichs des Referenzobjekts in einem zweiten Zustand umfassen, wobei das mindestens eine Eingabe-Referenzbild und das Ziel-Referenzbild jeweils eine Vielzahl an Bildelementen umfassen,

o wobei das Modell des maschinellen Lernens konfiguriert ist und trainiert wurde, für jedes Referenzobjekt auf Basis des mindestens einen Eingabe-Referenzbildes ein synthetisches Referenzbild zu erzeugen,

▪ wobei das synthetische Referenzbild eine Vielzahl an Bildelementen umfasst, wobei jedes Bildelement des synthetischen Referenzbildes mit jeweils einem Bildelement des Ziel-Referenzbildes korrespondiert,

▪ wobei das Modell des maschinellen Lernens trainiert wurde, für jedes Bildelement des synthetischen Referenzbildes einen Farbwert und einen Unsicherheitswert für den vorhergesagten Farbwert vorherzusagen,

▪ wobei das Trainieren ein Minimieren einer Fehlerfunktion umfasst, wobei die Fehlerfunktion (i) den vorhergesagten Farbwert und/oder eine Abweichung des vorhergesagten Farbwerts von einem Farbwert des korrespondierenden Bildelements des Ziel-Referenzbildes und (ii) den vorhergesagten Unsicherheitswert als Parameter umfasst,

- Empfangen mindestens eines Eingabe-Bildes eines Untersuchungsbereichs eines Untersuchungsobjekts, wobei das mindestens eine Eingabe-Bild den Untersuchungsbereich des Untersuchungsobjekts in dem ersten Zustand repräsentiert,
- Zuführen des mindestens einen Eingabe-Bildes dem trainierten Modell des maschinellen Lernens,
- Empfangen eines synthetischen Bildes von dem trainierten Modell des maschinellen Lernens, wobei das synthetische Bild den Untersuchungsbereich des Untersuchungsobjekts in dem zweiten Zustand repräsentiert,
- Empfangen eines Unsicherheitswertes für jedes Bildelement des synthetischen Bildes,
- Ermitteln mindestens eines Vertrauenswertes auf Basis der empfangenen Unsicherheitswerte,
- Ausgeben des mindestens einen Vertrauenswertes.

10. Ein Kontrastmittel zur Verwendung in einem radiologischen Untersuchungsverfahren, wobei das radiologische Untersuchungsverfahren umfasst:

- Bereitstellen eines trainierten Modells des maschinellen Lernens,

o wobei das trainierte Modell des maschinellen Lernens anhand von Trainingsdaten trainiert wurde,

o wobei die Trainingsdaten für jedes Referenzobjekt einer Vielzahl von Referenzobjekten (i) mindestens ein Eingabe-Referenzbild eines Referenzbereichs des Referenzobjekts in einem ersten Zustand und (ii) ein Ziel-Referenzbild des Referenzbereichs des Referenzobjekts in einem zweiten Zustand umfassen,

▪ wobei das mindestens eine Eingabe-Referenzbild und das Ziel-Referenzbild jeweils eine Vielzahl an Bildelementen umfassen,

▪ wobei das mindestens eine Eingabe-Referenzbild eine radiologische Referenzaufnahme des Referenzbereichs des Referenzobjekts in dem ersten Zustand umfasst,

▪ wobei das Ziel-Referenzbild eine radiologische Referenzaufnahme des Referenzbereichs des Referenzobjekts in dem zweiten Zustand umfasst,

▪ wobei der erste Zustand den Referenzbereich des Referenzobjekts in einer ersten Zeitspanne vor

und/oder nach der Applikation des Kontrastmittels repräsentiert und der zweite Zustand den Referenzbereich des Referenzobjekts in einer zweiten Zeitspanne nach der Applikation des Kontrastmittels repräsentiert, und/oder der erste Zustand den Referenzbereich des Referenzobjekts vor und/oder nach der Applikation einer ersten Menge des Kontrastmittels repräsentiert und der zweite Zustand den Referenzbereich des Referenzobjekts nach der Applikation einer zweiten Menge des Kontrastmittels repräsentiert,

o wobei das mindestens eine Eingabe-Referenzbild und das Ziel-Referenzbild jeweils eine Vielzahl an Bildelementen umfassen,

o wobei das Modell des maschinellen Lernens konfiguriert ist und trainiert wurde, für jedes Referenzobjekt auf Basis des mindestens einen Eingabe-Referenzbildes ein synthetisches Referenzbild zu erzeugen,

▪ wobei das synthetische Referenzbild eine Vielzahl an Bildelementen umfasst, wobei jedes Bildelement des synthetischen Referenzbildes mit jeweils einem Bildelement des Ziel-Referenzbildes korrespondiert,

▪ wobei das Modell des maschinellen Lernens trainiert wurde, für jedes Bildelement des synthetischen Referenzbildes einen Farbwert und einen Unsicherheitswert für den vorhergesagten Farbwert vorherzusagen,

▪ wobei das Trainieren ein Minimieren einer Fehlerfunktion umfasst, wobei die Fehlerfunktion (i) den vorhergesagten Farbwert und/oder eine Abweichung des vorhergesagten Farbwerts von einem Farbwert des korrespondierenden Bildelements des Ziel-Referenzbildes und (ii) den vorhergesagten Unsicherheitswert als Parameter umfasst,

- Empfangen mindestens eines Eingabe-Bildes eines Untersuchungsbereichs eines Untersuchungsobjekts, wobei das mindestens eine Eingabe-Bild eine radiologische Aufnahme des Untersuchungsbereichs des Untersuchungsobjekts in dem ersten Zustand umfasst,

- Zuführen des mindestens einen Eingabe-Bildes dem trainierten Modell des maschinellen Lernens,

- Empfangen eines synthetischen Bildes von dem trainierten Modell des maschinellen Lernens, wobei das synthetische Bild eine synthetische radiologische Aufnahme umfasst, die den Untersuchungsbereich des Untersuchungsobjekts in dem zweiten Zustand repräsentiert,

- Empfangen eines Unsicherheitswertes für jedes Bildelement des synthetischen Bildes,

- Ermitteln mindestens eines Vertrauenswertes auf Basis der empfangenen Unsicherheitswerte,

- Ausgeben des mindestens einen Vertrauenswertes.

11. Eine Verwendung eines Kontrastmittels in einem radiologischen Untersuchungsverfahren, wobei das radiologische Untersuchungsverfahren umfasst:

- Bereitstellen eines trainierten Modells des maschinellen Lernens,

o wobei das trainierte Modell des maschinellen Lernens anhand von Trainingsdaten trainiert wurde,

o wobei die Trainingsdaten für jedes Referenzobjekt einer Vielzahl von Referenzobjekten (i) mindestens ein Eingabe-Referenzbild eines Referenzbereichs des Referenzobjekts in einem ersten Zustand und (ii) ein Ziel-Referenzbild des Referenzbereichs des Referenzobjekts in einem zweiten Zustand umfassen,

▪ wobei das mindestens eine Eingabe-Referenzbild und das Ziel-Referenzbild jeweils eine Vielzahl an Bildelementen umfassen,

▪ wobei das mindestens eine Eingabe-Referenzbild eine radiologische Referenzaufnahme des Referenzbereichs des Referenzobjekts in dem ersten Zustand umfasst,

- ▪ wobei das Ziel-Referenzbild eine radiologische Referenzaufnahme des Referenzbereichs des Referenzobjekts in dem zweiten Zustand umfasst,

- ▪ wobei der erste Zustand den Referenzbereich des Referenzobjekts in einer ersten Zeitspanne vor und/oder nach der Applikation des Kontrastmittels repräsentiert und der zweite Zustand den Referenzbereich des Referenzobjekts in einer zweiten Zeitspanne nach der Applikation des Kontrastmittels repräsentiert, und/oder der erste Zustand den Referenzbereich des Referenzobjekts vor und/oder nach der Applikation einer ersten Menge des Kontrastmittels repräsentiert und der zweite Zustand den Referenzbereich des Referenzobjekts nach der Applikation einer zweiten Menge des Kontrastmittels repräsentiert,

o wobei das mindestens eine Eingabe-Referenzbild und das Ziel-Referenzbild jeweils eine Vielzahl an Bildelementen umfassen,

o wobei das Modell des maschinellen Lernens konfiguriert ist und trainiert wurde, für jedes Referenzobjekt auf Basis des mindestens einen Eingabe-Referenzbildes ein synthetisches Referenzbild zu erzeugen,

- ▪ wobei das synthetische Referenzbild eine Vielzahl an Bildelementen umfasst, wobei jedes Bildelement des synthetischen Referenzbildes mit jeweils einem Bildelement des Ziel-Referenzbildes korrespondiert,

- ▪ wobei das Modell des maschinellen Lernens trainiert wurde, für jedes Bildelement des synthetischen Referenzbildes einen Farbwert und einen Unsicherheitswert für den vorhergesagten Farbwert vorherzusagen,

- ▪ wobei das Trainieren ein Minimieren einer Fehlerfunktion umfasst, wobei die Fehlerfunktion (i) den vorhergesagten Farbwert und/oder eine Abweichung des vorhergesagten Farbwerts von einem Farbwert des korrespondierenden Bildelements des Ziel-Referenzbildes und (ii) den vorhergesagten Unsicherheitswert als Parameter umfasst,

- Empfangen mindestens eines Eingabe-Bildes eines Untersuchungsbereichs eines Untersuchungsobjekts, wobei das mindestens eine Eingabe-Bild eine radiologische Aufnahme des Untersuchungsbereichs des Untersuchungsobjekts in dem ersten Zustand umfasst,

- Zuführen des mindestens einen Eingabe-Bildes dem trainierten Modell des maschinellen Lernens,

- Empfangen eines synthetischen Bildes von dem trainierten Modell des maschinellen Lernens, wobei das synthetische Bild eine synthetische radiologische Aufnahme umfasst, die den Untersuchungsbereich des Untersuchungsobjekts in dem zweiten Zustand repräsentiert,

- Empfangen eines Unsicherheitswertes für jedes Bildelement des synthetischen Bildes,

- Ermitteln mindestens eines Vertrauenswertes auf Basis der empfangenen Unsicherheitswerte,

- Ausgeben des mindestens einen Vertrauenswertes.

12. Ein Kit umfassend ein Kontrastmittel und ein Computerprogramm, das in einen Arbeitsspeicher eines Computersystems geladen werden kann und dort das Computersystem dazu veranlasst, folgende Schritte ausführen:

- Bereitstellen eines trainierten Modells des maschinellen Lernens,

o wobei das trainierte Modell des maschinellen Lernens anhand von Trainingsdaten trainiert wurde,

o wobei die Trainingsdaten für jedes Referenzobjekt einer Vielzahl von Referenzobjekten (i) mindestens ein Eingabe-Referenzbild eines Referenzbereichs des Referenzobjekts in einem ersten Zustand und (ii) ein Ziel-Referenzbild des Referenzbereichs des Referenzobjekts in einem zweiten Zustand umfassen,

- ▪ wobei das mindestens eine Eingabe-Referenzbild und das Ziel-Referenzbild jeweils eine Vielzahl an

Bildelementen umfassen,

- wobei das mindestens eine Eingabe-Referenzbild eine radiologische Referenzaufnahme des Referenzbereichs des Referenzobjekts in dem ersten Zustand umfasst,

- wobei das Ziel-Referenzbild eine radiologische Referenzaufnahme des Referenzbereichs des Referenzobjekts in dem zweiten Zustand umfasst,

- wobei der erste Zustand den Referenzbereich des Referenzobjekts in einer ersten Zeitspanne vor und/oder nach der Applikation des Kontrastmittels repräsentiert und der zweite Zustand den Referenzbereich des Referenzobjekts in einer zweiten Zeitspanne nach der Applikation des Kontrastmittels repräsentiert, und/oder der erste Zustand den Referenzbereich des Referenzobjekts vor und/oder nach der Applikation einer ersten Menge des Kontrastmittels repräsentiert und der zweite Zustand den Referenzbereich des Referenzobjekts nach der Applikation einer zweiten Menge des Kontrastmittels repräsentiert,

o wobei das mindestens eine Eingabe-Referenzbild und das Ziel-Referenzbild jeweils eine Vielzahl an Bildelementen umfassen,

o wobei das Modell des maschinellen Lernens konfiguriert ist und trainiert wurde, für jedes Referenzobjekt auf Basis des mindestens einen Eingabe-Referenzbildes ein synthetisches Referenzbild zu erzeugen,

- wobei das synthetische Referenzbild eine Vielzahl an Bildelementen umfasst, wobei jedes Bildelement des synthetischen Referenzbildes mit jeweils einem Bildelement des Ziel-Referenzbildes korrespondiert,

- wobei das Modell des maschinellen Lernens trainiert wurde, für jedes Bildelement des synthetischen Referenzbildes einen Farbwert und einen Unsicherheitswert für den vorhergesagten Farbwert vorherzusagen,

- wobei das Trainieren ein Minimieren einer Fehlerfunktion umfasst, wobei die Fehlerfunktion (i) den vorhergesagten Farbwert und/oder eine Abweichung des vorhergesagten Farbwerts von einem Farbwert des korrespondierenden Bildelements des Ziel-Referenzbildes und (ii) den vorhergesagten Unsicherheitswert als Parameter umfasst,

- Empfangen mindestens eines Eingabe-Bildes eines Untersuchungsbereichs eines Untersuchungsobjekts, wobei das mindestens eine Eingabe-Bild eine radiologische Aufnahme des Untersuchungsbereichs des Untersuchungsobjekts in dem ersten Zustand umfasst,

- Zuführen des mindestens einen Eingabe-Bildes dem trainierten Modell des maschinellen Lernens,

- Empfangen eines synthetischen Bildes von dem trainierten Modell des maschinellen Lernens, wobei das synthetische Bild eine synthetische radiologische Aufnahme umfasst, die den Untersuchungsbereich des Untersuchungsobjekts in dem zweiten Zustand repräsentiert,

- Empfangen eines Unsicherheitswertes für jedes Bildelement des synthetischen Bildes,

- Ermitteln mindestens eines Vertrauenswertes auf Basis der empfangenen Unsicherheitswerte,

- Ausgeben des mindestens einen Vertrauenswertes.

13. Eine der Ausführungsformen 1 bis 12, wobei jedes Referenzobjekt und das Untersuchungsobjekt Säugetiere, vorzugsweise Menschen sind, wobei der Referenzbereich ein Teil des Referenzobjekts ist, wobei der Referenzbereich für alle Referenzobjekte der gleiche Teil des jeweiligen Referenzobjekts ist, wobei der Untersuchungsbereich der entsprechende Teil des Untersuchungsobjekts ist.

14. Eine der Ausführungsformen 1 bis 13, wobei das mindestens eine Eingabe-Referenzbild mindestens eine CT-Aufnahme und/oder eine MRT-Aufnahme umfasst, wobei das Ziel-Referenzbild eine CT-Aufnahme oder eine MRT-Aufnahme ist, wobei das synthetische Referenzbild eine synthetische CT-Aufnahme oder eine synthetische MRT-Aufnahme ist, wobei das mindestens eine Eingabe-Bild mindestens eine CT-Aufnahme und/oder eine MRT-Aufnahme umfasst, wobei das synthetische Bild eine synthetische CT-Aufnahme oder eine synthetische MRT-Aufnahme ist.

15. Eine der Ausführungsformen 1 bis 14,wobei das mindestens eine Eingabe-Referenzbild eine Zahl *m* an Eingabe-Referenzbildern umfasst, wobei *m* eine ganze Zahl größer als Null ist, wobei jedes der *m* Eingabe-Referenzbilder den Referenzbereich des Referenzobjekts in einem von *p* Zuständen repräsentiert, wobei *p* eine ganze Zahl ist, die die Werte von 1 bis *m* annehmen kann, wobei das Ziel-Referenzbild und das synthetische Referenzbild den Referenzbereich des Referenzobjekts in einem Zustand repräsentieren, der sich von den *p* Zuständen unterscheidet.

16. Eine der Ausführungsformen 1 bis 15, wobei das mindestens eine Eingabe-Bild eine Zahl *m* an Eingabe-Bildern umfasst, wobei *m* eine ganze Zahl größer als Null ist, wobei jedes der *m* Eingabe-Bilder den Untersuchungsbereich des Untersuchungsobjekts in einem von *p* Zuständen repräsentiert, wobei *p* eine ganze Zahl ist, die die Werte von 1 bis *m* annehmen kann, wobei das synthetische Bild den Untersuchungsbereich des Untersuchungsobjekts in einem Zustand repräsentiert, der sich von den *p* Zuständen unterscheidet.

17. Eine der Ausführungsformen 1 bis 16, wobei der erste Zustand und der zweite Zustand jeweils eine Menge an Kontrastmittel angeben, die dem Referenzobjekt und dem Untersuchungsobjekt verabreicht worden ist.

18. Eine der Ausführungsformen 1 bis 17, wobei der erste Zustand und der zweite Zustand jeweils eine Zeitspanne angeben, die seit einem Applizieren eines Kontrastmittels vergangen ist.

19. Eine der Ausführungsformen 1 bis 18, wobei der erste Zustand und der zweite Zustand jeweils einen Zeitpunkt vor und/oder nach einer Applikation eines Kontrastmittels angeben.

20. Eine der Ausführungsformen 1 bis 19, wobei der erste Zustand und der zweite Zustand jeweils ein spezifisches Kontrastmittel angeben, das dem Referenzobjekt und dem Untersuchungsobjekt verabreicht worden ist.

21. Eine der Ausführungsformen 1 bis 20, wobei der erste Zustand und der zweite Zustand jeweils eine Modalität angeben.

22. Eine der Ausführungsformen 1 bis 21, wobei der erste Zustand und der zweite Zustand jeweils ein Messprotokoll einer radiologischen Untersuchungsmethode angeben, der das Referenzobjekt und das Untersuchungsobjekt unterzogen wurden.

23. Eine der Ausführungsformen 1 bis 22, wobei das mindestens eine Eingabe-Referenzbild eine oder mehrere radiologische Aufnahmen des Referenzbereichs vor und/oder nach einer Applikation einer ersten Menge eines Kontrastmittels umfasst und das Ziel-Referenzbild sowie das synthetische Referenzbild jeweils eine radiologische Aufnahme des Untersuchungsbereichs nach der Applikation einer zweiten Menge eines Kontrastmittels sind, wobei die zweite Menge vorzugsweise größer als die erste Menge ist.

24. Eine der Ausführungsformen 1 bis 23, wobei das mindestens eine Eingabe-Bild eine oder mehrere radiologische Aufnahmen des Untersuchungsbereichs vor und/oder nach einer Applikation einer ersten Menge eines Kontrastmittels umfasst und das synthetische Bild eine synthetische radiologische Aufnahme des Untersuchungsbereichs nach der Applikation einer zweiten Menge eines Kontrastmittels ist, wobei die zweite Menge vorzugsweise größer als die erste Menge ist.

25. Eine der Ausführungsformen 1 bis 24, wobei das mindestens eine Eingabe-Referenzbild mindestens eine CT-Aufnahme vor und/oder nach Applikation einer ersten Menge eines MRT-Kontrastmittels umfasst, und das Ziel-Referenzbild sowie das synthetische Referenzbild jeweils eine CT-Aufnahme nach Applikation einer zweiten Menge des MRT-Kontrastmittels sind, wobei die zweite Menge vorzugsweise größer als die erste Menge und vorzugsweise größer als die Standardmenge des MRT-Kontrastmittels für MRT-Untersuchungen ist.

26. Eine der Ausführungsformen 1 bis 25, wobei das mindestens eine Eingabe-Bild mindestens eine CT-Aufnahme vor und/oder nach Applikation einer ersten Menge eines MRT-Kontrastmittels umfasst, und das synthetische Bild eine synthetische CT-Aufnahme nach Applikation einer zweiten Menge des MRT-Kontrastmittels ist, wobei die zweite Menge vorzugsweise größer als die erste Menge und vorzugsweise größer als die Standardmenge des MRT-Kontrastmittels für MRT-Untersuchungen ist.

27. Eine der Ausführungsformen 1 bis 26, wobei das mindestens eine Eingabe-Referenzbild eine oder mehrere radiologische Aufnahmen der Leber oder eines Teils der Leber eines Referenzobjekts umfasst, die die Leber oder den Teil der Leber in der nativen Phase und/oder in der arteriellen Phase und/oder in der portalvenösen Phase und/oder in der Übergangsphase nach der Applikation eines hepatobiliären Kontrastmittels repräsentieren, und das Ziel-Referenzbild sowie das synthetische Referenzbild jeweils eine radiologische Aufnahme sind, die die Leber oder den Teil der Leber in der hepatobiliären Phase nach der Applikation des hepatobiliären Kontrastmittels repräsentiert.

28. Eine der Ausführungsformen 1 bis 27, wobei das mindestens eine Eingabe-Bild eine oder mehrere radiologische Aufnahmen der Leber oder eines Teils der Leber eines Untersuchungsobjekts umfasst, die die Leber oder den Teil der Leber in der nativen Phase und/oder in der arteriellen Phase und/oder in der portalvenösen Phase und/oder in der Übergangsphase nach der Applikation eines hepatobiliären Kontrastmittels repräsentieren, und das synthetische Bild eine synthetische radiologische Aufnahme ist, die die Leber oder den Teil der Leber in der hepatobiliären Phase nach der Applikation des hepatobiliären Kontrastmittels repräsentiert.

29. Eine der Ausführungsformen 1 bis 28, wobei das mindestens eine Eingabe-Referenzbild eine oder mehrere CT-Aufnahmen des Referenzbereichs des Referenzobjekts umfasst, und das Ziel-Referenzbild sowie das synthetische Referenzbild jeweils eine CT-Aufnahme des Untersuchungsbereichs des Untersuchungsobjekts sind.

30. Eine der Ausführungsformen 1 bis 29, wobei das mindestens eine Eingabe-Bild eine oder mehrere CT-Aufnahmen des Untersuchungsbereichs des Untersuchungsobjekts umfasst, und das synthetische Bild eine synthetische CT-Aufnahme des Untersuchungsbereichs des Untersuchungsobjekts ist.

31. Eine der Ausführungsformen 1 bis 30, wobei das mindestens eine Eingabe-Referenzbild eine oder mehrere MRT-Aufnahmen des Referenzbereichs des Referenzobjekts umfasst, und das Ziel-Referenzbild sowie das synthetische Referenzbild jeweils eine MRT-Aufnahme des Untersuchungsbereichs des Untersuchungsobjekts sind.

32. Eine der Ausführungsformen 1 bis 31, wobei das mindestens eine Eingabe-Bild eine oder mehrere MRT-Aufnahmen des Untersuchungsbereichs des Untersuchungsobjekts umfasst, und das synthetische Bild eine synthetische MRT-Aufnahme des Untersuchungsbereichs des Untersuchungsobjekts ist.

33. Eine der Ausführungsformen 1 bis 32, wobei das mindestens eine Eingabe-Referenzbild eine oder mehrere MRT-Aufnahmen des Referenzbereichs des Referenzobjekts umfasst, und das Ziel-Referenzbild sowie das synthetische Referenzbild jeweils eine CT-Aufnahme des Untersuchungsbereichs des Untersuchungsobjekts sind.

34. Eine der Ausführungsformen 1 bis 33, wobei das mindestens eine Eingabe-Bild eine oder mehrere MRT-Aufnahmen des Untersuchungsbereichs des Untersuchungsobjekts umfasst, und das synthetische Bild eine synthetische CT-Aufnahme des Untersuchungsbereichs des Untersuchungsobjekts ist.

35. Eine der Ausführungsformen 1 bis 34, wobei das mindestens eine Eingabe-Referenzbild eine oder mehrere CT-Aufnahmen des Referenzbereichs des Referenzobjekts umfasst, und das Ziel-Referenzbild sowie das synthetische Referenzbild jeweils eine MRT-Aufnahme des Untersuchungsbereichs des Untersuchungsobjekts sind.

36. Eine der Ausführungsformen 1 bis 35, wobei das mindestens eine Eingabe-Bild eine oder mehrere CT-Aufnahmen des Untersuchungsbereichs des Untersuchungsobjekts umfasst, und das synthetische Bild eine synthetische MRT-Aufnahme des Untersuchungsbereichs des Untersuchungsobjekts ist.

37. Eine der Ausführungsformen 1 bis 36, wobei der erste Zustand den Referenzbereich des Referenzobjekts und den Untersuchungsbereich des Untersuchungsobjekts in einer ersten Zeitspanne vor und/oder nach der Applikation eines Kontrastmittels repräsentiert und der zweite Zustand den Referenzbereich des Referenzobjekts und den Untersuchungsbereich des Untersuchungsobjekts in einer zweiten Zeitspanne nach der Applikation des Kontrastmittels repräsentiert,

38. Eine der Ausführungsformen 1 bis 37, wobei der erste Zustand den Referenzbereich des Referenzobjekts und den Untersuchungsbereich des Untersuchungsobjekts vor und/oder nach der Applikation einer ersten Menge des Kontrastmittels repräsentiert und der zweite Zustand den Referenzbereich des Referenzobjekts und den Untersuchungsbereich des Untersuchungsobjekts nach der Applikation einer zweiten Menge des Kontrastmittels repräsentiert

39. Eine der Ausführungsformen 1 bis 38, wobei das mindestens eine Eingabe-Bild eine erste radiologische Aufnahme und eine zweite radiologische Aufnahme umfasst, wobei die erste radiologische Aufnahme den Untersuchungsbereich des Untersuchungsobjekts ohne Kontrastmittel oder nach Applikation einer ersten Menge eines Kontrastmittels repräsentiert und die zweite radiologische Aufnahme den Untersuchungsbereich des Untersuchungsobjekts nach Applikation einer zweiten Menge des Kontrastmittels repräsentiert,

40. Eine der Ausführungsformen 1 bis 39, wobei das synthetische Bild eine synthetische radiologische Aufnahme ist, wobei das synthetische Bild den Untersuchungsbereich des Untersuchungsobjekts nach Applikation einer dritten Menge des Kontrastmittels repräsentiert, wobei die zweite Menge verschieden von, vorzugweise größer ist als die erste Menge, und die dritte Menge verschieden von, vorzugsweise größer ist als die erste Menge und die zweite Menge.

41. Eine der Ausführungsformen 1 bis 40, wobei das trainierte Modell des maschinellen Lernens konfiguriert ist und trainiert wurde, bei einer Erhöhung der Abweichung des vorhergesagten Farbwerts des synthetischen Referenzbildes von dem Farbwert des korrespondierenden Bildelements des Ziel-Referenzbildes den Unsicherheitswert des vorhergesagten Farbwerts zu erhöhen, um die Fehlerfunktion zu minimieren.

42. Eine der Ausführungsformen 1 bis 41, wobei eine Erhöhung der Abweichung des vorhergesagten Farbwerts des synthetischen Referenzbildes von dem Farbwert des korrespondierenden Bildelements des Ziel-Referenzbildes zu einer Erhöhung eines mittels der Fehlerfunktion berechneten Fehlers führt, während eine Erhöhung des Unsicherheitswerts zu einer Erniedrigung des mittels der Fehlerfunktion berechneten Fehlers führt.

43. Eine der Ausführungsformen 1 bis 42, wobei die Fehlerfunktion folgende Gleichung (1) aufweist:

$$\mathcal{L} = \frac{1}{N}\sum_{i=1}^{N}\frac{1}{2\hat{\sigma}(x_i)^2}\|y_i - \hat{y}_i\|^2 + \frac{1}{2}\log\hat{\sigma}(x_i)^2 \qquad (1)$$

wobei N die Zahl der Bildelemente des synthetischen Referenzbildes ist, $\hat{y}_i$ der vorhergesagte Farbwert des Bildelements *i* des synthetischen Referenzbildes ist, $\hat{\sigma}(x_i)$ der Unsicherheitswert des vorhergesagten Farbwerts des Bildelements *i* ist und $y_i$ der Farbwert des korrespondierenden Bildelements des Ziel-Referenzbildes ist.

44. Eine der Ausführungsformen 1 bis 43, wobei der Unsicherheitswert des vorhergesagten Farbwerts eines jeden Bildelements des synthetischen Bildes oder ein davon abgeleiteter Wert als Vertrauenswert des Bildelements gesetzt wird.

45. Eine der Ausführungsformen 1 bis 44, wobei der Vertrauenswert ein Vertrauenswert für das gesamte synthetische Bild ist, wobei der Vertrauenswert ein Mittelwert oder ein Maximalwert oder ein Minimalwert ist, der auf Basis aller Unsicherheitswerte aller Bildelemente des synthetischen Bildes gebildet wird.

46. Eine der Ausführungsformen 1 bis 45, wobei für verschiedene Teilbereiche des Untersuchungsobjekts verschiedene Methoden zur Berechnung des Vertrauenswerts verwendet werden.

47. Eine der Ausführungsformen 1 bis 46, wobei der mindestens eine Vertrauenswert eine Vertrauensrepräsentation ist, in der bildhaft für jedes Bildelement des synthetischen Bildes eine Unsicherheit und/oder Vertrauenswürdigkeit des vorhergesagten Farbwerts dargestellt ist.

48. Eine der Ausführungsformen 1 bis 47, wobei die Vertrauensrepräsentation in einer überlagerten Darstellung zusammen mit dem synthetischen Bild ausgegeben wird.

49. Eine der Ausführungsformen 1 bis 48, wobei das Modell des maschinellen Lernens ein künstliches neuronales Netzwerk ist oder ein solches umfasst.

50. Eine der Ausführungsformen 1 bis 49,

wobei das mindestens eine Eingabe-Bild eine erste radiologische Aufnahme und eine zweite radiologische Aufnahme umfasst, wobei die erste radiologische Aufnahme den Untersuchungsbereich des Untersuchungsobjekts ohne Kontrastmittel oder nach Applikation einer ersten Menge eines Kontrastmittels repräsentiert und die zweite radiologische Aufnahme den Untersuchungsbereich des Untersuchungsobjekts nach Applikation einer zweiten Menge des Kontrastmittels repräsentiert,

wobei das synthetische Bild eine synthetische radiologische Aufnahme ist, wobei das synthetische Bild den Untersuchungsbereich des Untersuchungsobjekts nach Applikation einer dritten Menge des Kontrastmittels repräsentiert, wobei die zweite Menge verschieden von, vorzugweise größer ist als die erste Menge, und die dritte Menge verschieden von, vorzugsweise größer ist als die erste Menge und die zweite Menge.

51. Eine der Ausführungsformen 1 bis 50,

wobei das mindestens eine Eingabe-Bild eine erste radiologische Aufnahme und eine zweite radiologische Aufnahme umfasst, wobei die erste radiologische Aufnahme den Untersuchungsbereich des Untersuchungsobjekts in einer ersten Zeitspanne vor oder nach Applikation eines Kontrastmittels repräsentiert und die zweite radiologische Aufnahme den Untersuchungsbereich des Untersuchungsobjekts in einer zweiten Zeitspanne nach Applikation des Kontrastmittels repräsentiert,

wobei das synthetische Bild eine synthetische radiologische Aufnahme ist, wobei das synthetische Bild den Untersuchungsbereich des Untersuchungsobjekts in einer dritten Zeitspanne nach Applikation des Kontrastmittels repräsentiert, wobei die zweite Zeitspanne vorzugsweise zeitlich auf die erste Zeitspanne folgt und die dritte Zeitspanne vorzugsweise zeitlich auf die zweite Zeitspanne folgt.

52. Eine der Ausführungsformen 1 bis 51,
wobei das Trainieren des Modells des maschinellen Lernens umfasst:

(110) Empfangen der Trainingsdaten,

o wobei die Trainingsdaten für jedes Referenzobjekt der Vielzahl von Referenzobjekten (i) das mindestens eine Eingabe-Referenzbild des Referenzbereichs des Referenzobjekts in dem ersten Zustand und (ii) das Ziel-Referenzbild des Referenzbereichs des Referenzobjekts in dem zweiten Zustand umfassen,

o wobei sich der zweite Zustand von dem ersten Zustand unterscheidet,

o wobei das mindestens eine Eingabe-Referenzbild eine Vielzahl an Bildelementen umfasst, wobei jedes Bildelement des mindestens einen Eingabe-Referenzbildes einen Teilbereich des Referenzbereichs repräsentiert, wobei jedes Bildelement des mindestens einen Eingabe-Referenzbildes durch einen Farbwert gekennzeichnet ist,

o wobei das Ziel-Referenzbild eine Vielzahl an Bildelementen umfasst, wobei jedes Bildelement des Ziel-Referenzbildes einen Teilbereich des Referenzbereich repräsentiert, wobei jedes Bildelement des Ziel-Referenzbildes durch einen Farbwert gekennzeichnet ist,

(120) Bereitstellen des Modells des maschinellen Lernens,

o wobei das Modell des maschinellen Lernens konfiguriert ist, auf Basis des mindestens einen Eingabe-Referenzbildes des Referenzbereichs eines Referenzobjekts und Modellparametern ein synthetisches Referenzbild des Referenzbereichs des Referenzobjekts zu erzeugen, wobei das synthetische Referenzbild eine Vielzahl an Bildelementen umfasst, wobei jedes Bildelement des synthetischen Referenzbildes mit einem Bildelement des Ziel-Referenzbildes korrespondiert, wobei jedem Bildelement des synthetischen Referenzbildes ein vorhergesagter Farbwert zugeordnet ist, wobei das Modell des maschinellen Lernens konfiguriert ist für jeden vorhergesagten Farbwert einen Unsicherheitswert vorherzusagen,

(130) Trainieren des Modells des maschinellen Lernens, wobei das Trainieren für jedes Referenzobjekt der Vielzahl an Referenzobjekten umfasst:

(131) Eingeben des mindestens einen Eingabe-Referenzbildes in das Modell des maschinellen Lernens,

(132) Empfangen eines synthetischen Referenzbildes von dem Modell des maschinellen Lernens,

(133) Empfangen eines Unsicherheitswerts für jeden vorhergesagten Farbwert des synthetischen Referenzbildes,

(134) Berechnen eines Fehlers mittels einer Fehlerfunktion, wobei die Fehlerfunktion (i) den vorhergesagten Farbwert und/oder eine Abweichung zwischen dem vorhergesagten Farbwert und einem Farbwert des korrespondierenden Bildelements des Ziel-Referenzbildes und (ii) den vorhergesagten Unsicherheitswert als Parameter umfassen,

(135) Reduzieren des Fehlers durch Modifizieren von Modellparametern,

(140) Ausgeben und/oder Speichern des trainierten Modells des maschinellen Lernens und/oder Übermitteln des trainierten Modells des maschinellen Lernens an ein separates Computersystem und/oder Verwenden des trainierten Modells des maschinellen Lernens zur Vorhersage eines synthetischen Bildes und/oder zum Erzeugen mindestens eines Vertrauenswertes für ein synthetisches Bild.

53. Eine der Ausführungsformen 1 bis 52,
wobei es sich bei dem Kontrastmittel um ein hepatobiliäres Kontrastmittel handelt.

54. Eine der Ausführungsformen 1 bis 52,
wobei das Kontrastmittel eine oder mehrere der folgenden Verbindungen umfasst:

- Gadolinium(III) 2-[4,7,10-tris(carboxymethyl)-1,4,7,10-tetrazacyclododec-1 -yl]essigsäure
- Gadolinium(III) Ethoxybenzyl-diethylenetriaminepentaessigsäure
- Gadolinium(III) 2-[3,9-bis[1-carboxylato-4-(2,3-dihydroxypropylamino)-4-oxobutyl]-3,6,9,15-tetrazabicyclo[9.3.1]pentadeca-1(15),11,13-trien-6-yl]-5-(2,3-dihydroxypropylamino)-5-oxopentanoat
- Dihydrogen[(±)-4-carboxy-5,8,11-tris(carboxymethyl)-1-phenyl-2-oxa-5,8,11-triazatridecan-13-oato(5-)]gadolinat(2-)
- Tetragadolinium-[4,10-bis(carboxylatomethyl)-7-{3,6,12,15-tetraoxo-16-[4,7,10-tris-(carboxylatomethyl)-1,4,7,10-tetraazacyclododecan-1-yl]-9,9-bis({[({ 2-[4,7,10-tris-(carboxylatomethyl)-1,4,7,10-tetraazacyclododecan-1 -yl]propanoyl} amino)acetyl] - amino}methyl)-4,7,11,14-tetraazahepta-decan-2-yl}-1,4,7,10-tetraazacyclododecan-1-yl]acetat
- Gadolinium 2,2',2"-(10-{1-carboxy-2-[2-(4-ethoxyphenyl)ethoxy]ethyl}-1,4,7,10-tetraazacyclododecan-1,4,7-triyl)triacetat
- Gadolinium 2,2',2"-{10-[1-carboxy-2-{4-[2-(2-ethoxyethoxy)ethoxy]phenyl}ethyl]-1,4,7,10-tetraazacyclododecan-1,4,7-triyl}triacetat
- Gadolinium 2,2',2"-{10-[(1R)-1-carboxy-2-{4-[2-(2-ethoxyethoxy)ethoxy]phenyl}ethyl]-1,4,7,10-tetraazacyclododecan-1,4,7-triyl}triacetat
- Gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecan-1,4,7-triyl}tris(3-hydroxypropanoat)
- Gadolinium 2,2',2"-{10-[(1S)-4-(4-butoxyphenyl)-1-carboxybutyl]-1,4,7,10-tetraazacyclododecan-1,4,7-triyl}triacetat
- Gadolinium-2,2',2"-{(2*S*)-10-(carboxymethyl)-2-[4-(2-ethoxyethoxy)benzyl]-1,4,7,10-tetraazacyclododecane-1,4,7-triyl}triacetat
- Gadolinium-2,2',2"-[10-(carboxymethyl)-2-(4-ethoxybenzyl)-1,4,7,10-tetraazacyclododecane-1,4,7-triyl]triacetat
- Gadolinium(III) 5,8-bis(carboxylatomethyl)-2-[2-(methylamino)-2-oxoethyl]-10-oxo-2,5,8,11-tetraazadodecan-1-carboxylat-Hydrat
- Gadolinium(III) 2-[4-(2-hydroxypropyl)-7,10-bis(2-oxido-2-oxoethyl)-1,4,7,10-tetrazacyclododec-1-yl]acetat
- Gadolinium(III) 2,2',2"-(10-((2R,3S)-1,3,4-trihydroxybutan-2-yl)-1,4,7,10-tetraazacyclododecan-1,4,7-triyl)triacetat
- einen $Gd^{3+}$-Komplex einer Verbindung der Formel (I)

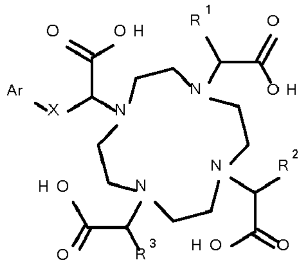

(I) ,

wobei

Ar eine Gruppe ausgewählt aus

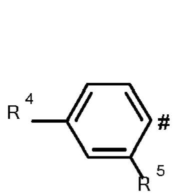

und

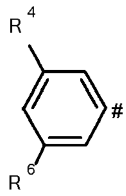

darstellt,

wobei # die Anknüpfung zu X darstellt,

X eine Gruppe darstellt, die aus $CH_2$, $(CH_2)_2$, $(CH_2)_3$, $(CH_2)_4$ und $*-(CH_2)_2-O-CH_2-^{\#}$ ausgewählt wird,

wobei * die Anknüpfung zu Ar darstellt und # die Anknüpfung zum Essigsäurerest darstellt,

$R^1$, $R^2$ and $R^3$ unabhängig voneinander ein Wasserstoffatom oder eine Gruppe ausgewählt aus $C_1$-$C_3$-Alkyl, $-CH_2OH$, $-(CH_2)_2OH$ und $-CH_2OCH_3$ darstellen,

$R^4$ eine Gruppe ausgewählt aus $C_2$-$C_4$-Alkoxy, $(H_3C-CH_2)-O-(CH_2)_2-O-$, $(H_3C-CH_2)-O-(CH_2)_2-O-(CH_2)_2-O-$ und $(H_3C-CH_2)-O-(CH_2)_2-O-(CH_2)_2-O-(CH_2)_2-O-$ darstellt,

$R^5$ ein Wasserstoffatom darstellt, und

$R^6$ ein Wasserstoffatom darstellt,

oder ein Stereoisomer, Tautomer, Hydrat, Solvat oder Salz davon, oder eine Mischung davon,

- einen $Gd^{3+}$-Komplex einer Verbindung der Formel (II)

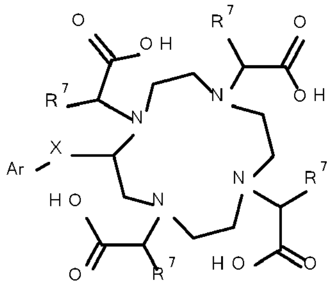

(II) ,

wobei

Ar eine Gruppe ausgewählt aus

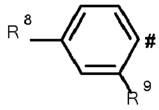

and

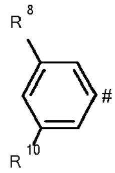

darstellt,

wobei # die Anknüpfung zu X darstellt,

X eine Gruppe darstellt, die aus $CH_2$, $(CH_2)_2$, $(CH_2)_3$, $(CH_2)_4$ and $*-(CH_2)_2-O-CH_2-^{\#}$ ausgewählt wird, wobei * die Anknüpfung zu Ar darstellt und $^{\#}$ die Anknüpfung zum Essigsäurerest darstellt,

$R^7$ ein Wasserstoffatom oder eine Gruppe ausgewählt aus $C_1$-$C_3$-Alkyl, $-CH_2OH$, $-(CH_2)_2OH$ und $-CH_2OCH_3$ darstellt;

$R^8$ eine Gruppe ausgewählt aus $C_2$-$C_4$-Alkoxy, $(H_3C-CH_2O)-(CH_2)_2-O-$, $(H_3C-CH_2O)-(CH_2)_2-O-(CH_2)_2-O-$ und $(H_3C-CH_2O)-(CH_2)_2-O-(CH_2)_2-O-(CH_2)_2-O-$ darstellt,

$R^9$ und $R^{10}$ unabhängig voneinander ein Wasserstoffatom darstellen;

oder ein Stereoisomer, Tautomer, Hydrat, Solvat oder Salz davon, oder eine Mischung davon.

Fig. 7 zeigt beispielhaft und schematisch ein Computersystem gemäß der vorliegenden Offenbarung.

Ein "Computersystem" ist ein System zur elektronischen Datenverarbeitung, das mittels programmierbarer Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Laufwerke, Kamera, Mikrofon, Lautsprecher etc. Auch interne Anschlüsse und Erweiterungskarten gelten in der Computertechnik als Peripherie.

Das in Fig. 7 gezeigte Computersystem (10) umfasst eine Empfangseinheit (11), eine Steuer- und Recheneinheit (12) und eine Ausgabeeinheit (13).

Die Steuer- und Recheneinheit (12) dient der Steuerung des Computersystems (10), der Koordinierung der Datenflüsse zwischen den Einheiten des Computersystems (10) und der Durchführung von Berechnungen.

Die Steuer- und Recheneinheit (12) ist konfiguriert:

- ein trainiertes Modell des maschinellen Lernens bereitzustellen,

  o wobei das trainierte Modell des maschinellen Lernens anhand von Trainingsdaten trainiert wurde,

  o wobei die Trainingsdaten für jedes Referenzobjekt einer Vielzahl von Referenzobjekten (i) mindestens ein Eingabe-Referenzbild eines Referenzbereichs des Referenzobjekts in einem ersten Zustand und (ii) ein Ziel-Referenzbild des Referenzbereichs des Referenzobjekts in einem zweiten Zustand umfassen, wobei das mindestens eine Eingabe-Referenzbild und das Ziel-Referenzbild jeweils eine Vielzahl an Bildelementen umfassen,

  o wobei das Modell des maschinellen Lernens konfiguriert ist und trainiert wurde, für jedes Referenzobjekt auf Basis des mindestens einen Eingabe-Referenzbildes ein synthetisches Referenzbild zu erzeugen,

    ▪ wobei das synthetische Referenzbild eine Vielzahl an Bildelementen umfasst, wobei jedes Bildelement des synthetischen Referenzbildes mit jeweils einem Bildelement des Ziel-Referenzbildes korrespondiert,

▪ wobei das Modell des maschinellen Lernens trainiert wurde, für jedes Bildelement des synthetischen Referenzbildes einen Farbwert und einen Unsicherheitswert für den vorhergesagten Farbwert vorherzusagen,

▪ wobei das Trainieren ein Minimieren einer Fehlerfunktion umfasst, wobei die Fehlerfunktion (i) den vorhergesagten Farbwert und/oder eine Abweichung des vorhergesagten Farbwerts von einem Farbwert des korrespondierenden Bildelements des Ziel-Referenzbildes und (ii) den vorhergesagten Unsicherheitswert als Parameter umfasst,

- die Empfangseinheit (11) zu veranlassen, mindestens ein Eingabe-Bild eines Untersuchungsbereichs eines Untersuchungsobjekts zu empfangen, wobei das mindestens eine Eingabe-Bild den Untersuchungsbereich des Untersuchungsobjekts in dem ersten Zustand repräsentiert,

- das mindestens eine erste Eingabe-Bild dem trainierten Modell des maschinellen Lernens zuzuführen,

- von dem trainierten Modell des maschinellen Lernens ein synthetisches Bildes zu empfangen, wobei das synthetische Bild den Untersuchungsbereich des Untersuchungsobjekts in dem zweiten Zustand repräsentiert,

- von dem trainierten Modell des maschinellen Lernens einen Unsicherheitswert für jedes Bildelement des synthetischen Bildes zu empfangen,

- mindestens einen Vertrauenswert auf Basis der empfangenen Unsicherheitswerte zu ermitteln,

- die Ausgabeeinheit (13) zu veranlassen, den mindestens einen Vertrauenswert auszugeben, in einem Datenspeicher zu speichern und/oder an ein separates Computersystem zu übermitteln.

Fig. 8 zeigt beispielhaft und schematisch eine weitere Ausführungsform des Computersystems. Das Computersystem (10) umfasst eine Verarbeitungseinheit (21), die mit einem Speicher (22) verbunden ist. Die Verarbeitungseinheit (21) und der Speicher (22) bilden eine Steuer- und Recheneinheit, wie sie in Fig. 7 gezeigt ist.

Die Verarbeitungseinheit (21) (engl.: *processing unit)* kann einen oder mehrere Prozessoren allein oder in Kombination mit einem oder mehreren Speichern umfassen. Bei der Verarbeitungseinheit (21) kann es sich um gewöhnliche Computerhardware handeln, die in der Lage ist, Informationen wie z.B. digitale Bildaufnahmen, Computerprogramme und/oder andere digitale Informationen zu verarbeiten. Die Verarbeitungseinheit (21) besteht üblicherweise aus einer Anordnung elektronischer Schaltungen, von denen einige als integrierter Schaltkreis oder als mehrere miteinander verbundene integrierte Schaltkreise (ein integrierter Schaltkreis wird manchmal auch als "Chip" bezeichnet) ausgeführt sein können. Die Verarbeitungseinheit (21) kann konfiguriert sein, Computerprogramme auszuführen, die in einem Arbeitsspeicher der Verarbeitungseinheit (21) oder im Speicher (22) desselben oder eines anderen Computersystems gespeichert sein können.

Der Speicher (22) kann eine gewöhnliche Computerhardware sein, die in der Lage ist, Informationen wie z.B. digitale Bildaufnahmen (z.B. Repräsentationen des Untersuchungsbereichs), Daten, Computerprogramme und/oder andere digitale Informationen entweder vorübergehend und/oder dauerhaft zu speichern. Der Speicher (22) kann einen flüchtigen und/oder nichtflüchtigen Speicher umfassen und kann fest eingebaut oder entfernbar sein. Beispiele für geeignete Speicher sind RAM (Random Access Memory), ROM (Read-Only Memory), eine Festplatte, ein Flash-Speicher, eine austauschbare Computerdiskette, eine optische Disc, ein Magnetband oder eine Kombination der oben genannten. Zu den optischen Discs können Compact Discs mit Nur-Lese-Speicher (CD-ROM), Compact Discs mit Lese-/Schreibfunktion (CD-R/W), DVDs, Blu-ray-Discs und ähnliche gehören.

Zusätzlich zum Speicher (22) kann die Verarbeitungseinheit (21) auch mit einer oder mehreren Schnittstellen (11, 12, 31, 32, 33) verbunden sein, um Informationen anzuzeigen, zu übertragen und/oder zu empfangen. Die Schnittstellen können eine oder mehrere Kommunikationsschnittstellen (11, 32, 33) und/oder eine oder mehrere Benutzerschnittstellen (12, 31) umfassen. Die eine oder mehrere Kommunikationsschnittstellen können so konfiguriert sein, dass sie Informationen senden und/oder empfangen, z.B. zu und/oder von einer MRT-Scanner, einem CT-Scanner, einer Ultraschallkamera, anderen Computersystemen, Netzwerken, Datenspeichern oder dergleichen. Die eine oder mehrere Kommunikationsschnittstellen können so konfiguriert sein, dass sie Informationen über physische (verdrahtete) und/oder drahtlose Kommunikationsverbindungen übertragen und/oder empfangen. Die eine oder die mehreren Kommunikationsschnittstellen können eine oder mehrere Schnittstellen für die Verbindung mit einem Netzwerk enthalten, z.B. unter Verwendung von Technologien wie Mobiltelefon, Wi-Fi, Satellit, Kabel, DSL, Glasfaser und/oder dergleichen. In einigen Beispielen können die eine oder die mehreren Kommunikationsschnittstellen eine oder mehrere Nahbereichskommunikationsschnittstellen umfassen, die so konfiguriert sind, dass sie Geräte mit Nahbereichskommunikationstechnologien

wie NFC, RFID, Bluetooth, Bluetooth LE, ZigBee, Infrarot (z. B. IrDA) oder Ähnlichem verbinden.

Die Benutzerschnittstellen können eine Anzeige (31) umfassen. Eine Anzeige (31) kann so konfiguriert sein, dass sie einem Benutzer Informationen anzeigt. Geeignete Beispiele hierfür sind eine Flüssigkristallanzeige (LCD), eine Leuchtdiodenanzeige (LED), ein Plasmabildschirm (PDP) oder Ähnliches. Die Benutzereingabeschnittstelle(n) (11, 12) kann/können verdrahtet oder drahtlos sein und kann/können so konfiguriert sein, dass sie Informationen von einem Benutzer in das Computersystem (1) empfängt/empfangen, z.B. zur Verarbeitung, Speicherung und/oder Anzeige. Geeignete Beispiele für Benutzereingabeschnittstellen sind ein Mikrofon, ein Bild- oder Videoaufnahmegerät (z.B. eine Kamera), eine Tastatur oder ein Tastenfeld, ein Joystick, eine berührungsempfindliche Oberfläche (getrennt von einem Touchscreen oder darin integriert) oder ähnliches. In einigen Beispielen können die Benutzerschnittstellen eine automatische Identifikations- und Datenerfassungstechnologie (AIDC) für maschinenlesbare Informationen enthalten. Dazu können Barcodes, Radiofrequenz-Identifikation (RFID), Magnetstreifen, optische Zeichenerkennung (OCR), Karten mit integrierten Schaltkreisen (ICC) und ähnliches gehören. Die Benutzerschnittstellen können ferner eine oder mehrere Schnittstellen für die Kommunikation mit Peripheriegeräten wie Druckern und dergleichen umfassen.

Ein oder mehrere Computerprogramme (40) können im Speicher (22) gespeichert sein und von der Verarbeitungseinheit (21) ausgeführt werden, die dadurch programmiert wird, die in dieser Beschreibung beschriebenen Funktionen zu erfüllen. Das Abrufen, Laden und Ausführen von Anweisungen des Computerprogramms (40) kann sequenziell erfolgen, so dass jeweils ein Befehl abgerufen, geladen und ausgeführt wird. Das Abrufen, Laden und/oder Ausführen kann aber auch parallel erfolgen.

Das Computersystem der vorliegenden Offenbarung kann als Laptop, Notebook, Netbook und/der Tablet-PC ausgeführt sein, es kann auch ein Bestandteil eines MRT-Scanners, eines CT-Scanners oder eines Ultraschalldiagnosegeräts sein.

Gegenstand der vorliegenden Erfindung ist auch ein Computerprogrammprodukt. Ein solches Computerprogrammprodukt umfasst einen nichtflüchtigen Datenträger wie beispielsweise eine CD, eine DVD, ein USB-Stick oder ein anderes Medium zum Speichern von Daten. Auf dem Datenträger ist ein Computerprogramm gespeichert. Das Computerprogramm kann in einen Arbeitsspeicher eines Computersystems (insbesondere in einen Arbeitsspeicher eines Computersystems der vorliegenden Offenbarung) geladen werden und dort das Computersystem dazu veranlassen, folgende Schritte ausführen:

- Bereitstellen eines trainierten Modells des maschinellen Lernens,

    o wobei das trainierte Modell des maschinellen Lernens anhand von Trainingsdaten trainiert wurde,

    o wobei die Trainingsdaten für jedes Referenzobjekt einer Vielzahl von Referenzobjekten (i) mindestens ein Eingabe-Referenzbild eines Referenzbereichs des Referenzobjekts in einem ersten Zustand und (ii) ein Ziel-Referenzbild des Referenzbereichs des Referenzobjekts in einem zweiten Zustand umfassen, wobei das mindestens eine Eingabe-Referenzbild und das Ziel-Referenzbild jeweils eine Vielzahl an Bildelementen umfassen,

    o wobei das Modell des maschinellen Lernens konfiguriert ist und trainiert wurde, für jedes Referenzobjekt auf Basis des mindestens einen Eingabe-Referenzbildes ein synthetisches Referenzbild zu erzeugen,

        ▪ wobei das synthetische Referenzbild eine Vielzahl an Bildelementen umfasst, wobei jedes Bildelement des synthetischen Referenzbildes mit jeweils einem Bildelement des Ziel-Referenzbildes korrespondiert,

        ▪ wobei das Modell des maschinellen Lernens trainiert wurde, für jedes Bildelement des synthetischen Referenzbildes einen Farbwert und einen Unsicherheitswert für den vorhergesagten Farbwert vorherzusagen,

        ▪ wobei das Trainieren ein Minimieren einer Fehlerfunktion umfasst, wobei die Fehlerfunktion (i) den vorhergesagten Farbwert und/oder eine Abweichung des vorhergesagten Farbwerts von einem Farbwert des korrespondierenden Bildelements des Ziel-Referenzbildes und (ii) den vorhergesagten Unsicherheitswert als Parameter umfasst,

- Empfangen mindestens eines Eingabe-Bildes eines Untersuchungsbereichs eines Untersuchungsobjekts, wobei das mindestens eine Eingabe-Bild den Untersuchungsbereich des Untersuchungsobjekts in dem ersten Zustand repräsentiert,

- Zuführen des mindestens einen Eingabe-Bildes dem trainierten Modell des maschinellen Lernens,

- Empfangen eines synthetischen Bildes von dem trainierten Modell des maschinellen Lernens, wobei das synthetische Bild den Untersuchungsbereich des Untersuchungsobjekts in dem zweiten Zustand repräsentiert,
- Empfangen eines Unsicherheitswertes für jedes Bildelement des synthetischen Bildes,
- Ermitteln mindestens eines Vertrauenswertes auf Basis der empfangenen Unsicherheitswerte,
- Ausgeben des mindestens einen Vertrauenswertes.

Das Computerprogrammprodukt kann auch in Kombination (in einer Zusammenstellung) mit dem Kontrastmittel vermarktet werden. Eine solche Zusammenstellung wird auch als Kit bezeichnet. Ein solches Kit umfasst das Kontrastmittel und das Computerprogrammprodukt. Es ist auch möglich, dass ein solches Kit das Kontrastmittel und Mittel umfasst, die es einem Käufer erlauben, das Computerprogramm zu beziehen, z.B. von einer Internetseite herunterzuladen. Diese Mittel können einen Link, d.h. eine Adresse der Internetseite umfassen, auf der das Computerprogramm bezogen, z.B. von der das Computerprogramm auf ein mit dem Internet verbundenes Computersystem heruntergeladen werden kann. Diese Mittel können einen Code (z.B. eine alphanumerische Zeichenkette oder einen QR-Code, oder einen DataMatrix-Code oder einen Barcode oder eine anderen optisch und/oder elektronisch lesbaren Code) umfassen, mit dem der Käufer Zugang zu dem Computerprogramm erhält. Ein solcher Link und/oder Code kann beispielsweise auf einer Verpackung des Kontrastmittels aufgedruckt und/oder auf einem Beipackzettel zum Kontrastmittel aufgedruckt sein. Ein Kit ist somit ein Kombinationsprodukt umfassend ein Kontrastmittel und ein Computerprogramm (z.B. in Form eines Zugangs zu dem Computerprogramm oder in Form von ausführbarem Programmcode auf einem Datenträger), die zusammen zum Kauf angeboten werden.

## Patentansprüche

**1.** Computer-implementiertes Verfahren umfassend:

- Bereitstellen eines trainierten Modells ($MLM^t$) des maschinellen Lernens,

o wobei das trainierte Modell ($MLM^t$) des maschinellen Lernens anhand von Trainingsdaten (TD) trainiert wurde,
o wobei die Trainingsdaten (TD) für jedes Referenzobjekt einer Vielzahl von Referenzobjekten (i) mindestens ein Eingabe-Referenzbild ($RI_1(x_i)$) eines Referenzbereichs des Referenzobjekts in einem ersten Zustand und (ii) ein Ziel-Referenzbild ($RI_2(y_i)$) des Referenzbereichs des Referenzobjekts in einem zweiten Zustand umfassen, wobei das mindestens eine Eingabe-Referenzbild ($RI_1(x_i)$) und das Ziel-Referenzbild ($RI_2(y_i)$) jeweils eine Vielzahl an Bildelementen umfassen, wobei das mindestens eine Eingabe-Referenzbild ($RI_1(x_i)$) mindestens eine computertomographische und/oder magnetresonanztomographische Aufnahme des Referenzbereichs des Referenzobjekts umfasst, wobei das Ziel-Referenzbild ($RI_2(y_i)$) eine computertomographische und/oder magnetresonanztomographische Aufnahme des Referenzbereichs des Referenzobjekts ist,
o wobei das Modell ($MLM^t$) des maschinellen Lernens konfiguriert ist und trainiert wurde, für jedes Referenzobjekt auf Basis des mindestens einen Eingabe-Referenzbildes ($RI_1(x_i)$) ein synthetisches Referenzbild ($RI_2^*(\hat{y}_i)$) zu erzeugen,

▪ wobei das synthetische Referenzbild ($RI_2^*(\hat{y}_i)$) eine Vielzahl an Bildelementen umfasst, wobei jedes Bildelement des synthetischen Referenzbildes ($RI_2^*(\hat{y}_i)$) mit jeweils einem Bildelement des Ziel-Referenzbildes ($RI_2(y_i)$) korrespondiert,
▪ wobei das Modell ($MLM^t$) des maschinellen Lernens trainiert wurde, für jedes Bildelement des synthetischen Referenzbildes ($RI_2^*(\hat{y}_i)$) einen Farbwert ($\hat{y}_i$) und einen Unsicherheitswert ($\hat{\sigma}(x_i)$) für den vorhergesagten Farbwert ($\hat{y}_i$) vorherzusagen,
▪ wobei das Trainieren ein Minimieren einer Fehlerfunktion ($\mathcal{L}$) umfasst, wobei die Fehlerfunktion ($\mathcal{L}$) (i) den vorhergesagten Farbwert ($\hat{y}_i$) und/oder eine Abweichung des vorhergesagten Farbwerts ($\hat{y}_i$) von einem Farbwert ($\hat{y}_i$) des korrespondierenden Bildelements des Ziel-Referenzbildes ($RI_2(y_i)$) und (ii) den vorhergesagten Unsicherheitswert ($\hat{\sigma}(x_i)$) als Parameter umfasst,

- Empfangen mindestens eines Eingabe-Bildes ($I_1(x_i)$) eines Untersuchungsbereichs eines Untersuchungsobjekts, wobei das mindestens eine Eingabe-Bild ($I_1(x_i)$) den Untersuchungsbereich des Untersuchungsobjekts

in dem ersten Zustand repräsentiert, wobei das mindestens eine Eingabe-Bild ($I_1(x_i)$) mindestens eine computertomographische und/oder magnetresonanztomographische Aufnahme des Untersuchungsbereichs des Untersuchungsobjekts umfasst,
- Zuführen des mindestens einen Eingabe-Bildes ($I_1(x_i)$) dem trainierten Modell ($MLM^t$) des maschinellen Lernens,
- Empfangen eines synthetischen Bildes ($I_2^*(\hat{y}_i)$) von dem trainierten Modell des maschinellen Lernens, wobei das synthetische Bild ($I_2^*(\hat{y}_i)$) den Untersuchungsbereich des Untersuchungsobjekts in dem zweiten Zustand repräsentiert,
- Empfangen eines Unsicherheitswertes ($\hat{\sigma}(x_i)$) für jedes Bildelement des synthetischen Bildes ($I_2^*(\hat{y}_i)$,
- Ermitteln mindestens eines Vertrauenswertes auf Basis der empfangenen Unsicherheitswerte,
- Ausgeben des mindestens einen Vertrauenswertes.

**2.** Verfahren gemäß Anspruch 1, wobei der erste Zustand ein Zustand vor oder nach Applikation eines Kontrastmittels ist und der zweite Zustand ein Zustand nach Applikation des Kontrastmittels ist.

**3.** Verfahren gemäß Anspruch einem der Ansprüche 1 oder 2, wobei der erste Zustand und der zweite Zustand jeweils eine Menge eines Kontrastmittels angeben, das dem Referenzobjekt und dem Untersuchungsobjekt verabreicht worden ist.

**4.** Verfahren gemäß einem der Ansprüche 1 bis 3,

wobei das mindestens eine Eingabe-Bild ($I_1(x_i)$) eine erste computertomographische oder magnetresonanztomographische Aufnahme und eine zweite computertomographische oder magnetresonanztomographische Aufnahme umfasst, wobei die erste computertomographische oder magnetresonanztomographische Aufnahme den Untersuchungsbereich des Untersuchungsobjekts ohne Kontrastmittel oder nach Applikation einer ersten Menge eines Kontrastmittels repräsentiert und die zweite computertomographische oder magnetresonanztomographische Aufnahme den Untersuchungsbereich des Untersuchungsobjekts nach Applikation einer zweiten Menge des Kontrastmittels repräsentiert,
wobei das synthetische Bild ($I_2^*(\hat{y}_i)$) eine synthetische computertomographische oder magnetresonanztomographische Aufnahme ist, wobei das synthetische Bild ($I_2^*(\hat{y}_i)$) den Untersuchungsbereich des Untersuchungsobjekts nach Applikation einer dritten Menge des Kontrastmittels repräsentiert, wobei die zweite Menge verschieden von, vorzugsweise größer ist als die erste Menge, und die dritte Menge verschieden von, vorzugsweise größer ist als die erste Menge und die zweite Menge.

**5.** Verfahren gemäß einem der Ansprüche 1 bis 4,

wobei das mindestens eine Eingabe-Bild ($I_1(x_i)$) eine erste computertomographische oder magnetresonanztomographische und eine zweite computertomographische oder magnetresonanztomographische Aufnahme umfasst, wobei die erste computertomographische oder magnetresonanztomographische Aufnahme den Untersuchungsbereich des Untersuchungsobjekts in einer ersten Zeitspanne vor oder nach Applikation eines Kontrastmittels repräsentiert und die zweite computertomographische oder magnetresonanztomographische Aufnahme den Untersuchungsbereich des Untersuchungsobjekts in einer zweiten Zeitspanne nach Applikation des Kontrastmittels repräsentiert,
wobei das synthetische Bild ($I_2^*(\hat{y}_i)$) eine synthetische computertomographische oder magnetresonanztomographische Aufnahme ist, wobei das synthetische Bild ($I_2^*(\hat{y}_i)$) den Untersuchungsbereich des Untersuchungsobjekts in einer dritten Zeitspanne nach Applikation des Kontrastmittels repräsentiert, wobei die zweite Zeitspanne vorzugsweise zeitlich auf die erste Zeitspanne folgt und die dritte Zeitspanne vorzugsweise zeitlich auf die zweite Zeitspanne folgt.

**6.** Verfahren gemäß einem der Ansprüche 1 bis 5, wobei der Unsicherheitswert ($\hat{\sigma}(x_i)$) des vorhergesagten Farbwerts ($\hat{y}_i$) eines jeden Bildelements des synthetischen Bildes ($I_2^*(\hat{y}_i)$) oder ein davon abgeleiteter Wert als Vertrauenswert des Bildelements gesetzt wird.

**7.** Verfahren gemäß einem der Ansprüche 1 bis 6, ferner umfassend:

- Erzeugen einer Vertrauensrepräsentation, wobei die Vertrauensrepräsentation eine Vielzahl an Bildelementen umfasst, wobei jedes Bildelement der Vielzahl an Bildelementen einen Teilbereich des Untersuchungsbereichs repräsentiert, wobei jedes Bildelement der Vielzahl an Bildelementen mit jeweils einem Bildelement des

synthetischen Bildes ($I_2^*(\hat{y}_i)$) korrespondiert, wobei jedes Bildelement der Vielzahl an Bildelementen einen Farbwert aufweist, wobei der Farbwert mit dem jeweiligen Unsicherheitswert ($\hat{\sigma}(x_i)$) des vorhergesagten Farbwerts ($\hat{y}_i$) des korrespondierenden Bildelements des synthetischen Bildes ($I_2^*(\hat{y}_i)$) korreliert,
- Ausgeben der Vertrauensrepräsentation in einer überlagerten Darstellung mit dem synthetischen Bild ($I_2^*(\hat{y}_i)$).

**8.** Verfahren gemäß einem der Ansprüche 1 bis 6, wobei der mindestens eine Vertrauenswert ein Vertrauenswert für das gesamte synthetische Bild ($I_2^*(\hat{y}_i)$) ist, wobei der Vertrauenswert ein Mittelwert oder ein Maximalwert oder ein Minimalwert ist, der auf Basis aller Unsicherheitswerte ($\hat{\sigma}(x_i)$) aller Bildelemente des synthetischen Bildes ($I_2^*(\hat{y}_i)$) gebildet wird.

**9.** Verfahren gemäß einem der Ansprüche 1 bis 6, ferner umfassend:

- Ermitteln eines Vertrauenswertes für einen oder mehrere Teilbereiche des synthetischen Bildes ($I_2^*(\hat{y}_i)$),
- Ausgeben des Vertrauenswertes für den einen oder die mehreren Teilbereiche des synthetischen Bildes ($I_2^*(\hat{y}_i)$).

**10.** Verfahren gemäß einem Anspruch 8, wobei für verschiedene Teilbereiche des Untersuchungsbereichs des Untersuchungsobjekts verschiedene Methoden zur Berechnung des Vertrauenswerts verwendet werden.

**11.** Verfahren gemäß einem der Ansprüche 1 bis 10, wobei das trainierte Modell ($MLM^t$) des maschinellen Lernens konfiguriert ist und trainiert wurde, bei einer Erhöhung der Abweichung des vorhergesagten Farbwerts ($\hat{y}_i$) des synthetischen Referenzbildes ($RI_2^*(\hat{y}_i)$) von dem Farbwert ($y_i$) des korrespondierenden Bildelements des Ziel-Referenzbildes ($RI_2(y_i)$) den Unsicherheitswert ($\hat{\sigma}(x_i)$) des vorhergesagten Farbwerts ($\hat{y}_i$) zu erhöhen, um die Fehlerfunktion zu minimieren.

**12.** Verfahren gemäß einem der Ansprüche 1 bis 11, wobei eine Erhöhung der Abweichung des vorhergesagten Farbwerts ($\hat{y}_i$) des synthetischen Referenzbildes ($RI_2^*(\hat{y}_i)$) von dem Farbwert ($y_i$) des korrespondierenden Bildelements des Ziel-Referenzbildes ($RI_2(y_i)$) zu einer Erhöhung eines mittels der Fehlerfunktion ( $\mathcal{L}$ ) berechneten Fehlers führt, während eine Erhöhung des Unsicherheitswerts ($\hat{\sigma}(x_i)$) zu einer Erniedrigung des mittels der Fehlerfunktion ( $\mathcal{L}$ ) berechneten Fehlers führt.

**13.** Verfahren gemäß einem der Ansprüche 1 bis 12, wobei die Fehlerfunktion ( $\mathcal{L}$ ) folgende Gleichung (1) aufweist:

$$\mathcal{L} = \frac{1}{N}\sum_{i=1}^{N}\frac{1}{2\hat{\sigma}(x_i)^2}\|y_i - \hat{y}_i\|^2 + \frac{1}{2}\log\hat{\sigma}(x_i)^2 \qquad (1)$$

wobei $N$ die Zahl der Bildelemente des synthetischen Referenzbildes ($RI_2^*(\hat{y}_i)$) ist, $\hat{y}_i$ der vorhergesagte Farbwert des Bildelements $i$ des synthetischen Referenzbildes ($RI_2^*(\hat{y}_i)$) ist, $\hat{\sigma}(x_i)$ der Unsicherheitswert des vorhergesagten Farbwerts des Bildelements $i$ ist und $y_i$ der Farbwert des korrespondierenden Bildelements des Ziel-Referenzbildes ($RI_2(y_i)$) ist.

**14.** Verfahren gemäß einem der Ansprüche 1 bis 13, wobei das Trainieren des Modells (MLM) des maschinellen Lernens umfasst:

(110) Empfangen der Trainingsdaten (TD),

o wobei die Trainingsdaten (TD) für jedes Referenzobjekt der Vielzahl von Referenzobjekten (i) das mindestens eine Eingabe-Referenzbild ($RI_1(x_i)$) des Referenzbereichs des Referenzobjekts in dem ersten Zustand und (ii) das Ziel-Referenzbild ($RI_2(y_i)$) des Referenzbereichs des Referenzobjekts in dem zweiten Zustand umfassen,
o wobei sich der zweite Zustand von dem ersten Zustand unterscheidet,
o wobei das mindestens eine Eingabe-Referenzbild ($RI_1(x_i)$) eine Vielzahl an Bildelementen umfasst, wobei jedes Bildelement des mindestens einen Eingabe-Referenzbildes ($RI_1(x_i)$) einen Teilbereich des Referenzbereichs repräsentiert, wobei jedes Bildelement des mindestens einen Eingabe-Referenzbildes ($RI_1(x_i)$) durch einen Farbwert ($x_i$) gekennzeichnet ist,

o wobei das Ziel-Referenzbild ($RI_2(y_i)$) eine Vielzahl an Bildelementen umfasst, wobei jedes Bildelement des Ziel-Referenzbildes ($RI_2(y_i)$) einen Teilbereich des Referenzbereich repräsentiert, wobei jedes Bildelement des Ziel-Referenzbildes ($RI_2(y_i)$) durch einen Farbwert ($y_i$) gekennzeichnet ist,

(120) Bereitstellen des Modells (MLM) des maschinellen Lernens,

o wobei das Modell (MLM) des maschinellen Lernens konfiguriert ist, auf Basis des mindestens einen Eingabe-Referenzbildes ($RI_1(x_i)$) des Referenzbereichs eines Referenzobjekts und Modellparametern (MP) ein synthetisches Referenzbild ($RI_2^*(\hat{y}_i)$) des Referenzbereichs des Referenzobjekts zu erzeugen, wobei das synthetische Referenzbild ($RI_2^*(\hat{y}_i)$) eine Vielzahl an Bildelementen umfasst, wobei jedes Bildelement des synthetischen Referenzbildes ($RI_2^*(\hat{y}_i)$) mit einem Bildelement des Ziel-Referenzbildes ($RI_2(y_i)$) korrespondiert, wobei jedem Bildelement des synthetischen Referenzbildes ($RI_2^*(\hat{y}_i)$) ein vorhergesagter Farbwert ($\hat{y}_i$) zugeordnet ist, wobei das Modell (MLM) des maschinellen Lernens konfiguriert ist für jeden vorhergesagten Farbwert ($\hat{y}_i$) einen Unsicherheitswert ($\hat{\sigma}(x_i)$) vorherzusagen,

(130) Trainieren des Modells (MLM) des maschinellen Lernens, wobei das Trainieren für jedes Referenzobjekt der Vielzahl an Referenzobjekten umfasst:

(131) Eingeben des mindestens einen Eingabe-Referenzbildes ($RI_1(x_i)$) in das Modell (MLM) des maschinellen Lernens,
(132) Empfangen eines synthetischen Referenzbildes ($RI_2^*(\hat{y}_i)$) von dem Modell (MLM) des maschinellen Lernens,
(133) Empfangen eines Unsicherheitswerts ($\hat{\sigma}(x_i)$) für jeden vorhergesagten Farbwert ($\hat{y}_i$) des synthetischen Referenzbildes ($RI_2^*(\hat{y}_i)$),
(134) Berechnen eines Fehlers mittels einer Fehlerfunktion ( $\mathcal{L}$ ), wobei die Fehlerfunktion ( $\mathcal{L}$ ) (i) den vorhergesagten Farbwert ($\hat{y}_i$) und/oder eine Abweichung zwischen dem vorhergesagten Farbwert ($\hat{y}_i$) und einem Farbwert ($y_i$) des korrespondierenden Bildelements des Ziel-Referenzbildes ($RI_2(y_i)$) und (ii) den vorhergesagten Unsicherheitswert ($\hat{\sigma}(x_i)$) als Parameter umfassen,
(135) Reduzieren des Fehlers durch Modifizieren von Modellparametern (MP),

(140) Ausgeben und/oder Speichern des trainierten Modells ($MLM^t$) des maschinellen Lernens und/oder Übermitteln des trainierten Modells ($MLM^t$) des maschinellen Lernens an ein separates Computersystem und/oder Verwenden des trainierten Modells ($MLM^t$) des maschinellen Lernens zur Vorhersage eines synthetischen Bildes und/oder zum Erzeugen mindestens eines Vertrauenswertes für ein synthetisches Bild.

**15.** Computersystem (10) umfassend

- eine Empfangseinheit (11),
- eine Steuer- und Recheneinheit (12) und
- eine Ausgabeeinheit (13),

wobei die Steuer- und Recheneinheit (12) konfiguriert ist,
- ein trainiertes Modell ($MLM^t$) des maschinellen Lernens bereitzustellen,

o wobei das trainierte Modell ($MLM^t$) des maschinellen Lernens anhand von Trainingsdaten (TD) trainiert wurde,
o wobei die Trainingsdaten (TD) für jedes Referenzobjekt einer Vielzahl von Referenzobjekten (i) mindestens ein Eingabe-Referenzbild ($RI_1(x_i)$) eines Referenzbereichs des Referenzobjekts in einem ersten Zustand und (ii) ein Ziel-Referenzbild ($RI_2(y_i)$) des Referenzbereichs des Referenzobjekts in einem zweiten Zustand umfassen, wobei das mindestens eine Eingabe-Referenzbild ($RI_1(x_i)$) und das Ziel-Referenzbild ($RI_2(y_i)$) jeweils eine Vielzahl an Bildelementen umfassen, wobei das mindestens eine Eingabe-Referenzbild ($RI_1(x_i)$) mindestens eine computertomographische und/oder magnetresonanztomographische Aufnahme des Referenzbereichs des Referenzobjekts umfasst, wobei das Ziel-Referenzbild ($RI_2(y_i)$) eine computertomographische und/oder magnetresonanztomographische Aufnahme des Referenzbereichs des Referenzobjekts ist,
o wobei das Modell ($MLM^t$) des maschinellen Lernens konfiguriert ist und trainiert wurde, für jedes Referenzobjekt auf Basis des mindestens einen Eingabe-Referenzbildes ($RI_1(x_i)$) ein synthetisches Referenzbild ($RI_2^*(\hat{y}_i)$) zu erzeugen,

▪ wobei das synthetische Referenzbild ($RI_2^*(\hat{y}_i)$) eine Vielzahl an Bildelementen umfasst, wobei jedes Bildelement des synthetischen Referenzbildes ($RI_2^*(\hat{y}_i)$) mit jeweils einem Bildelement des Ziel-Referenzbildes ($RI_2(y_i)$) korrespondiert,
▪ wobei das Modell ($MLM^t$) des maschinellen Lernens trainiert wurde, für jedes Bildelement des synthetischen Referenzbildes ($RI_2^*(\hat{y}_i)$) einen Farbwert ($\hat{y}_i$) und einen Unsicherheitswert ($\hat{\sigma}(x_i)$) für den vorhergesagten Farbwert ($\hat{y}_i$) vorherzusagen,

▪ wobei das Trainieren ein Minimieren einer Fehlerfunktion ($\mathcal{L}$) umfasst, wobei die Fehlerfunktion ($\mathcal{L}$) (i) den vorhergesagten Farbwert ($\hat{y}_i$) und/oder eine Abweichung des vorhergesagten Farbwerts ($\hat{y}_i$) von einem Farbwert ($y_i$) des korrespondierenden Bildelements des Ziel-Referenzbildes ($RI_2(y_i)$) und (ii) den vorhergesagten Unsicherheitswert ($\hat{\sigma}(x_i)$) als Parameter umfasst,

- die Empfangseinheit (11) zu veranlassen, mindestens ein Eingabe-Bild ($I_1(x_i)$) eines Untersuchungsbereichs eines Untersuchungsobjekts zu empfangen, wobei das mindestens eine Eingabe-Bild ($I_1(x_i)$) einen Untersuchungsbereich eines Untersuchungsobjekts in dem ersten Zustand repräsentiert, wobei das mindestens eine Eingabe-Bild ($I_1(x_i)$) mindestens eine computertomographische und/oder magnetresonanztomographische Aufnahme des Untersuchungsbereichs des Untersuchungsobjekts umfasst,
- das mindestens eine Eingabe-Bild ($I_1(x_i)$) einem trainierten Modell ($MLM^t$) des maschinellen Lernens zuzuführen,
- von dem trainierten Modell ($MLM^t$) des maschinellen Lernens ein synthetisches Bild ($I_2^*(\hat{y}_i)$) zu empfangen, wobei das synthetische Bild ($I_2^*(\hat{y}_i)$) den Untersuchungsbereich des Untersuchungsobjekts in dem zweiten Zustand repräsentiert,
- von dem trainierten Modell ($MLM^t$) des maschinellen Lernens einen Unsicherheitswert ($\hat{\sigma}(x_i)$) für jedes Bildelement des synthetischen Bildes ($I_2^*(\hat{y}_i)$) zu empfangen,
- mindestens einen Vertrauenswert auf Basis der empfangenen Unsicherheitswerte zu ermitteln,

die Ausgabeeinheit (13) zu veranlassen, den mindestens einen Vertrauenswert auszugeben, in einem Datenspeicher zu speichern und/oder an ein separates Computersystem zu übermitteln.

**16.** Computerlesbares Speichermedium umfassend ein Computerprogramm, das, wenn es in einen Arbeitsspeicher eines Computersystem geladen wird, das Computersystem veranlasst, folgende Schritte auszuführen:

- Bereitstellen eines trainierten Modells ($MLM^t$) des maschinellen Lernens,

o wobei das trainierte Modell ($MLM^t$) des maschinellen Lernens anhand von Trainingsdaten (TD) trainiert wurde,
o wobei die Trainingsdaten (TD) für jedes Referenzobjekt einer Vielzahl von Referenzobjekten (i) mindestens ein Eingabe-Referenzbild ($RI_1(x_i)$) eines Referenzbereichs des Referenzobjekts in einem ersten Zustand und (ii) ein Ziel-Referenzbild ($RI_2(y_i)$) des Referenzbereichs des Referenzobjekts in einem zweiten Zustand umfassen, wobei das mindestens eine Eingabe-Referenzbild ($RI_1(x_i)$) und das Ziel-Referenzbild ($RI_2(y_i)$) jeweils eine Vielzahl an Bildelementen umfassen, wobei das mindestens eine Eingabe-Referenzbild ($RI_1(x_i)$) mindestens eine computertomographische und/oder magnetresonanztomographische Aufnahme des Referenzbereichs des Referenzobjekts umfasst, wobei das Ziel-Referenzbild ($RI_2(y_i)$) eine computertomographische und/oder magnetresonanztomographische Aufnahme des Referenzbereichs des Referenzobjekts ist,
o wobei das Modell ($MLM^t$) des maschinellen Lernens konfiguriert ist und trainiert wurde, für jedes Referenzobjekt auf Basis des mindestens einen Eingabe-Referenzbildes ($RI_1(x_i)$) ein synthetisches Referenzbild ($RI_2^*(\hat{y}_i)$) zu erzeugen,

▪ wobei das synthetische Referenzbild ($RI_2^*(\hat{y}_i)$) eine Vielzahl an Bildelementen umfasst, wobei jedes Bildelement des synthetischen Referenzbildes ($RI_2^*(\hat{y}_i)$) mit jeweils einem Bildelement des Ziel-Referenzbildes ($RI_2(y_i)$) korrespondiert,
▪ wobei das Modell ($MLM^t$) des maschinellen Lernens trainiert wurde, für jedes Bildelement des synthetischen Referenzbildes ($RI_2^*(\hat{y}_i)$) einen Farbwert ($\hat{y}_i$) und einen Unsicherheitswert ($\hat{\sigma}(x_i)$) für den vorhergesagten Farbwert ($\hat{y}_i$) vorherzusagen,

▪ wobei das Trainieren ein Minimieren einer Fehlerfunktion ($\mathcal{L}$) umfasst, wobei die Fehlerfunktion ($\mathcal{L}$) (i) den vorhergesagten Farbwert ($\hat{y}_i$) und/oder eine Abweichung des vorhergesagten Farbwerts ($\hat{y}_i$) von einem Farbwert ($y_i$) des korrespondierenden Bildelements des Ziel-Referenzbildes ($RI_2(y_i)$) und (ii) den

vorhergesagten Unsicherheitswert ($\hat{\sigma}(x_i)$) als Parameter umfasst,

- Empfangen mindestens eines Eingabe-Bildes ($I_1(x_i)$) eines Untersuchungsbereichs eines Untersuchungsobjekts, wobei das mindestens eine Eingabe-Bild ($I_1(x_i)$) den Untersuchungsbereich des Untersuchungsobjekts in dem ersten Zustand repräsentiert, wobei das mindestens eine Eingabe-Bild ($I_1(x_i)$) mindestens eine computertomographische und/oder magnetresonanztomographische Aufnahme des Untersuchungsbereichs des Untersuchungsobjekts umfasst,
- Zuführen des mindestens einen Eingabe-Bildes ($I_1(x_i)$) dem trainierten Modell ($MLM^t$) des maschinellen Lernens,
- Empfangen eines synthetischen Bildes ($I_2^*(\hat{y}_i)$) von dem trainierten Modell des maschinellen Lernens, wobei das synthetische Bild ($I_2^*(\hat{y}_i)$) den Untersuchungsbereich des Untersuchungsobjekts in dem zweiten Zustand repräsentiert,
- Empfangen eines Unsicherheitswertes ($\hat{\sigma}(x_i)$) für jedes Bildelement des synthetischen Bildes ($I_2^*(\hat{y}_i)$),
- Ermitteln mindestens eines Vertrauenswertes auf Basis der empfangenen Unsicherheitswerte,
- Ausgeben des mindestens einen Vertrauenswertes.

**17.** Kontrastmittel zur Verwendung in einem radiologischen Untersuchungsverfahren, wobei das radiologische Untersuchungsverfahren umfasst:

- Bereitstellen eines trainierten Modells ($MLM^t$) des maschinellen Lernens,

o wobei das trainierte Modell ($MLM^t$) des maschinellen Lernens anhand von Trainingsdaten (TD) trainiert wurde,
o wobei die Trainingsdaten (TD) für jedes Referenzobjekt einer Vielzahl von Referenzobjekten (i) mindestens ein Eingabe-Referenzbild ($RI_1(x_i)$) eines Referenzbereichs des Referenzobjekts in einem ersten Zustand und (ii) ein Ziel-Referenzbild ($RI_2(y_i)$) des Referenzbereichs des Referenzobjekts in einem zweiten Zustand umfassen,

▪ wobei das mindestens eine Eingabe-Referenzbild ($RI_1(x_i)$) und das Ziel-Referenzbild ($RI_2(y_i)$) jeweils eine Vielzahl an Bildelementen umfassen,
▪ wobei das mindestens eine Eingabe-Referenzbild ($RI_1(x_i)$) mindestens eine computertomographische und/oder magnetresonanztomographische Aufnahme des Referenzbereichs des Referenzobjekts in dem ersten Zustand umfasst,
▪ wobei das Ziel-Referenzbild ($RI_2(y_i)$) eine computertomographische oder magnetresonanztomographische Aufnahme des Referenzbereichs des Referenzobjekts in dem zweiten Zustand ist,
▪ wobei der erste Zustand den Referenzbereich des Referenzobjekts in einer ersten Zeitspanne vor und/oder nach der Applikation des Kontrastmittels repräsentiert und der zweite Zustand den Referenzbereich des Referenzobjekts in einer zweiten Zeitspanne nach der Applikation des Kontrastmittels repräsentiert, und/oder der erste Zustand den Referenzbereich des Referenzobjekts vor und/oder nach der Applikation einer ersten Menge des Kontrastmittels repräsentiert und der zweite Zustand den Referenzbereich des Referenzobjekts nach der Applikation einer zweiten Menge des Kontrastmittels repräsentiert,

o wobei das mindestens eine Eingabe-Referenzbild ($RI_1(x_i)$) und das Ziel-Referenzbild ($RI_2(y_i)$) jeweils eine Vielzahl an Bildelementen umfassen,
o wobei das Modell ($MLM^t$) des maschinellen Lernens konfiguriert ist und trainiert wurde, für jedes Referenzobjekt auf Basis des mindestens einen Eingabe-Referenzbildes ($RI_1(x_i)$) ein synthetisches Referenzbild ($RI_2^*(\hat{y}_i)$) zu erzeugen,

▪ wobei das synthetische Referenzbild ($RI_2^*(\hat{y}_i)$) eine Vielzahl an Bildelementen umfasst, wobei jedes Bildelement des synthetischen Referenzbildes ($RI_2^*(\hat{y}_i)$) mit jeweils einem Bildelement des Ziel-Referenzbildes ($RI_2(y_i)$) korrespondiert,
▪ wobei das Modell ($MLM^t$) des maschinellen Lernens trainiert wurde, für jedes Bildelement des synthetischen Referenzbildes ($RI_2^*(\hat{y}_i)$) einen Farbwert ($\hat{y}_i$) und einen Unsicherheitswert ($\hat{\sigma}(x_i)$) für den vorhergesagten Farbwert ($\hat{y}_i$) vorherzusagen,
▪ wobei das Trainieren ein Minimieren einer Fehlerfunktion ($\mathcal{L}$) umfasst, wobei die Fehlerfunktion ($\mathcal{L}$) (i) den vorhergesagten Farbwert ($\hat{y}_i$) und/oder eine Abweichung des vorhergesagten Farbwerts ($\hat{y}_i$) von einem Farbwert ($y_i$) des korrespondierenden Bildelements des Ziel-Referenzbildes ($RI_2(y_i)$) und (ii) den

vorhergesagten Unsicherheitswert ($\hat{\sigma}(x_i)$) als Parameter umfasst,

- Empfangen mindestens eines Eingabe-Bildes ($I_1(x_i)$) eines Untersuchungsbereichs eines Untersuchungsobjekts, wobei das mindestens eine Eingabe-Bild ($I_1(x_i)$) mindestens eine computertomographische oder magnetresonanztomographische Aufnahme des Untersuchungsbereichs des Untersuchungsobjekts in dem ersten Zustand umfasst,
- Zuführen des mindestens einen Eingabe-Bildes ($I_1(x_i)$) dem trainierten Modell ($MLM^t$) des maschinellen Lernens,
- Empfangen eines synthetischen Bildes ($I_2^*(\hat{y}_i)$) von dem trainierten Modell des maschinellen Lernens, wobei das synthetische Bild ($I_2^*(\hat{y}_i)$) eine synthetische radiologische Aufnahme umfasst, die den Untersuchungsbereich des Untersuchungsobjekts in dem zweiten Zustand repräsentiert,
- Empfangen eines Unsicherheitswertes ($\hat{\sigma}(x_i)$) für jedes Bildelement des synthetischen Bildes ($I_2^*(\hat{y}_i)$),
- Ermitteln mindestens eines Vertrauenswertes auf Basis der empfangenen Unsicherheitswerte,
- Ausgeben des mindestens einen Vertrauenswertes.

**18.** Verwendung eines Kontrastmittels in einem radiologischen Untersuchungsverfahren, wobei das radiologische Untersuchungsverfahren umfasst:

- Bereitstellen eines trainierten Modells ($MLM^t$) des maschinellen Lernens,

  o wobei das trainierte Modell ($MLM^t$) des maschinellen Lernens anhand von Trainingsdaten (TD) trainiert wurde,
  o wobei die Trainingsdaten (TD) für jedes Referenzobjekt einer Vielzahl von Referenzobjekten (i) mindestens ein Eingabe-Referenzbild ($RI_1(x_i)$) eines Referenzbereichs des Referenzobjekts in einem ersten Zustand und (ii) ein Ziel-Referenzbild ($RI_2(y_i)$) des Referenzbereichs des Referenzobjekts in einem zweiten Zustand umfassen,

    ▪ wobei das mindestens eine Eingabe-Referenzbild ($RI_1(x_i)$) und das Ziel-Referenzbild ($RI_2(y_i)$) jeweils eine Vielzahl an Bildelementen umfassen,
    ▪ wobei das mindestens eine Eingabe-Referenzbild ($RI_1(x_i)$) mindestens eine computertomographische und/oder magnetresonanztomographische Aufnahme des Referenzbereichs des Referenzobjekts in dem ersten Zustand umfasst,
    ▪ wobei das Ziel-Referenzbild ($RI_2(y_i)$) eine computertomographische oder magnetresonanztomographische Aufnahme des Referenzbereichs des Referenzobjekts in dem zweiten Zustand ist,
    ▪ wobei der erste Zustand den Referenzbereich des Referenzobjekts in einer ersten Zeitspanne vor und/oder nach der Applikation des Kontrastmittels repräsentiert und der zweite Zustand den Referenzbereich des Referenzobjekts in einer zweiten Zeitspanne nach der Applikation des Kontrastmittels repräsentiert, und/oder der erste Zustand den Referenzbereich des Referenzobjekts vor und/oder nach der Applikation einer ersten Menge des Kontrastmittels repräsentiert und der zweite Zustand den Referenzbereich des Referenzobjekts nach der Applikation einer zweiten Menge des Kontrastmittels repräsentiert,

  o wobei das mindestens eine Eingabe-Referenzbild ($RI_1(x_i)$) und das Ziel-Referenzbild ($RI_2(y_i)$) jeweils eine Vielzahl an Bildelementen umfassen,
  o wobei das Modell ($MLM^t$) des maschinellen Lernens konfiguriert ist und trainiert wurde, für jedes Referenzobjekt auf Basis des mindestens einen Eingabe-Referenzbildes ($RI_1(x_i)$) ein synthetisches Referenzbild ($RI_2^*(\hat{y}_i)$) zu erzeugen,

    ▪ wobei das synthetische Referenzbild ($RI_2^*(\hat{y}_i)$) eine Vielzahl an Bildelementen umfasst, wobei jedes Bildelement des synthetischen Referenzbildes ($RI_2^*(\hat{y}_i)$) mit jeweils einem Bildelement des Ziel-Referenzbildes ($RI_2(y_i)$) korrespondiert,
    ▪ wobei das Modell ($MLM^t$) des maschinellen Lernens trainiert wurde, für jedes Bildelement des synthetischen Referenzbildes ($RI_2^*(\hat{y}_i)$) einen Farbwert ($\hat{y}_i$) und einen Unsicherheitswert ($\hat{\sigma}(x_i)$) für den vorhergesagten Farbwert ($\hat{y}_i$) vorherzusagen,
    ▪ wobei das Trainieren ein Minimieren einer Fehlerfunktion ($\mathcal{L}$) umfasst, wobei die Fehlerfunktion ($\mathcal{L}$) (i) den vorhergesagten Farbwert ($\hat{y}_i$) und/oder eine Abweichung des vorhergesagten Farbwerts ($\hat{y}_i$) von einem Farbwert ($y_i$) des korrespondierenden Bildelements des Ziel-Referenzbildes ($RI_2(y_i)$) und (ii) den vorhergesagten Unsicherheitswert ($\hat{\sigma}(x_i)$) als Parameter umfasst,

- Empfangen mindestens eines Eingabe-Bildes ($I_1(x_i)$) eines Untersuchungsbereichs eines Untersuchungsobjekts, wobei das mindestens eine Eingabe-Bild ($I_1(x_i)$) mindestens eine computertomographische oder magnetresonanztomographische Aufnahme des Untersuchungsbereichs des Untersuchungsobjekts in dem ersten Zustand umfasst,
- Zuführen des mindestens einen Eingabe-Bildes ($I_1(x_i)$) dem trainierten Modell ($MLM^t$) des maschinellen Lernens,
- Empfangen eines synthetischen Bildes ($I_2^*(\hat{y}_i)$) von dem trainierten Modell des maschinellen Lernens, wobei das synthetische Bild ($I_2^*(\hat{y}_i)$) eine synthetische radiologische Aufnahme umfasst, die den Untersuchungsbereich des Untersuchungsobjekts in dem zweiten Zustand repräsentiert,
- Empfangen eines Unsicherheitswertes ($\hat{\sigma}(x_i)$) für jedes Bildelement des synthetischen Bildes ($I_2^*(\hat{y}_i)$),
- Ermitteln mindestens eines Vertrauenswertes auf Basis der empfangenen Unsicherheitswerte,
- Ausgeben des mindestens einen Vertrauenswertes.

**19.** Verfahren gemäß einer der Ansprüche 1 bis 18, wobei das Kontrastmittel eine oder mehrere der folgenden Verbindungen umfasst:

- Gadolinium(III) 2-[4,7,10-tris(carboxymethyl)-1,4,7,10-tetrazacyclododec-1 -yl]essigsäure
- Gadolinium(III) Ethoxybenzyl-diethylenetriaminepentaessigsäure
- Gadolinium(III) 2-[3,9-bis[1-carboxylato-4-(2,3-dihydroxypropylamino)-4-oxobutyl]-3,6,9,15-tetrazabicyclo [9.3.1]pentadeca-1(15),11,13-trien-6-yl]-5-(2,3-dihydroxypropylamino)-5-oxopentanoat
- Dihydrogen[(±)-4-carboxy-5,8,11-tris(carboxymethyl)-1-phenyl-2-oxa-5,8,11-triazatridecan-13-oato(5-)]gadolinat(2-)
- Tetragadolinium-[4,10-bis(carboxylatomethyl)-7-{3,6,12,15-tetraoxo-16-[4,7,10-tris-(carboxylatomethyl)-1,4,7,10-tetraazacyclododecan-1-yl]-9,9-bis({[({2-[4,7,10-tris-(carboxylatomethyl)-1,4,7,10-tetraazacyclododecan-1-yl]propanoyl}amino)acetyl]-amino}methyl)-4,7,11,14-tetraazahepta-decan-2-yl}-1,4,7,10-tetraazacyclododecan-1-yl]acetat
- Gadolinium 2,2',2"-(10-{1-carboxy-2-[2-(4-ethoxyphenyl)ethoxy]ethyl}-1,4,7,10-tetraazacyclododecan-1,4,7-triyl)triacetat
- Gadolinium 2,2',2"-{10-[1-carboxy-2-{4-[2-(2-ethoxyethoxy)ethoxy]phenyl}ethyl]-1,4,7,10-tetraazacyclododecan-1,4,7-triyl}triacetat
- Gadolinium 2,2',2"-{10-[(1R)-1-carboxy-2-{4-[2-(2-ethoxyethoxy)ethoxy]phenyl}ethyl]-1,4,7,10-tetraazacyclododecan-1,4,7-triyl}triacetat
- Gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecan-1,4,7-triyl}tris(3-hydroxypropanoat)
- Gadolinium 2,2',2"-{10-[(1S)-4-(4-butoxyphenyl)-1-carboxybutyl]-1,4,7,10-tetraazacyclododecan-1,4,7-triyl} triacetat
- Gadolinium-2,2',2"-{(2*S*)-10-(carboxymethyl)-2-[4-(2-ethoxyethoxy)benzyl]-1,4,7,10-tetraazacyclododecane-1,4,7-triyl}triacetat
- Gadolinium-2,2',2"-[10-(carboxymethyl)-2-(4-ethoxybenzyl)-1,4,7,10-tetraazacyclododecane-1,4,7-triyl]triacetat
- Gadolinium(III) 5,8-bis(carboxylatomethyl)-2-[2-(methylamino)-2-oxoethyl]-10-oxo-2,5,8,11-tetraazadodecan-1-carboxylat-Hydrat
- Gadolinium(III) 2-[4-(2-hydroxypropyl)-7,10-bis(2-oxido-2-oxoethyl)-1,4,7,10-tetrazacyclododec-1-yl]acetat
- Gadolinium(III) 2,2',2"-(10-((2R,3 S)-1,3,4-trihydroxybutan-2-yl)-1,4,7,10-tetraazacyclododecan-1,4,7-triyl) triacetat
- einen $Gd^{3+}$-Komplex einer Verbindung der Formel (I)

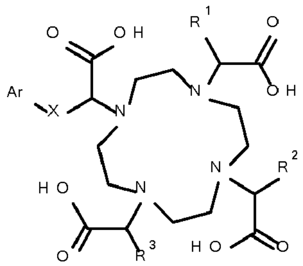

(I) ,

wobei

Ar eine Gruppe ausgewählt aus

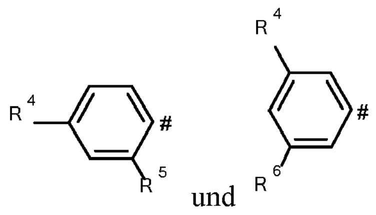

darstellt,
wobei # die Anknüpfung zu X darstellt,
X eine Gruppe darstellt, die aus $CH_2$, $(CH_2)_2$, $(CH_2)_3$, $(CH_2)_4$ und *-$(CH_2)_2$-O-$CH_2$-# ausgewählt wird, wobei * die Anknüpfung zu Ar darstellt und # die Anknüpfung zum Essigsäurerest darstellt,
$R^1$, $R^2$ and $R^3$ unabhängig voneinander ein Wasserstoffatom oder eine Gruppe ausgewählt aus $C_1$-$C_3$-Alkyl, -$CH_2OH$, -$(CH_2)_2OH$ und -$CH_2OCH_3$ darstellen,
$R^4$ eine Gruppe ausgewählt aus $C_2$-$C_4$-Alkoxy, ($H_3C$-$CH_2$)-O-$(CH_2)_2$-O-, ($H_3C$-$CH_2$)-O-$(CH_2)_2$-O-$(CH_2)_2$-O- und ($H_3C$-$CH_2$)-O-$(CH_2)_2$-O-$(CH_2)_2$-O-$(CH_2)_2$-O- darstellt,
$R^5$ ein Wasserstoffatom darstellt, und
$R^6$ ein Wasserstoffatom darstellt,
oder ein Stereoisomer, Tautomer, Hydrat, Solvat oder Salz davon, oder eine Mischung davon,

- einen $Gd^{3+}$-Komplex einer Verbindung der Formel (II)

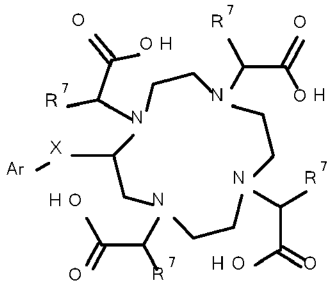

(II) ,

wobei

Ar eine Gruppe ausgewählt aus

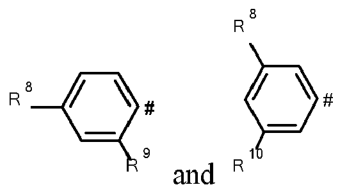

darstellt,
wobei # die Anknüpfung zu X darstellt,
X eine Gruppe darstellt, die aus $CH_2$, $(CH_2)_2$, $(CH_2)_3$, $(CH_2)_4$ and *-$(CH_2)_2$-O-$CH_2$-# ausgewählt wird, wobei * die Anknüpfung zu Ar darstellt und # die Anknüpfung zum Essigsäurerest darstellt,
$R^7$ ein Wasserstoffatom oder eine Gruppe ausgewählt aus $C_1$-$C_3$-Alkyl, -$CH_2OH$, -$(CH_2)_2OH$ und -$CH_2OCH_3$ darstellt,
$R^8$ eine Gruppe ausgewählt aus $C_2$-$C_4$-Alkoxy, ($H_3C$-$CH_2O$)-$(CH_2)_2$-O-, ($H_3C$-$CH_2O$)-$(CH_2)_2$-O-$(CH_2)_2$-O- und ($H_3C$-$CH_2O$)-$(CH_2)_2$-O-$(CH_2)_2$-O-$(CH_2)_2$-O- darstellt,

$R^9$ und $R^{10}$ unabhängig voneinander ein Wasserstoffatom darstellen;
oder ein Stereoisomer, Tautomer, Hydrat, Solvat oder Salz davon, oder eine Mischung davon.

**20.** Kit umfassend ein Kontrastmittel und ein Computerprogramm, das in einen Arbeitsspeicher eines Computersystems geladen werden kann und dort das Computersystem dazu veranlasst, folgende Schritte ausführen:

- Bereitstellen eines trainierten Modells ($MLM^t$) des maschinellen Lernens,

o wobei das trainierte Modell ($MLM^t$) des maschinellen Lernens anhand von Trainingsdaten (TD) trainiert wurde,
o wobei die Trainingsdaten (TD) für jedes Referenzobjekt einer Vielzahl von Referenzobjekten (i) mindestens ein Eingabe-Referenzbild ($RI_1(x_i)$) eines Referenzbereichs des Referenzobjekts in einem ersten Zustand und (ii) ein Ziel-Referenzbild ($RI_2(y_i)$) des Referenzbereichs des Referenzobjekts in einem zweiten Zustand umfassen,

▪ wobei das mindestens eine Eingabe-Referenzbild ($RI_1(x_i)$) und das Ziel-Referenzbild ($RI_2(y_i)$) jeweils eine Vielzahl an Bildelementen umfassen,
▪ wobei das mindestens eine Eingabe-Referenzbild ($RI_1(x_i)$) mindestens eine computertomographische und/oder magnetresonanztomographische Aufnahme des Referenzbereichs des Referenzobjekts in dem ersten Zustand umfasst,
▪ wobei das Ziel-Referenzbild ($RI_2(y_i)$) eine computertomographische oder magnetresonanztomographische Aufnahme des Referenzbereichs des Referenzobjekts in dem zweiten Zustand ist,
▪ wobei der erste Zustand den Referenzbereich des Referenzobjekts in einer ersten Zeitspanne vor und/oder nach der Applikation des Kontrastmittels repräsentiert und der zweite Zustand den Referenzbereich des Referenzobjekts in einer zweiten Zeitspanne nach der Applikation des Kontrastmittels repräsentiert, und/oder der erste Zustand den Referenzbereich des Referenzobjekts vor und/oder nach der Applikation einer ersten Menge des Kontrastmittels repräsentiert und der zweite Zustand den Referenzbereich des Referenzobjekts nach der Applikation einer zweiten Menge des Kontrastmittels repräsentiert,

o wobei das mindestens eine Eingabe-Referenzbild ($RI_1(x_i)$) und das Ziel-Referenzbild ($RI_2(y_i)$) jeweils eine Vielzahl an Bildelementen umfassen,
o wobei das Modell ($MLM^t$) des maschinellen Lernens konfiguriert ist und trainiert wurde, für jedes Referenzobjekt auf Basis des mindestens einen Eingabe-Referenzbildes ($RI_1(x_i)$) ein synthetisches Referenzbild ($RI_2^*(\hat{y}_i)$) zu erzeugen,

▪ wobei das synthetische Referenzbild ($RI_2^*(\hat{y}_i)$) eine Vielzahl an Bildelementen umfasst, wobei jedes Bildelement des synthetischen Referenzbildes ($RI_2^*(\hat{y}_i)$) mit jeweils einem Bildelement des Ziel-Referenzbildes ($RI_2(y_i)$) korrespondiert,
▪ wobei das Modell ($MLM^t$) des maschinellen Lernens trainiert wurde, für jedes Bildelement des synthetischen Referenzbildes ($RI_2^*(\hat{y}_i)$) einen Farbwert ($\hat{y}_i$) und einen Unsicherheitswert ($\hat{\sigma}(x_i)$) für den vorhergesagten Farbwert ($\hat{y}_i$) vorherzusagen,
▪ wobei das Trainieren ein Minimieren einer Fehlerfunktion ($\mathcal{L}$) umfasst, wobei die Fehlerfunktion ($\mathcal{L}$) (i) den vorhergesagten Farbwert ($\hat{y}_i$) und/oder eine Abweichung des vorhergesagten Farbwerts ($\hat{y}_i$) von einem Farbwert ($y_i$) des korrespondierenden Bildelements des Ziel-Referenzbildes ($RI_2(y_i)$) und (ii) den vorhergesagten Unsicherheitswert ($\hat{\sigma}(x_i)$) als Parameter umfasst,

- Empfangen mindestens eines Eingabe-Bildes ($I_1(x_i)$) eines Untersuchungsbereichs eines Untersuchungsobjekts, wobei das mindestens eine Eingabe-Bild ($I_1(x_i)$) mindestens eine computertomographische oder magnetresonanztomographische Aufnahme des Untersuchungsbereichs des Untersuchungsobjekts in dem ersten Zustand umfasst,
- Zuführen des mindestens einen Eingabe-Bildes ($I_1(x_i)$) dem trainierten Modell ($MLM^t$) des maschinellen Lernens,
- Empfangen eines synthetischen Bildes ($I_2^*(\hat{y}_i)$) von dem trainierten Modell des maschinellen Lernens, wobei das synthetische Bild ($I_2^*(\hat{y}_i)$) eine synthetische radiologische Aufnahme umfasst, die den Untersuchungsbereich des Untersuchungsobjekts in dem zweiten Zustand repräsentiert,
- Empfangen eines Unsicherheitswertes ($\hat{\sigma}(x_i)$) für jedes Bildelement des synthetischen Bildes ($I_2^*(\hat{y}_i)$),
- Ermitteln mindestens eines Vertrauenswertes auf Basis der empfangenen Unsicherheitswerte,

- Ausgeben des mindestens einen Vertrauenswertes.

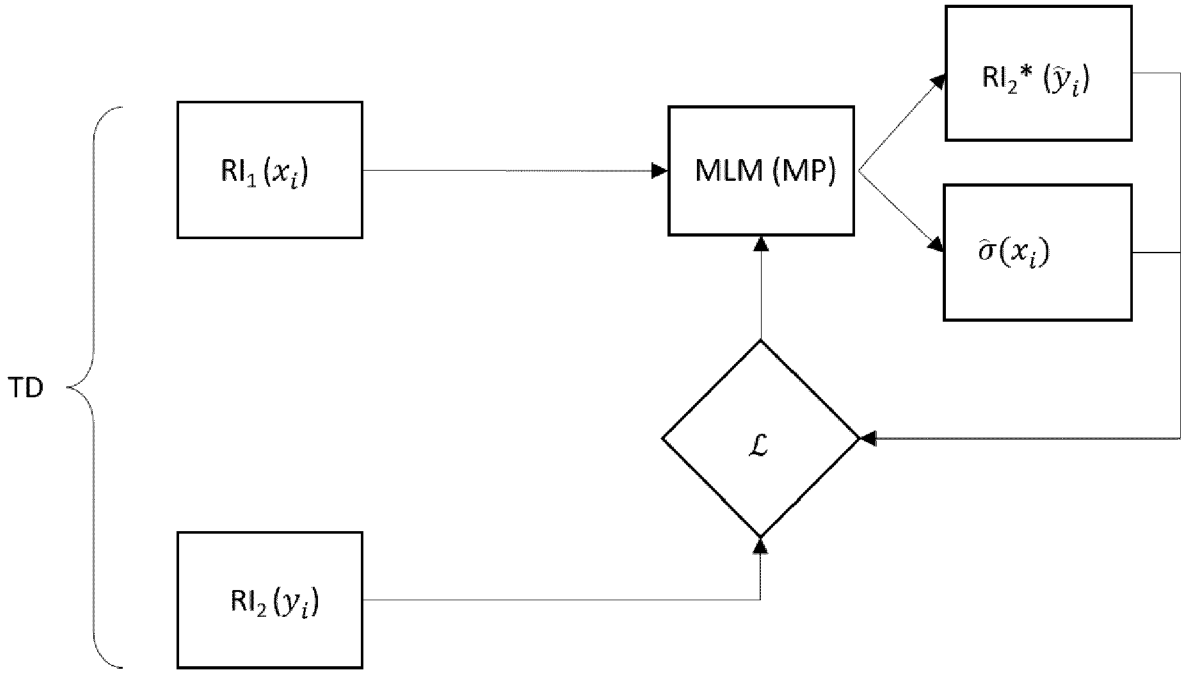
$RI_1(x_i)$
MLM (MP)
$RI_2^*(\hat{y}_i)$
$\hat{\sigma}(x_i)$
TD
$\mathcal{L}$
$RI_2(y_i)$

Fig. 1

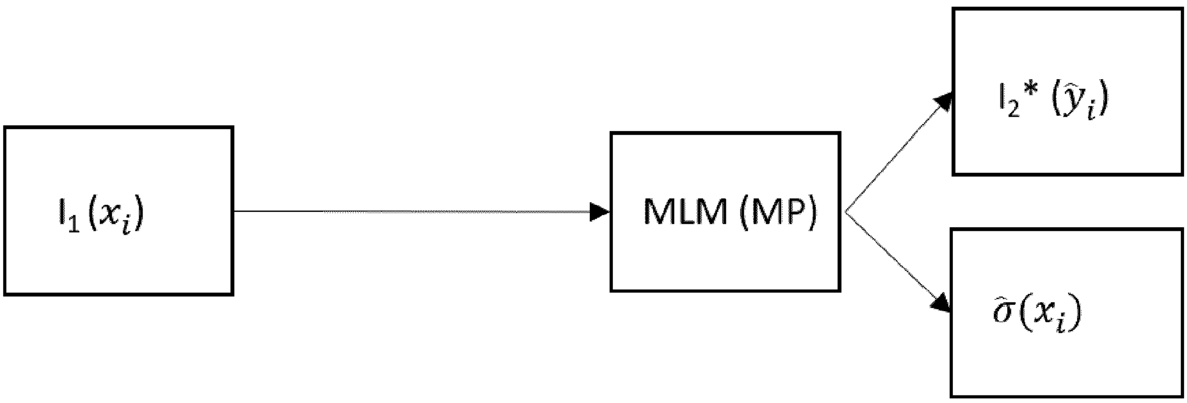
$I_1(x_i)$
MLM (MP)
$I_2^*(\hat{y}_i)$
$\hat{\sigma}(x_i)$

Fig. 2

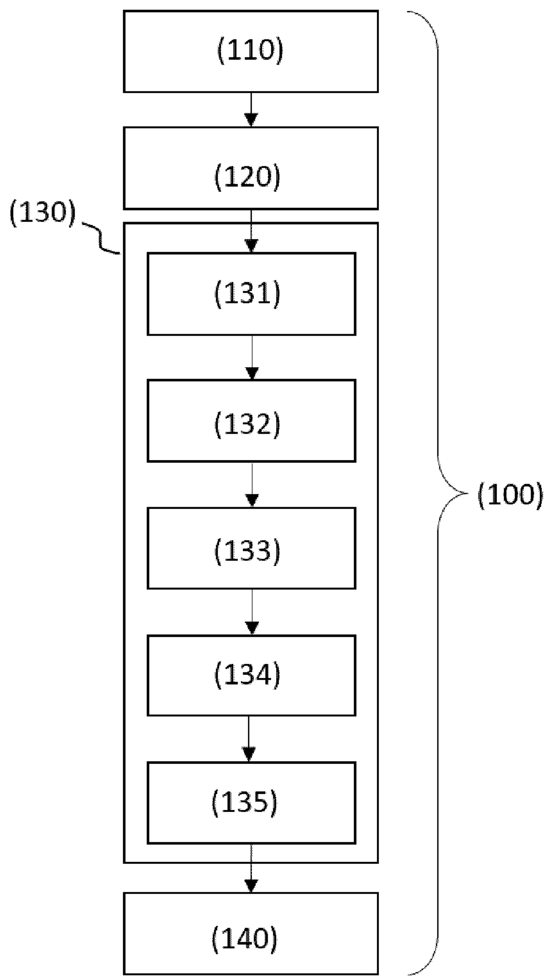


Fig. 3

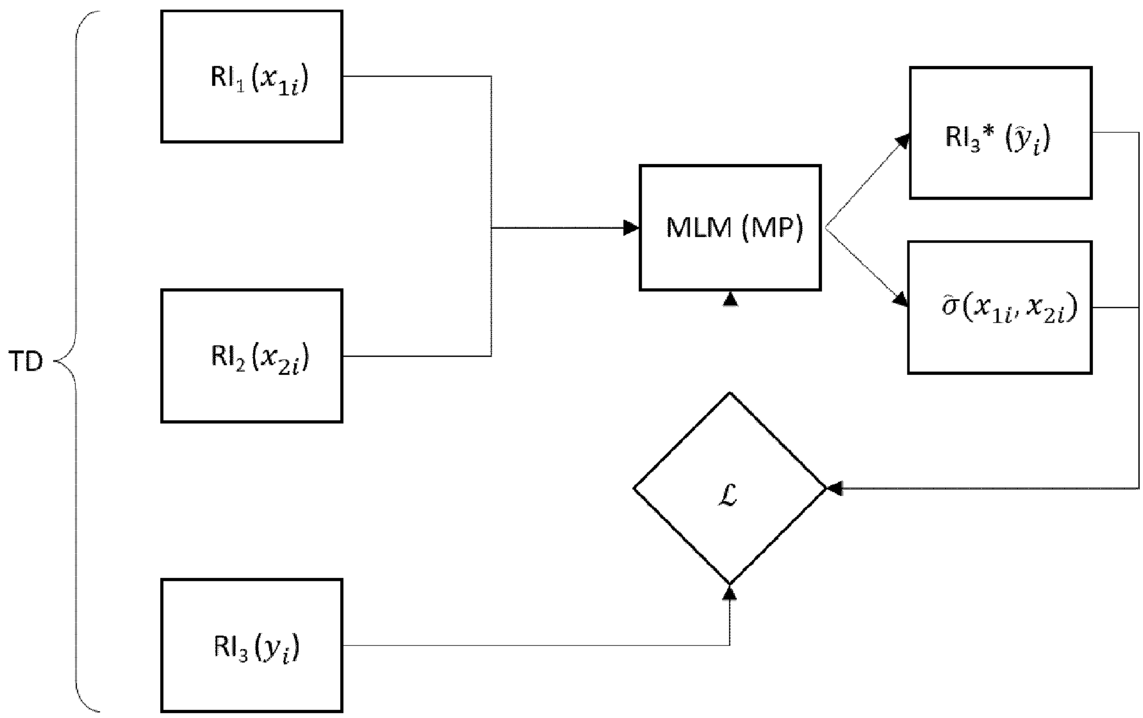

TD
$RI_1 (x_{1i})$
$RI_2 (x_{2i})$
$RI_3 (y_i)$
MLM (MP)
$RI_3^* (\hat{y}_i)$
$\hat{\sigma}(x_{1i}, x_{2i})$
$\mathcal{L}$


Fig. 4

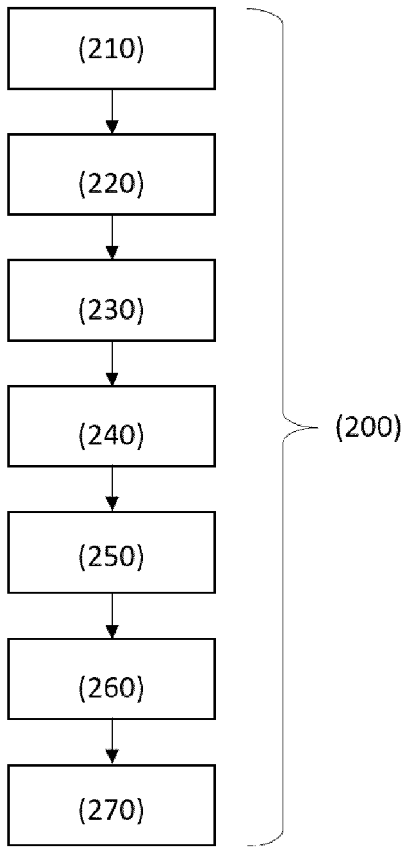

(210)
(220)
(230)
(240)
(250)
(260)
(270)
(200)


Fig. 5

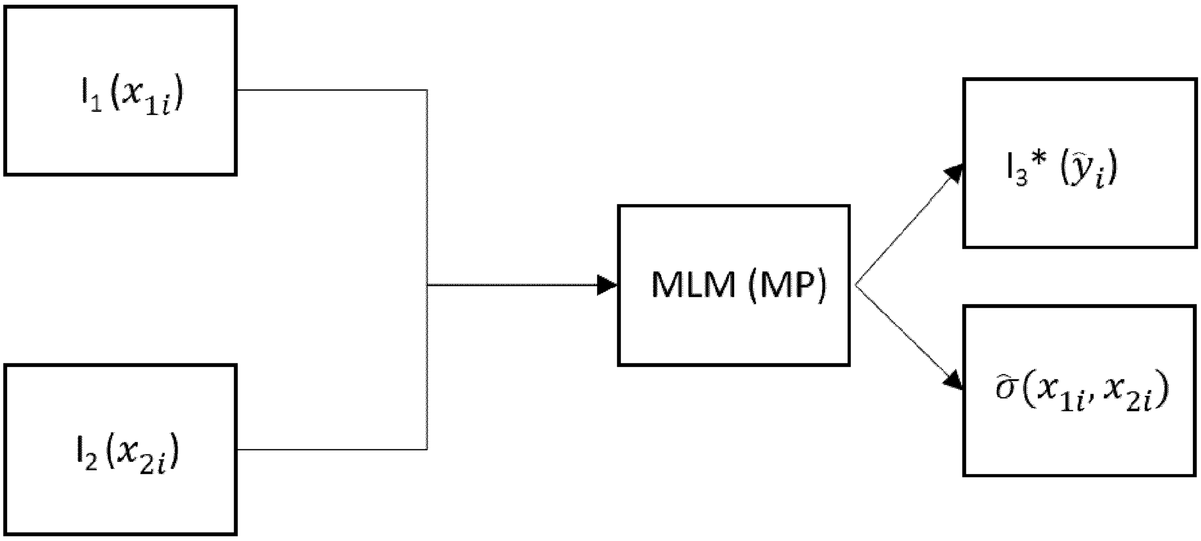


Fig. 6

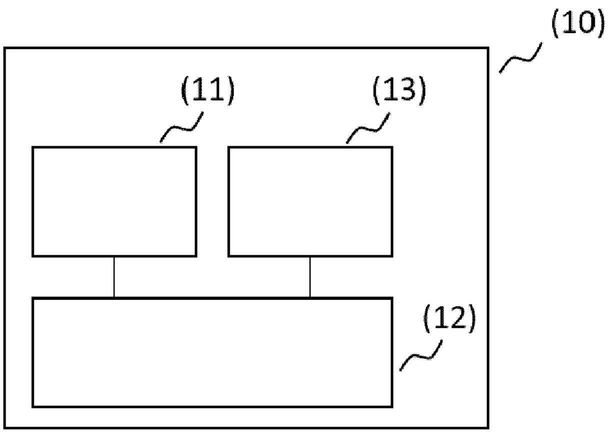


Fig. 7

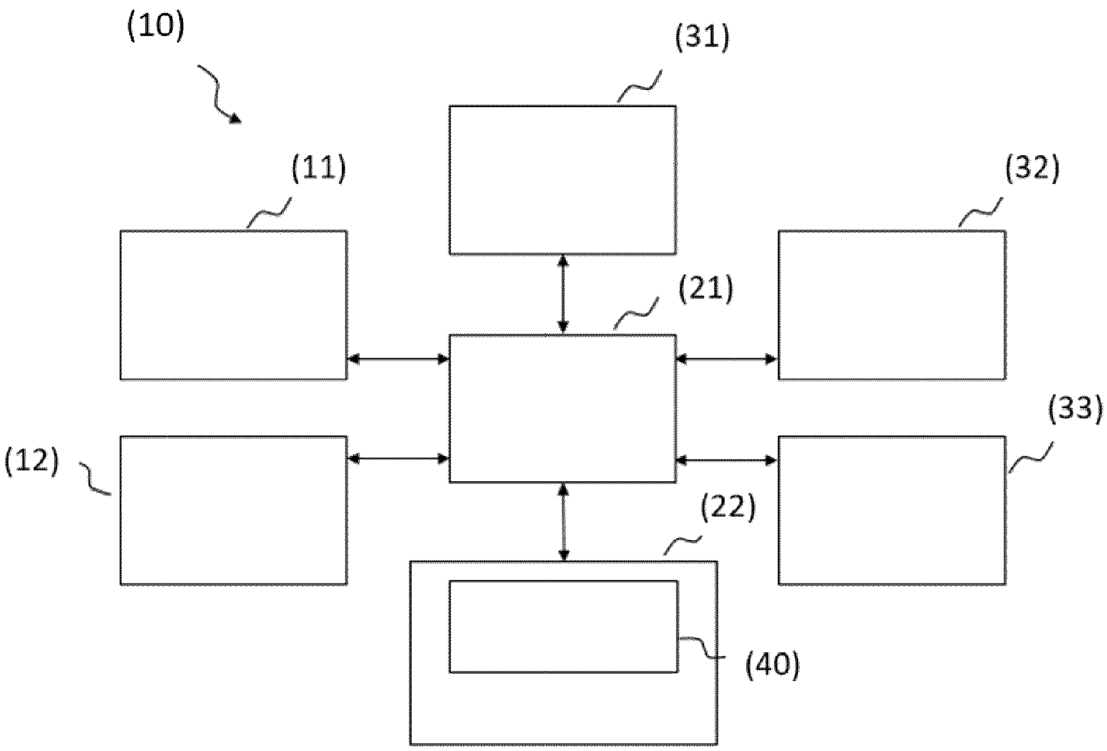


Fig. 8

Europäisches Patentamt
European Patent Office
Office européen des brevets

# EUROPÄISCHER RECHERCHENBERICHT

Nummer der Anmeldung
EP 24 18 9305

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | VISWANATH P SUDARSHAN ET AL: "Towards Lower-Dose PET using Physics-Based Uncertainty-Aware Multimodal Learning with Robustness to Out-of-Distribution Data", ARXIV.ORG, CORNELL UNIVERSITY LIBRARY, 201 OLIN LIBRARY CORNELL UNIVERSITY ITHACA, NY 14853, 21. Juli 2021 (2021-07-21), XP091014733, * das ganze Dokument * ----- | 1-20 | INV. G06T11/00 G06T5/60 |
| X | KLEESIEK JENS ET AL: "Can Virtual Contrast Enhancement in Brain MRI Replace Gadolinium? : A Feasibility Study", INVESTIGATIVE RADIOLOGY, Bd. 54, Nr. 10, Oktober 2019 (2019-10), Seiten 653-660, XP055804228, US ISSN: 0020-9996, DOI: 10.1097/RLI.0000000000000583 | 1,15-20 | |
| A | * das ganze Dokument * ----- | 2-14 | RECHERCHIERTE SACHGEBIETE (IPC) |
| A | HESS ANDREAS ET AL: "Synthetic Perfusion Maps: Imaging Perfusion Deficits in DSC-MRI with Deep Learning", 26. Januar 2019 (2019-01-26), ADVANCES IN DATABASES AND INFORMATION SYSTEMS; [LECTURE NOTES IN COMPUTER SCIENCE; LECT.NOTES COMPUTER], SPRINGER INTERNATIONAL PUBLISHING, CHAM, PAGE(S) 447 - 455, XP047502348, ISBN: 978-3-319-10403-4 [gefunden am 2019-01-26] * das ganze Dokument * ----- | 1-20 | G06T |

1 Der vorliegende Recherchenbericht wurde für alle Patentansprüche erstellt

| Recherchenort | Abschlußdatum der Recherche | Prüfer |
|---|---|---|
| München | 5. Dezember 2024 | Werling, Alexander |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2018202541 A1
- WO 2020229152 A1
- WO 2021052896 A1
- WO 2021069338 A1
- WO 2019074938 A1
- WO 2022184297 A1
- WO 2022184297 A
- WO 2007042504 A
- WO 2020030618 A
- WO 2022013454 A
- WO 2016193190 A
- WO 2022194777 A
- WO 2023161041 A1
- US 6039931 A


### In der Beschreibung aufgeführte Nicht-Patentliteratur


- **K. SCHWARZ et al.** *On the Frequency Bias of Generative Models*, https://doi.org/10.48550/arXiv.2111.02447
- **A. S. L. JASCINTH et al.** Contrast Agents in computed tomography: A Review. *Journal of Applied Dental and Medical Sciences*, 2016, vol. 2 (2), 143-149
- **H. LUSIC et al.** X-ray-Computed Tomography Contrast Agents. *Chem. Rev.*, 2013, vol. 113 (3), 1641-1666, https://www.radiology.wisc.edu/wp-content/uploads/2017/10/contrast-agents-tutorial.pdf
- **M. R. NOUGH et al.** Radiographie and magnetic resonances contrast agents: Essentials and tips for safe practices. *World J Radiol.*, 28 September 2017, vol. 9 (9), 339-349
- **L. C. ABONYI et al.** Intravascular Contrast Media in Radiography: Historical Development & Review of Risk Factors for Adverse Reactions. *South American Journal of Clinical Research*, 2016, vol. 3 (1), 1-10
- *ACR Manual on Contrast Media*, 2020, ISBN 978-1-55903-012-0
- **A. IGNEE et al.** Ultrasound contrast agents. *Endosc Ultrasound.*, November 2016, vol. 5 (6), 355-362
- **Z.B. J. LOHRKE et al.** Preclinical Profile of Gadoquatrane: A Novel Tetramerie, Macrocyclic High Relaxivity Gadolinium-Based Contrast Agent. *Invest Radiol.*, 2022, vol. 1 (10), 629-638
- *J. Magn. Reson. Imaging*, 2012, vol. 35 (3), 492-511
- *Clujul Medical*, 2015, vol. 88 (4), 438-448
- *Journal of Hepatology*, 2019, vol. 71, 534-542
- **O. RONNEBERGER et al.** : U-Net: Convolutional Networks for Biomedical Image Segmentation. *arXiv:1505.04597v1*
- **V. P. SUDARSHAN et al.** : Towards lower-dose PET usingphysics-based uncertainty-aware multimodal learning with robustness to out-of-distribution data,. *Medical Image Analysis*, 2021, vol. 73, 102187